(19) Europäisches Patentamt
European Patent Office
Office européen des brevets

(11) EP 4 098 281 A1

(12) EUROPEAN PATENT APPLICATION
published in accordance with Art. 153(4) EPC

(43) Date of publication:
**07.12.2022 Bulletin 2022/49**

(21) Application number: **21748225.6**

(22) Date of filing: **27.01.2021**

(51) International Patent Classification (IPC):
***A61K 47/24** (2006.01)* ***A61K 9/08** (2006.01)*
***A61K 9/107** (2006.01)* ***A61K 9/12** (2006.01)*
***A61K 38/08** (2019.01)* ***A61K 45/00** (2006.01)*
***A61K 47/14** (2017.01)* ***A61K 47/69** (2017.01)*
***A61P 5/06** (2006.01)* ***A61P 35/00** (2006.01)*
***A61P 41/00** (2006.01)* ***C07C 69/003** (2006.01)*
***C07D 307/20** (2006.01)* ***C07D 493/04** (2006.01)*

(52) Cooperative Patent Classification (CPC):
**A61K 9/08; A61K 9/107; A61K 9/12; A61K 38/08; A61K 45/00; A61K 47/14; A61K 47/24; A61K 47/69; A61P 5/06; A61P 35/00; A61P 41/00; C07C 69/003; C07D 307/20; C07D 493/04**

(86) International application number:
**PCT/JP2021/002917**

(87) International publication number:
**WO 2021/153635 (05.08.2021 Gazette 2021/31)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **27.01.2020 JP 2020011237**
**21.12.2020 JP 2020211758**

(71) Applicant: **Farnex Incorporated**
**Ota-ku**
**Tokyo 145-0064 (JP)**

(72) Inventors:
- **TABATA Yasuhiko**
  **Kyoto-shi, Kyoto 606-8501 (JP)**
- **MURAKAMI Takahide**
  **Kyoto-shi, Kyoto 606-8501 (JP)**
- **TODO Hiroaki**
  **Tsurugashima-shi, Saitama 350-2206 (JP)**
- **ITAKURA Shoko**
  **Tsurugashima-shi, Saitama 350-2213 (JP)**
- **SUGIBAYASHI Kenji**
  **Kawagoe-shi, Saitama 350-1123 (JP)**
- **HIJIKURO Ichiro**
  **Yokohama-shi, Kanagawa 230-0045 (JP)**
- **TANOMURA Masahisa**
  **Yokohama-shi, Kanagawa 230-0045 (JP)**

(74) Representative: **Mewburn Ellis LLP**
**Aurora Building**
**Counterslip**
**Bristol BS1 6BX (GB)**

# (54) HIGHLY SAFE NON-LAMELLAR LIQUID CRYSTAL FORMING COMPOSITION

(57) The present invention provides a highly safe non-lamellar liquid crystal-forming composition. The present invention relates to a non-lamellar liquid crystal-forming composition comprising a phospholipid and an amphipathic compound represented by the following general formula (I), wherein X and Y each denotes a hydrogen atom or together denote an oxygen atom, n denotes the integer 1 or 2, m denotes the integer 1 or 2, and R denotes a hydrophilic group having one or more hydroxyl groups, and wherein the composition has an increased biocompatibility by the phospholipid.

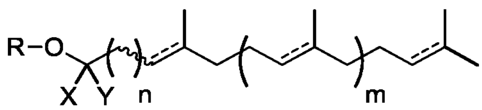

(I)

EP 4 098 281 A1

## Description

### Technical Field

The present invention relates to a highly safe non-lamellar liquid crystal-forming composition.

### Background Art

Many reports have mentioned the usefulness of lyotropic liquid crystals such as liposomes as biomimetic drug delivery system (DDS) carriers since the DDS concept was proposed. In recent years, non-lamellar liquid crystals (NLLCs), one type of the lyotropic liquid crystals, have been reported to have advantages such as high drug contents and easy preparability, as compared with conventional DDS carriers.

Various liquid crystal-forming compounds are used for various applications in the fields of cosmetics, pharmaceutical products, and the like. In recent years, amphipathic compounds which have isoprenoid fatty acid chains and are capable of forming cubic liquid crystals that exhibit high stability even at low temperatures (lower than 6°C) have been developed (Patent Literature 1). Amphipathic compounds having isoprenoid fatty acid chains which stably form non-lamellar liquid crystals, and have low viscosities and thus are useful as bases for injections have also been developed (Patent Literature 2). Patent Literature 3 has reported that amphipathic compounds which have isoprenoid fatty acid chains and are capable of forming non-lamellar liquid crystals are useful as adhesion preventing agents.

Meanwhile, glyceryl monooleate (GMO) or phytantriol (PHY) that is a liquid crystal-forming compound (self-organizing lipid/SOL) used as food additives or in skin or hair cosmetics is recognized as being safe for humans by external application or oral ingestion, whereas serious problems with safeties, such as cytotoxicity and hemolysis, have been pointed out as to *in vivo* use thereof (e.g., Non Patent Literatures 1 and 2).

However, any general-use technique that can increase safeties in *in vivo* application while maintaining the effectiveness of pharmaceutical formulations containing liquid crystal-forming compounds has not yet been established.

### Citation List

#### Patent Literature

Patent Literature 1: International Patent Publication WO 2006/043705
Patent Literature 2: International Patent Publication WO 2011/078383
Patent Literature 3: International Patent Publication WO 2014/178256

#### Non Patent Literature

Non Patent Literature 1: Hinton T.M., et al., Toxicology Research, Vol. 3, (2014) p. 11-22
Non Patent Literature 2: Wibroe P.P., et al., Nanomedicine: Nanotechnology, Biology and Medicine, Vol. 11 (2015) p. 1909-1914

### Summary of Invention

#### Technical Problem

An object of the present invention is to provide a highly safe non-lamellar liquid crystal-forming composition. Another object of the present invention is to provide a non-lamellar liquid crystal-forming composition using a novel amphipathic compound.

#### Solution to Problem

The present inventors have conducted diligent studies to attain the objects and consequently found that a non-lamellar liquid crystal-forming composition having an increased biocompatibility, which has a high safety that permits *in vivo* application, can be prepared by adding a phospholipid to an amphipathic compound having a certain isoprenoid fatty acid chain. Further, the present inventors have also successfully synthesized novel amphipathic compounds having

an isoprenoid fatty acid chain that can be used in the preparation of non-lamellar liquid crystal-forming compositions. Thus, the present inventors have completed the present invention.

Specifically, the present invention encompasses the following.

[1] A non-lamellar liquid crystal-forming composition comprising an amphipathic compound represented by the following general formula (I) and a phospholipid:

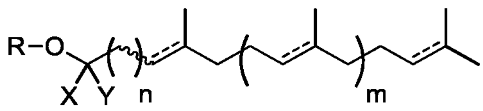

(I)

wherein X and Y each denotes a hydrogen atom or together denote an oxygen atom, n denotes the integer 1 or 2, m denotes the integer 1 or 2, the designation: ------
denotes a single bond or double bond, and R denotes a hydrophilic group having one or more hydroxyl groups, and
wherein the composition has an increased biocompatibility by the phospholipid.

[2] The composition according to [1] above, wherein the amphipathic compound is represented by the following general formula (III) or (IV):

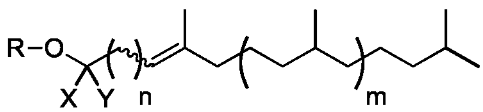

(III)

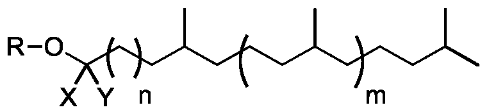

(IV)

wherein X and Y each denotes a hydrogen atom or together denote an oxygen atom, n denotes the integer 1 or 2, and m denotes the integer 1 or 2.
[3] The composition according to [1] or [2] above, wherein n = 2 and m = 2 in the general formula.
[4] The composition according to any of [1] to [3] above, wherein a weight ratio between the amphipathic compound and the phospholipid is 80:20 to 20:80, or 70:30 to 30:70.
[5] The composition according to [4] above, wherein the weight ratio between the amphipathic compound and the phospholipid is 50:50 to 30:70, or 45:55 to 30:70.
[6] The composition according to any of [1] to [5] above, wherein the phospholipid is phosphatidylcholine or phosphatidylethanolamine.
[7] The composition according to any of [1] to [6] above, wherein the phospholipid is selected from the group consisting of soybean phosphatidylcholine, egg yolk phosphatidylcholine, dimyristoyl phosphatidylcholine, dioleyl phosphatidylcholine, and dioleyl phosphatidylethanolamine.
[8] The composition according to any of [1] to [7] above, wherein R in the general formula (I) denotes a hydrophilic group generated by removal of one hydroxyl group from any one selected from the group consisting of glycerol, erythritol, pentaerythritol, diglycerol, sorbitan, isosorbide, and glycol.
[9] The composition according to [1] or [2] above, wherein the amphipathic compound is selected from the group consisting of the following:

mono-O-(5,9,13,17-tetramethyloctadec-4-enoyl)glycerol,
mono-O-(5,9,13,17-tetramethyloctadecanoyl)glycerol,
mono-O-(5,9,13,17-tetramethyloctadeca-4,8,12,16-tetraenoyl)glycerol,
mono-O-(5,9,13,17-tetramethyloctadecanoyl)erythritol,
mono-O-(5,9,13,17-tetramethyloctadec-4-enoyl)pentaerythritol,
mono-O-(5,9,13,17-tetramethyloctadecanoyl)pentaerythritol,
mono-O-(5,9,13,17-tetramethyloctadec-4-enoyl)diglycerol,
mono-O-(5,9,13,17-tetramethyloctadec-4-enoyl)sorbitan,
mono-O-(5,9,13,17-tetramethyloctadecanoyl)sorbitan,

mono-O-(5,9,13,17-tetramethyloctadec-4-enoyl)isosorbide,
mono-O-(5,9,13,17-tetramethyloctadecanoyl)isosorbide,
mono-O-(5,9,13-trimethyltetradec-4-enoyl)glycerol,
mono-O-(5,9,13-trimethyltetradec-4-enoyl)sorbitan,
mono-O-(5,9,13-trimethyltetradecanoyl)sorbitan,
mono-O-(5,9,13-trimethyltetradec-4-enoyl)propylene glycol,
mono-O-(5,9,13,17-tetramethyloctadec-4-enoyl)propylene glycol,
mono-O-(5,9,13,17-tetramethyloctadecanoyl)propylene glycol,
mono-O-(4,8,12,16-tetramethylheptadec-3-enoyl)sorbitan,
mono-O-(4,8,12,16-tetramethylheptadec-3-enoyl)isosorbide,
mono-O-(4,8,12,16-tetramethylheptadec-3-enoyl)propylene glycol,
mono-O-(5,9,13,17-tetramethyloctadec-4-enoyl)ethylene glycol,
mono-O-(5,9,13,17-tetramethyloctadec-4-enoyl)1,3-butylene glycol, and
mono-O-(5,9,13,17-tetramethyloctadec-4-enoyl)3-methyl-1,3-butanediol.

[10] The composition according to any of [1] to [9] above, wherein the increased biocompatibility is indicated by reduction or disappearance of a toxicity as compared with a control composition comprising no phospholipid.
[11] The composition according to any of [1] to [10] above, further comprising at least one of an oil and an organic solvent.
[12] The composition according to any of [1] to [11] above, wherein the composition further comprises an aqueous medium and is a non-lamellar liquid crystal composition.
[13] The composition according to [12] above, wherein the composition further comprises a surfactant and is a non-lamellar liquid crystal emulsion composition.
[14] The composition according to any of [1] to [11] above, wherein the composition comprises no aqueous medium and is a liquid crystal precursor composition capable of forming a non-lamellar liquid crystal in the presence of an aqueous medium.
[15] A pharmaceutical formulation comprising the composition according to any of [1] to [14] above.
[16] The pharmaceutical formulation according to [15] above for adhesion prevention of living tissue.
[17] The pharmaceutical formulation according to [15] above, wherein the composition further comprises a drug and is a sustained release formulation.
[18] The pharmaceutical formulation according to [17] above, wherein the drug is a gonadotropin-releasing hormone (GnRH) agonist.
[19] The pharmaceutical formulation according to [18] above, wherein the GnRH agonist is leuprolide or a salt thereof.
[20] The pharmaceutical formulation according to any of [15] to [19] above, wherein the pharmaceutical formulation is a spray formulation, an aerosol formulation, an injection, or a depot formulation.
[21] An amphipathic compound represented by the following general formula (I') or a salt thereof:

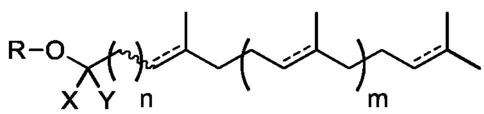

(I')

wherein X and Y each denotes a hydrogen atom or together denote an oxygen atom, n denotes the integer 1 or 2, m denotes the integer 1 or 2, the designation:

denotes a single bond or double bond, and R denotes a hydrophilic group generated by removal of one hydroxyl group from any one selected from the group consisting of sorbitan, isosorbide, and glycol.

[22] The compound or the salt thereof according to [21] above, wherein n = 2 in the general formula (I').
[23] The compound or the salt thereof according to [21] or [22] above, wherein the amphipathic compound is selected from the group consisting of the following:

mono-O-(5,9,13,17-tetramethyloctadec-4-enoyl)sorbitan,
mono-O-(5,9,13,17-tetramethyloctadecanoyl)sorbitan,
mono-O-(5,9,13-trimethyltetradec-4-enoyl)sorbitan,
mono-O-(5,9,13-trimethyltetradecanoyl)sorbitan,
mono-O-(5,9,13,17-tetramethyloctadec-4-enoyl)isosorbide,

mono-O-(5,9,13,17-tetramethyloctadecanoyl)isosorbide,
mono-O-(5,9,13-trimethyltetradec-4-enoyl)propylene glycol,
mono-O-(5,9,13,17-tetramethyloctadec-4-enoyl)propylene glycol,
mono-O-(5,9,13,17-tetramethyloctadecanoyl)propylene glycol,
mono-O-(4,8,12,16-tetramethylheptadec-3-enoyl)sorbitan,
mono-O-(4,8,12,16-tetramethylheptadec-3-enoyl)isosorbide,
mono-O-(4,8,12,16-tetramethylheptadec-3-enoyl)propylene glycol,
mono-O-(5,9,13,17-tetramethyloctadec-4-enoyl)ethylene glycol,
mono-O-(5,9,13,17-tetramethyloctadec-4-enoyl)1,3-butylene glycol, and
mono-O-(5,9,13,17-tetramethyloctadec-4-enoyl)3-methyl-1,3-butanediol.

The present application encompasses the disclosures in Japanese Patent Application Nos. 2020-011237 and 2020-211758 from which the present application claims priority.

Advantageous Effects of Invention

The present invention can provide a highly safe non-lamellar liquid crystal-forming composition. The present invention can also provide a non-lamellar liquid crystal-forming composition using a novel amphipathic compound.

Brief Description of Drawings

[Figure 1] Figure 1 is photographs showing the appearance of abscess developed by the administration of a formulation. Figures 1A to 1C: a precursor formulation of formulation No. 1. Figure 1D: a precursor formulation of formulation No. 71. Figure 1A shows abscess developed along a sutured incision wound, Figure 1B shows a part of abscess isolated from the inside, Figure 1C shows an abscess development site after isolation of abscess (partial), Figure ID shows abscess developed along a sutured incision wound. The arrows depict abscess (partial).
[Figure 2] Figure 2 shows the release rate of leuprolide acetate from each precursor formulation. As a phospholipid, DMPC is contained in precursor formulation No. 121, DOPC is contained in precursor formulation No. 122, DOPE is contained in precursor formulation No. 123, and DOPG-Na is contained in precursor formulation No. 124. Aqueous solution No. 125 contains neither C17 glycerin ester nor a phospholipid.
[Figure 3] Figure 3 is photographs showing results of a subcutaneous implantation test using precursor formulations containing DMPC (Figure 3A) or DOPC (Figure 3B) as a phospholipid.
[Figure 4] Figure 4 shows the release rate of FD-4 from each precursor formulation. The values denote mean ± S.D. (n = 3).
[Figure 5] Figure 5 shows the release rate of leuprolide acetate from each precursor formulation. The values denote mean ± S.D. (n = 3).
[Figure 6] Figure 6 is photographs showing results of a subcutaneous implantation test using emulsions or precursor formulations containing an isoprenoid lipid and a phospholipid.
[Figure 7] Figure 7 is photographs showing results of a subcutaneous implantation test using precursor formulations containing an isoprenoid lipid and a phospholipid.

Description of Embodiments

Hereinafter, the present invention will be described in detail.
The present invention relates to a composition, particularly, a non-lamellar liquid crystal-forming composition, comprising an amphipathic compound having a predetermined isoprenoid fatty acid chain. In one embodiment, the present invention relates to a non-lamellar liquid crystal-forming composition comprising an amphipathic compound having a predetermined isoprenoid fatty acid chain, and a phospholipid. In particular, the non-lamellar liquid crystal-forming composition according to the present invention is preferably a non-lamellar liquid crystal-forming composition having an increased biocompatibility by a phospholipid. Such a non-lamellar liquid crystal-forming composition is highly safe and therefore permits *in vivo* application.
In the present invention, an amphipathic compound having an isoprenoid fatty acid chain (an isoprenoid lipid), represented by the general formula (I) given below can be used in combination with a phospholipid in the production of a non-lamellar liquid crystal-forming composition. The present invention provides a composition, particularly, a non-lamellar liquid crystal-forming composition, comprising an amphipathic compound represented by the general formula (I) and a phospholipid.

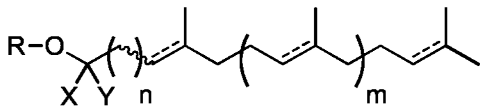

(I)

In one embodiment, in the general formula (I), X and Y each denotes a hydrogen atom or together denote an oxygen atom, n denotes the integer 1 or 2 (n = 1 or 2), and m denotes the integer 1 or 2 (m = 1 or 2). The combination of n and m may be: n = 1 and m = 1; n = 1 and m = 2; n = 2 and m = 1; or n = 2 and m = 2. The combination is preferably n = 2 and m = 1, or n = 2 and m = 2, more preferably n = 2 and m = 2. In one embodiment, in the general formula (I), X and Y together denote an oxygen atom, and n = 2 and m = 2, or n = 2 and m = 1.

In another embodiment, in the general formula (I) and the general formulas (II) to (IV) mentioned later, X and Y each denotes a hydrogen atom or together denote an oxygen atom, n denotes an integer from 0 to 2, and m denotes the integer 1 or 2.

In the present invention, the designation:



in the chemical formula that represents the amphipathic compound denotes a single bond or double bond.

R in the general formula (I) denotes a hydrophilic group having one, or two or more (e.g., two, three, four, or five) hydroxyl groups. R in the general formula (I) may be a residue generated by removal of one hydroxyl group (OH) from polyol. R in the general formula (I) may have an ether bond and/or a cyclic structure. R in the general formula (I) may be, but not limited to, for example, a hydrophilic group generated by removal of one hydroxyl group (OH) from any one selected from the group consisting of glycerol, erythritol, pentaerythritol, diglycerol, glyceric acid, triglycerol, xylose, sorbitol, ascorbic acid, glucose, galactose, mannose, dipentaerythritol, maltose, mannitol, xylitol, sorbitan, isosorbide, and glycol (which may be, but not limited to, for example, propylene glycol, ethylene glycol, diethylene glycol, butylene glycol such as 1,3-butylene glycol, and isoprene glycol). Isoprene glycol is also called 3-methyl-1,3-butanediol. R in the general formula (I) is more preferably a hydrophilic group generated by removal of one hydroxyl group (OH) from glycerol, erythritol, pentaerythritol, diglycerol, sorbitan, isosorbide, or glycol (e.g., propylene glycol, ethylene glycol, butylene glycol such as 1,3-butylene glycol, or isoprene glycol). In one embodiment, R in the general formula (I) may be, for example, a hydrophilic group having one hydroxyl group generated by removal of one hydroxyl group (OH) from glycol having no ether bond, and the amphipathic compound represented by the thus defined general formula (I) is not a self-organizing lipid (SOL).

In the present invention, the designation:

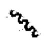

in the chemical formula that represents the amphipathic compound means that the amphipathic compound is an E-(cis-) or Z-(trans-) geometric isomer, or a mixture thereof.

An example of the amphipathic compound represented by the general formula (I) is an amphipathic compound represented by the following general formula (II):

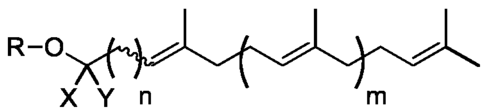

(II)

In the general formula (II), X and Y each denotes a hydrogen atom or together denote an oxygen atom, n denotes the integer 1 or 2 (n = 1 or 2), and m denotes the integer 1 or 2 (m = 1 or 2). The combination of n and m may be: n = 1 and m = 1; n = 1 and m = 2; n = 2 and m = 1; or n = 2 and m = 2. The combination is preferably n = 2 and m = 1, or n = 2 and m = 2, more preferably n = 2 and m = 2. In one embodiment, in the general formula (II), X and Y together denote an oxygen atom, and n = 2 and m = 2, or n = 2 and m = 1.

R in the general formula (II) denotes a hydrophilic group having one, or two or more hydroxyl groups. R in the general formula (II) may be the same as R in the general formula (I). R in the general formula (II) may be, but not limited to, for example, a hydrophilic group generated by removal of one hydroxyl group (OH) from any one selected from the group consisting of glycerol, erythritol, pentaerythritol, diglycerol, glyceric acid, triglycerol, xylose, sorbitol, ascorbic acid, glucose, galactose, mannose, dipentaerythritol, maltose, mannitol, xylitol, sorbitan, isosorbide, and glycol (which may

be, but not limited to, for example, propylene glycol, ethylene glycol, diethylene glycol, butylene glycol such as 1,3-butylene glycol, and isoprene glycol). R in the general formula (II) is more preferably a hydrophilic group generated by removal of one hydroxyl group (OH) from glycerol, erythritol, pentaerythritol, diglycerol, sorbitan, isosorbide, or glycol (e.g., propylene glycol, ethylene glycol, butylene glycol such as 1,3-butylene glycol, or isoprene glycol). In one embodiment, R in the general formula (II) may be, for example, a hydrophilic group having one hydroxyl group generated by removal of one hydroxyl group (OH) from glycol having no ether bond.

Another example of the amphipathic compound represented by the general formula (I) is an amphipathic compound represented by the following general formula (III):

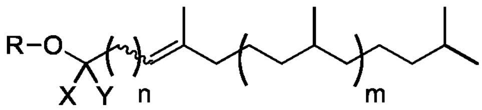

(III)

In the general formula (III), X and Y each denotes a hydrogen atom or together denote an oxygen atom, n denotes the integer 1 or 2 (n = 1 or 2), and m denotes the integer 1 or 2 (m = 1 or 2). The combination of n and m may be: n = 1 and m = 1; n = 1 and m = 2; n = 2 and m = 1; or n = 2 and m = 2. The combination is preferably n = 2 and m = 1, or n = 2 and m = 2, more preferably n = 2 and m = 2. In one embodiment, in the general formula (III), X and Y together denote an oxygen atom, and n = 2 and m = 2; or n = 2 and m = 1.

R in the general formula (III) denotes a hydrophilic group having one, or two or more hydroxyl groups. R in the general formula (III) may be the same as R in the general formula (I). R in the general formula (III) may be, but not limited to, for example, a hydrophilic group generated by removal of one hydroxyl group (OH) from any one selected from the group consisting of glycerol, erythritol, pentaerythritol, diglycerol, glyceric acid, triglycerol, xylose, sorbitol, ascorbic acid, glucose, galactose, mannose, dipentaerythritol, maltose, mannitol, xylitol, sorbitan, isosorbide, and glycol (which may be, but not limited to, for example, propylene glycol, ethylene glycol, diethylene glycol, butylene glycol such as 1,3-butylene glycol, and isoprene glycol). R in the general formula (III) is more preferably a hydrophilic group generated by removal of one hydroxyl group (OH) from glycerol, erythritol, pentaerythritol, diglycerol, sorbitan, isosorbide, or glycol (e.g., propylene glycol, ethylene glycol, butylene glycol such as 1,3-butylene glycol, or isoprene glycol). In one embodiment, R in the general formula (II) may be, for example, a hydrophilic group having one hydroxyl group generated by removal of one hydroxyl group (OH) from glycol having no ether bond.

A further alternative example of the amphipathic compound represented by the general formula (I) is an amphipathic compound represented by the following general formula (IV):

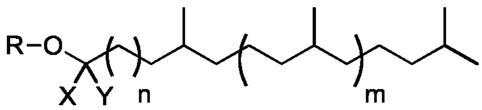

(IV)

In the general formula (IV), X and Y each denotes a hydrogen atom or together denote an oxygen atom, n denotes the integer 1 or 2 (n = 1 or 2), and m denotes the integer 1 or 2 (m = 1 or 2). The combination of n and m may be: n = 1 and m = 1; n = 1 and m = 2; n = 2 and m = 1; or n = 2 and m = 2. The combination is preferably n = 2 and m = 1, or n = 2 and m = 2, more preferably n = 2 and m = 2. In one embodiment, in the general formula (IV), X and Y together denote an oxygen atom, and n = 2 and m = 2; or n = 2 and m = 1.

R in the general formula (IV) denotes a hydrophilic group having one, or two or more hydroxyl groups. R in the general formula (IV) may be the same as R in the general formula (I). R in the general formula (IV) may be, but not limited to, for example, a hydrophilic group generated by removal of one hydroxyl group (OH) from any one selected from the group consisting of glycerol, erythritol, pentaerythritol, diglycerol, glyceric acid, triglycerol, xylose, sorbitol, ascorbic acid, glucose, galactose, mannose, dipentaerythritol, maltose, mannitol, xylitol, sorbitan, isosorbide, and glycol (which may be, but not limited to, for example, propylene glycol, ethylene glycol, diethylene glycol, butylene glycol such as 1,3-butylene glycol, and isoprene glycol). R in the general formula (IV) is more preferably a hydrophilic group generated by removal of one hydroxyl group (OH) from glycerol, erythritol, pentaerythritol, diglycerol, sorbitan, isosorbide, or glycol (e.g., propylene glycol, ethylene glycol, butylene glycol such as 1,3-butylene glycol, or isoprene glycol). In one embodiment, R in the general formula (IV) may be, for example, a hydrophilic group having one hydroxyl group generated by removal of one hydroxyl group (OH) from glycol having no ether bond.

Preferred examples of the amphipathic compound represented by the general formula (I) to be used in the present invention include, but are not limited to,

mono-O-(5,9,13,17-tetramethyloctadec-4-enoyl)glycerol,

mono-O-(5,9,13,17-tetramethyloctadecanoyl)glycerol,
mono-O-(5,9,13,17-tetramethyloctadeca-4,8,12,16-tetraenoyl)glycerol,
mono-O-(5,9,13,17-tetramethyloctadecanoyl)erythritol,
mono-O-(5,9,13,17-tetramethyloctadec-4-enoyl)pentaerythritol,
mono-O-(5,9,13,17-tetramethyloctadecanoyl)pentaerythritol,
mono-O-(5,9,13,17-tetramethyloctadec-4-enoyl)diglycerol,
mono-O-(5,9,13,17-tetramethyloctadec-4-enoyl)sorbitan,
mono-O-(5,9,13,17-tetramethyloctadecanoyl)sorbitan,
mono-O-(5,9,13,17-tetramethyloctadec-4-enoyl)isosorbide,
mono-O-(5,9,13,17-tetramethyloctadecanoyl)isosorbide
mono-O-(5,9,13-trimethyltetradec-4-enoyl)glycerol,
mono-O-(5,9,13-trimethyltetradec-4-enoyl)sorbitan,
mono-O-(5,9,13-trimethyltetradecanoyl)sorbitan,
mono-O-(5,9,13-trimethyltetradec-4-enoyl)propylene glycol,
mono-O-(5,9,13,17-tetramethyloctadec-4-enoyl)propylene glycol,
mono-O-(5,9,13,17-tetramethyloctadecanoyl)propylene glycol,
mono-O-(4,8,12,16-tetramethylheptadec-3-enoyl)sorbitan,
mono-O-(4,8,12,16-tetramethylheptadec-3-enoyl)isosorbide,
mono-O-(4,8,12,16-tetramethylheptadec-3-enoyl)propylene glycol,
mono-O-(5,9,13,17-tetramethyloctadec-4-enoyl)ethylene glycol,
mono-O-(5,9,13,17-tetramethyloctadec-4-enoyl)1,3-butylene glycol, and
mono-O-(5,9,13,17-tetramethyloctadec-4-enoyl)3-methyl-1,3-butanediol.

Other examples of the amphipathic compound represented by the general formula (I) include, but are not limited to,

mono-O-(3,7,11,15-tetramethylhexadec-2-enoyl)sorbitan,
mono-O-(3,7,11,15-tetramethylhexadec-2-enoyl)isosorbide,
mono-O-(3,7,11,15-tetramethylhexadecanoyl)sorbitan,
mono-O-(3,7,11,15-tetramethylhexadec-2-enoyl)propylene glycol, and
mono-O-(3,7,11,15-tetramethylhexadecanoyl)propylene glycol.

In a preferred embodiment, the amphipathic compound represented by the general formula (I) used in the present invention may exhibit a low viscosity in itself. Specifically, the amphipathic compound represented by the general formula (I) has a viscosity of preferably 15.0 Pa·s or less, more preferably 11.0 Pa·s or less, further preferably 6.0 Pa·s or less, by itself, as measured at 25°C. This viscosity can be measured using, for example, a viscosity and viscoelasticity measuring apparatus (Gemini II, Malvern Instruments Ltd.) at a temperature of 25°C.

The non-lamellar liquid crystal-forming composition according to the present invention may comprise one, or two or more amphipathic compounds having an isoprenoid fatty acid chain, represented by the general formula (I). When the non-lamellar liquid crystal-forming composition according to the present invention comprises a plurality of amphipathic compounds having an isoprenoid fatty acid chain, the weight ratio between these amphipathic compounds is not particularly limited. However, in one embodiment, the non-lamellar liquid crystal-forming composition according to the present invention may comprise mono-O-(5,9,13-trimethyltetradec-4-enoyl)sorbitan and mono-O-(5,9,13-trimethyltetradec-4-enoyl)isosorbide at a weight ratio of 50:50 to 99:1, preferably 60:40 to 90:10, 70:30 to 90:10, or 80:20 to 90:10, for example, 8:2 or 85:15. In one embodiment, the non-lamellar liquid crystal-forming composition according to the present invention may comprise mono-O-(5,9,13-trimethyltetradecanoyl)sorbitan and mono-O-(5,9,13-trimethyltetradecanoyl)isosorbide at a weight ratio of 50:50 to 99:1, preferably 60:40 to 90:10, 70:30 to 90:10, or 80:20 to 90:10, for example, 8:2 or 85:15. In one embodiment, the non-lamellar liquid crystal-forming composition according to the present invention may comprise mono-O-(5,9,13,17-tetramethyloctadec-4-enoyl)sorbitan and mono-O-(5,9,13,17-tetramethyloctadec-4-enoyl)isosorbide at a weight ratio of 50:50 to 99:1, preferably 60:40 to 90:10, 70:30 to 90:10, or 80:20 to 90:10, for example, 8:2 or 85:15. In one embodiment, the non-lamellar liquid crystal-forming composition according to the present invention may comprise mono-O-(5,9,13,17-tetramethyloctadecanoyl)sorbitan and mono-O-(5,9,13,17-tetramethyloctadecanoyl)isosorbide at a weight ratio of 50:50 to 99:1, preferably 60:40 to 90:10, 70:30 to 90:10, or 80:20 to 90:10, for example, 8:2 or 85:15. In one embodiment, the non-lamellar liquid crystal-forming composition according to the present invention may comprise 2-O-(5,9,13,17-tetramethyloctadec-4-enoyl)isosorbide and 5-O-(5,9,13,17-tetramethyloctadec-4-enoyl)isosorbide at a weight ratio of 50:50 to 99:1, preferably 50:50 to 70:30, or 55:45 to 65:35, for example, 60:40. In one embodiment, the non-lamellar liquid crystal-forming composition according to the present invention may comprise mono-O-(4,8,12,16-tetramethylheptadec-3-enoyl)sorbitan and mono-O-(4,8,12,16-tetramethylheptadec-3-enoyl)isosorbide at a weight ratio of 50:50 to 99:1, preferably 60:40 to 90:10, 70:30 to 90:10, or 80:20 to 90:10, for example, 8:2 or 85:15. In one embodiment, the non-lamellar liquid crystal-forming composition may comprise mono-O-(3,7,11,15-tetramethylhexadec-2-enoyl)sorbitan and mono-O-(3,7,11,15-tetramethylhexadec-2-enoyl)isosorbide at a weight ratio of 50:50 to 99:1, preferably 60:40 to 90:10, 70:30 to 90:10, or 80:20 to 90:10, for example, 8:2 or 85:15.

The present invention also provides a newly found amphipathic compound represented by the general formula (I') given below, which is included in the scope of the amphipathic compound represented by the general formula (I). The amphipathic compound represented by the general formula (I') can also be suitably used in combination with a phospholipid in the production of a non-lamellar liquid crystal-forming composition.

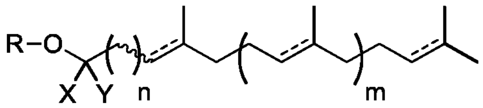

(I')

In one embodiment, in the general formula (I'), X and Y each denotes a hydrogen atom or together denote an oxygen atom, n denotes the integer 1 or 2, preferably n = 2, m denotes the integer 1 or 2, the designation: denotes a single bond or double bond, and R denotes a hydrophilic group generated by removal of one hydroxyl group from sorbitan, isosorbide, or glycol (which may be, but not limited to, for example, propylene glycol, ethylene glycol, diethylene glycol, butylene glycol such as 1,3-butylene glycol, and isoprene glycol).

In the general formula (I'), the combination of n and m may be: n = 1 and m = 1; n = 1 and m = 2; n = 2 and m= 1; or n = 2 and m = 2. In one embodiment, in the general formula (I'), X and Y together denote an oxygen atom, and n = 2 and m = 2, or n = 2 and m = 1.

In another embodiment, in the general formula (I'), X and Y each denotes a hydrogen atom or together denote an oxygen atom, n denotes an integer from 0 to 2, m denotes the integer 1 or 2, the designation: denotes a single bond or double bond, and R may denote a hydrophilic group generated by removal of one hydroxyl group from sorbitan, isosorbide, or glycol (e.g., propylene glycol). The combination of n and m may be: n = 0 and m = 1; n = 0 and m = 2; n = 1 and m = 1; n = 1 and m = 2; n = 2 and m = 1; or n = 2 and m = 2. In one embodiment, in the general formula (I'), X and Y together denote an oxygen atom, and n = 2 and m = 2, or n = 2 and m = 1.

The amphipathic compound represented by the general formula (I') may be a compound represented by the general formula (II), (III) or (IV) described above wherein X and Y each denotes a hydrogen atom or together denote an oxygen atom, n denotes an integer from 0 to 2 (preferably, n = 1 or 2), m denotes the integer 1 or 2, and R may denote a hydrophilic group generated by removal of one hydroxyl group from sorbitan, isosorbide, or glycol (e.g., propylene glycol).

Preferred examples of the amphipathic compound represented by the general formula (I') include, but are not limited to,

mono-O-(5,9,13,17-tetramethyloctadec-4-enoyl)sorbitan,
mono-O-(5,9,13,17-tetramethyloctadecanoyl)sorbitan,
mono-O-(5,9,13-trimethyltetradec-4-enoyl)sorbitan,
mono-O-(5,9,13 -trimethyltetradecanoyl)sorbitan,
mono-O-(5,9,13,17-tetramethyloctadec-4-enoyl)isosorbide,
mono-O-(5,9,13,17-tetramethyloctadecanoyl)isosorbide,
mono-O-(5,9,13-trimethyltetradec-4-enoyl)propylene glycol,
mono-O-(5,9,13,17-tetramethyloctadec-4-enoyl)propylene glycol,
mono-O-(5,9,13,17-tetramethyloctadecanoyl)propylene glycol,
mono-O-(4,8,12,16-tetramethylheptadec-3-enoyl)sorbitan,
mono-O-(4,8,12,16-tetramethylheptadec-3-enoyl)isosorbide,
mono-O-(4,8,12,16-tetramethylheptadec-3-enoyl)propylene glycol,
mono-O-(5,9,13,17-tetramethyloctadec-4-enoyl)ethylene glycol,
mono-O-(5,9,13,17-tetramethyloctadec-4-enoyl)1,3-butylene glycol, and
mono-O-(5,9,13,17-tetramethyloctadec-4-enoyl)3-methyl-1,3-butanediol.

The non-lamellar liquid crystal-forming composition according to the present invention comprises an amphipathic compound represented by the general formula (I). The amphipathic compound represented by the general formula (I) encompasses an amphipathic compound represented by the general formula (I'). The non-lamellar liquid crystal-forming composition according to the present invention may comprise the amphipathic compound represented by the general formula (I) or (I') in the form of a salt thereof. The salt of the amphipathic compound represented by the general formula (I) or the general formula (I') according to the present invention may be any type of salt, including, but not limited to, salts of alkali metals and alkaline-earth metals such as sodium, potassium, calcium, and magnesium. The salt of the amphipathic compound represented by the general formula (I) or the general formula (I') according to the present invention may be a pharmaceutically acceptable salt. The non-lamellar liquid crystal-forming composition according to the present invention comprising the amphipathic compound represented by the general formula (I) or the general formula (I') according to the present invention in the form of a salt thereof is also included in the scope of the non-lamellar liquid crystal-forming composition comprising the amphipathic compound represented by the general formula (I) or the general formula (I') according to the present invention.

The amphipathic compound represented by the general formula (I) according to the present invention can be used in combination with a phospholipid. When the amphipathic compound of the general formula (I) has a toxicity, the phospholipid reduces the toxicity of the amphipathic compound and increases the biocompatibility of the non-lamellar liquid crystal-forming composition so that the non-lamellar liquid crystal-forming composition can be highly safe when applied into a living body. Specifically, the non-lamellar liquid crystal-forming composition comprising the amphipathic compound represented by the general formula (I) and the phospholipid according to the present invention preferably has a biocompatibility increased by the phospholipid as compared with a control composition comprising no phospholipid. In the present invention, the "biocompatibility" refers to a property of causing little or no adverse reaction (side effect) in a living body in *in vivo* application. The increased biocompatibility by the phospholipid in the non-lamellar liquid crystal-forming composition comprising the amphipathic compound represented by the general formula (I) and the phospholipid according to the present invention can be confirmed by reduction or disappearance of a toxicity in *in vivo* application of the composition, as compared with a control composition comprising no phospholipid. The "control composition comprising no phospholipid" in the context of the present invention means a non-lamellar liquid crystal-forming composition having the same composition as a test non-lamellar liquid crystal-forming composition except that no phospholipid is contained. Examples of the "toxicity" in the context of the present invention include, but are not limited to, systemic toxicities such as hepatotoxicity, and local toxicities which cause foreign body responses such as abscess development or tissue damages such as bleeding or discoloration. The hepatotoxicity can be shown by the occurrence of at least one symptom that manifests liver damage, selected from, for example, occurrence of ascites, enlargement of the liver, perihepatic adhesion (particularly, adhesion at a non-injured or non-inflammatory site), and whitening of the liver. In one embodiment, the reduction or disappearance of a toxicity of the non-lamellar liquid crystal-forming composition of the present invention may bring about decrease in death rate after *in vivo* application of the non-lamellar liquid crystal-forming composition, as compared with a control composition comprising no phospholipid, in small laboratory animals including rodents such as mice and rats. The non-lamellar liquid crystal-forming composition comprising the amphipathic compound represented by the general formula (I) and a phospholipid according to the present invention permits *in vivo* application (preferably, parenteral administration such as intraperitoneal administration, intramuscular administration, or subcutaneous administration). The phrase "permit *in vivo* application" in the context of the present invention means that administration into a living body (typically, parenteral administration such as intraperitoneal administration, intramuscular administration, or subcutaneous administration into the body) causes no toxicity or causes a mere low toxicity to a pharmaceutically acceptable extent. However, the non-lamellar liquid crystal-forming composition according to the present invention is not limited to one intended for *in vivo* application.

The phospholipid used in the present invention may be, but not limited to, for example, one, or two or more phospholipids selected from the group consisting of phosphatidylcholine, phosphatidylethanolamine, phosphatidylserine, phosphatidylinositol, phosphatidylglycerin, phosphatidic acid, and sphingomyelin, and salts thereof; or a phospholipid formulation or fraction containing the phospholipid(s). Examples of phosphatidylcholine include, but are not limited to, dioleyl phosphatidylcholine (DOPC), dimyristoyl phosphatidylcholine (DMPC), dipalmitoyl phosphatidylcholine (DPPC), soybean phosphatidylcholine (SPC; also called soybean lecithin), and egg yolk phosphatidylcholine (EPC; also called egg yolk lecithin). Examples of phosphatidylethanolamine include, but are not limited to, dioleyl phosphatidylethanolamine (DOPE). Examples of phosphatidylglycerin include, but are not limited to, dioleyl phosphatidylglycerin, and examples of salts of phosphatidylglycerin include, but are not limited to, sodium dioleyl phosphatidylglycerin (DOPG-Na). The phospholipid used in the present invention may be a synthetic product or may be naturally derived. In one embodiment, the phospholipid used in the present invention may be phosphatidylcholine, for example, soybean phosphatidylcholine or egg yolk phosphatidylcholine. In another embodiment, the phospholipid used in the present invention may be phosphatidylcholine, phosphatidylethanolamine, or phosphatidylglycerin, or a salt thereof. In one embodiment, the phospholipid used in the present invention may be selected from the group consisting of soybean phosphatidylcholine (SPC), and egg yolk phosphatidylcholine (EPC), dimyristoyl phosphatidylcholine (DMPC), dioleyl phosphatidylcholine (DOPC), and dioleyl phosphatidylethanolamine (DOPE). The non-lamellar liquid crystal-forming composition according to the present invention can comprise one, or two or more phospholipids.

For the purpose of increasing the biocompatibility of the amphipathic compound so that the non-lamellar liquid

crystal-forming composition is highly safe, the weight ratio between the amphipathic compound (isoprenoid-type) represented by the general formula (I) and the phospholipid contained in the non-lamellar liquid crystal-forming composition according to the present invention is, but not limited to, preferably 90:10 to 10:90 of amphipathic compound:phospholipid and may be, for example, 80:20 to 20:80, 80:20 to 30:70, 70:30 to 10:90, 70:30 to 20:80, 70:30 to 30:70, 60:40 to 10:90, 60:40 to 20:80, 60:40 to 30:70, 60:40 to 40:60, 45:55 to 10:90, 45:55 to 20:80, 45:55 to 30:70, 45:55 to 35:65, 45:55 to 55:45, 50:50 to 20:80, 50:50 to 30:70, 40:60 to 10:90, 40:60 to 20:80, 40:60 to 30:70, 35:65 to 20:80, or 35:65 to 25:75 of amphipathic compound:phospholipid.

In one embodiment, a non-lamellar liquid crystal-forming composition comprising an amphipathic compound represented by the general formula (I) (wherein n = 2 and m = 2, and R denotes a hydrophilic group generated by removal of one hydroxyl group from any one selected from the group consisting of glycerol, erythritol, pentaerythritol, diglycerol, sorbitan, isosorbide, and glycol (which may be, but not limited to, for example, propylene glycol, ethylene glycol, diethylene glycol, butylene glycol such as 1,3-butylene glycol, and isoprene glycol)), and a phospholipid preferably has a weight ratio of amphipathic compound:phospholipid of 90:10 to 10:90 and may have a weight ratio of amphipathic compound:phospholipid of 80:20 to 20:80, 80:20 to 30:70, 70:30 to 10:90, 70:30 to 20:80, 70:30 to 30:70, 60:40 to 10:90, 60:40 to 20:80, 60:40 to 30:70, 60:40 to 40:60, 45:55 to 10:90, 45:55 to 20:80, 45:55 to 30:70, 45:55 to 35:65, 45:55 to 55:45, 50:50 to 20:80, 50:50 to 30:70, 40:60 to 10:90, 40:60 to 20:80, 40:60 to 30:70, 35:65 to 20:80, or 35:65 to 25:75.

In one embodiment, a non-lamellar liquid crystal-forming composition comprising an amphipathic compound represented by the general formula (I) (wherein n = 1 and m = 2, and R denotes a hydrophilic group generated by removal of one hydroxyl group from any one selected from the group consisting of glycerol, erythritol, pentaerythritol, diglycerol, sorbitan, isosorbide, and glycol (which may be, but not limited to, for example, propylene glycol, ethylene glycol, diethylene glycol, butylene glycol such as 1,3-butylene glycol, and isoprene glycol)), and a phospholipid preferably has a weight ratio of amphipathic compound:phospholipid of 90:10 to 10:90 and may have a weight ratio of amphipathic compound:phospholipid of 80:20 to 20:80, 80:20 to 30:70, 70:30 to 10:90, 70:30 to 20:80, 70:30 to 30:70, 60:40 to 10:90, 60:40 to 20:80, 60:40 to 30:70, 60:40 to 40:60, 45:55 to 10:90, 45:55 to 20:80, 45:55 to 30:70, 45:55 to 35:65, 45:55 to 55:45, 50:50 to 20:80, 50:50 to 30:70, 40:60 to 10:90, 40:60 to 20:80, 40:60 to 30:70, 35:65 to 20:80, or 35:65 to 25:75.

In one embodiment, a non-lamellar liquid crystal-forming composition comprising an amphipathic compound represented by the general formula (I) (wherein n = 2 and m = 1, and R denotes a hydrophilic group generated by removal of one hydroxyl group from any one selected from the group consisting of glycerol, erythritol, pentaerythritol, diglycerol, sorbitan, isosorbide, and glycol (which may be, but not limited to, for example, propylene glycol, ethylene glycol, diethylene glycol, butylene glycol such as 1,3-butylene glycol, and isoprene glycol)), and a phospholipid preferably has a weight ratio of amphipathic compound:phospholipid of 90:10 to 10:90 and may have a weight ratio of amphipathic compound:phospholipid of 80:20 to 20:80, 80:20 to 30:70, 70:30 to 10:90, 70:30 to 20:80, 70:30 to 30:70, 60:40 to 10:90, 60:40 to 20:80, 60:40 to 30:70, 60:40 to 40:60, 45:55 to 10:90, 45:55 to 20:80, 45:55 to 30:70, 45:55 to 35:65, 45:55 to 55:45, 50:50 to 20:80, 50:50 to 30:70, 40:60 to 10:90, 40:60 to 20:80, 40:60 to 30:70, 35:65 to 20:80, or 35:65 to 25:75.

In one embodiment, a non-lamellar liquid crystal-forming composition comprising an amphipathic compound represented by the general formula (I) (wherein n = an integer from 0 to 2 and m = 1 or 2, and R denotes a hydrophilic group generated by removal of one hydroxyl group from sorbitan, isosorbide, or glycol (which may be, but not limited to, for example, propylene glycol, ethylene glycol, diethylene glycol, butylene glycol such as 1,3-butylene glycol, and isoprene glycol)), and a phospholipid preferably has a weight ratio of amphipathic compound:phospholipid of 90:10 to 10:90 and may have a weight ratio of amphipathic compound:phospholipid of 80:20 to 20:80, 80:20 to 30:70, 70:30 to 10:90, 70:30 to 20:80, 70:30 to 30:70, 60:40 to 10:90, 60:40 to 20:80, 60:40 to 30:70, 60:40 to 40:60, 45:55 to 10:90, 45:55 to 20:80, 45:55 to 30:70, 45:55 to 35:65, 45:55 to 55:45, 50:50 to 20:80, 50:50 to 30:70, 40:60 to 10:90, 40:60 to 20:80, 40:60 to 30:70, 35:65 to 20:80, or 35:65 to 25:75.

In the case of using a plurality of the amphipathic compounds (isoprenoid-type) represented by the general formula (I), the above-mentioned weight ratio between the amphipathic compound and the phospholipid is calculated using a total amount (weight) of the amphipathic compounds used. In the case of using a plurality of phospholipids, the above-mentioned weight ratio between the amphipathic compound and the phospholipid is calculated using a total amount (weight) of the phospholipids used. The term "weight" is used herein interchangeably with "mass".

In an alternative embodiment, the non-lamellar liquid crystal-forming composition according to the present invention may comprise no phospholipid, depending on use, a dosage form, composition, etc., as long as the non-lamellar liquid crystal-forming composition has a sufficiently high biocompatibility (i.e., safety). Thus, the present invention also relates to a composition, particularly, a non-lamellar liquid crystal-forming composition, comprising an amphipathic compound represented by the general formula (I). Such a non-lamellar liquid crystal-forming composition, for example, when comprising a drug, may be used as a sustained release formulation.

In the present invention, the "non-lamellar liquid crystal-forming composition" refers to a composition having a formed non-lamellar liquid crystal structure (non-lamellar liquid crystal composition) or a composition that has no formed non-lamellar liquid crystal structure in itself, but is capable of forming a non-lamellar liquid crystal structure in the presence of water (i.e., by contact with an aqueous medium) (liquid crystal precursor composition).

The non-lamellar liquid crystal-forming composition according to the present invention, when it is a liquid crystal precursor composition, comprises no aqueous medium, or does not comprise an aqueous medium in an amount sufficient for forming a non-lamellar liquid crystal structure. The non-lamellar liquid crystal-forming composition according to the present invention, when it is a non-lamellar liquid crystal composition, comprises an aqueous medium, preferably an aqueous medium in an amount sufficient for forming a non-lamellar liquid crystal structure. The aqueous medium may be, but not limited to, for example, sterile water, purified water, distilled water, ion exchanged water, ultrapure water, injectable water, physiological saline, or a phosphate buffer. The non-lamellar liquid crystal-forming composition according to the present invention may be a liquid crystal emulsion (more specifically, a non-lamellar liquid crystal emulsion composition). The non-lamellar liquid crystal-forming composition according to the present invention which is a liquid crystal emulsion preferably further comprises a surfactant. A non-lamellar liquid crystal emulsion composition is also called "dispersion". The non-lamellar liquid crystal emulsion composition according to the present invention comprising the amphipathic compound and phospholipid exhibits a high stability.

An example of surfactant used in the non-lamellar liquid crystal-forming composition according to the present invention is P80 (polyoxyethylene sorbitan monooleate (20E.O.). Other examples of the surfactant include nonionic surfactants including block copolymers of hydrophilic ethylene oxide and hydrophobic propylene oxide (polyoxyethylene polyoxypropylene glycol), polyoxyethylene alkyl ether, polyoxyethylene alkyl ester, and polyoxyethylene hydrogenated castor oil. The nonionic surfactant more preferably has a molecular weight of 1000 or higher (more preferably 5000 or higher). Examples of the block copolymer of ethylene oxide and propylene oxide include polyoxyethylene(200) polyoxypropylene(70) glycol, polyoxyethylene(196) polyoxypropylene(67) glycol, polyoxyethylene(160) polyoxypropylene(30) glycol, polyoxyethylene(120) polyoxypropylene(40) glycol, and the like. These block copolymers of ethylene oxide and propylene oxide are commercially available under various names such as Pluronic$^{(R)}$, Poloxamer$^{(R)}$, Unilube$^{(R)}$, and Pronon$^{(R)}$. Particularly preferred examples of the nonionic surfactant include polyoxyethylene(200) polyoxypropylene(70) glycol, polyoxyethylene(196) polyoxypropylene(67) glycol (also called Pluronic$^{(R)}$ F127, Unilube 70DP-950B, and Poloxamer$^{(R)}$407), and the like. In the present invention, the amphipathic compound represented by the general formula (I) and (I') used in the present invention is not included in the scope of the surfactant. The non-lamellar liquid crystal-forming composition according to the present invention may comprise one, or two or more of surfactants as described above.

The non-lamellar liquid crystal-forming composition according to the present invention may comprise at least one of an oil and an organic solvent.

Examples of the oil used in the non-lamellar liquid crystal-forming composition according to the present invention include, but are not limited to, plant oils such as sesame oil, soybean oil, corn oil, coconut oil, safflower oil, perilla oil, olive oil, castor oil, and cottonseed oil; animal oils such as egg yolk oil, fish oil, and lanoline; mineral oils such as medium-chain fatty acid triglyceride (MCT), triglyceride, and liquid paraffin; hydrocarbon oils such as squalene and squalane; ester oils such as isopropyl myristate (IPM); cholesterol, tocopherol, tocopherol acetate, glyceryl dioleate (GDO), gelled hydrocarbon, methyl tetrahydrofarnesylacetate, and methyl hexahydrogeranylgeranylacetate, and the like. The oil is preferably a pharmaceutically acceptable one.

The total amount of the amphipathic compound (isoprenoid-type) represented by the general formula (I), phospholipid, and oil contained in the non-lamellar liquid crystal-forming composition according to the present invention may be, but not limited to, 30% or more, typically 60 to 100%, preferably 65 to 95%, for example, about 75 to 95%, 75 to 93%, or 80 to 95%, of the amount of the whole composition when the non-lamellar liquid crystal-forming composition is a liquid crystal precursor composition, and may be, albeit differing depending on its use or the like, 0.01 to 40%, preferably about 1 to 30%, for example, 20 to 30%, 20 to 23%, or 25 to 30%, of the amount of the whole composition when the non-lamellar liquid crystal-forming composition is a liquid crystal emulsion composition.

The percentage (%) of a component in the non-lamellar liquid crystal-forming composition used herein means % by weight and can be indicated by the unit w/w%.

Examples of the organic solvent used in the non-lamellar liquid crystal-forming composition according to the present invention include, but are not limited to, alcohols such as ethanol, propylene glycol, and isopropanol, ethers such as diethyl ether and polyethylene glycol, dimethyl sulfoxide (DMSO), N-methylpyrrolidone (NMP), and dimethylacetamide (DMA). The organic solvent is preferably a pharmaceutically acceptable one. In the non-lamellar liquid crystal-forming composition according to the present invention, a protic organic solvent or an aprotic organic solvent may be used alone or a protic organic solvent and an aprotic organic solvent may be used in combination. Examples of the protic organic solvent include alcohols such as ethanol and propylene glycol, and polyethylene glycol. Examples of the aprotic organic solvent include ethers such as diethyl ether, dimethyl sulfoxide (DMSO), N-methylpyrrolidone (NMP), and dimethylacetamide (DMA).

The non-lamellar liquid crystal-forming composition according to the present invention may comprise a water soluble polymer. Examples of the water soluble polymer include, but are not limited to, hydroxypropylcellulose (HPC), hydroxyethylcellulose, polyvinylpyrrolidone, Carbopol, carrageenan, chitosan, chondroitin sulfate salt, xanthan gum, hyaluronic acid salt (sodium hyaluronate, etc.), alginic acid salt (sodium alginate, etc.), gelatin, and dextran. Examples

of the hydroxypropylcellulose (HPC) include HPC of 5 grades: HPC-SSL (molecular weight: approximately 40,000, viscosity: 2 to 2.9 mPa·s), HPC-SL (molecular weight: approximately 100,000, viscosity: 3 to 5.9 mPa·s), HPC-L (molecular weight: approximately 140,000, viscosity: 6 to 10 mPa·s), HPC-M (molecular weight: approximately 620,000, viscosity: 150 to 400 mPa·s), and HPC-H (molecular weight: approximately 910,000, viscosity: 1000 to 4000 mPa·s), commercially available from Nippon Soda Co., Ltd. (Japan). In one embodiment, the hydroxypropylcellulose may have a molecular weight of 1,000,000 or lower, or 800,000 or lower, for example, 10,000 to 700,000, or 10,000 to 80,000.

The non-lamellar liquid crystal-forming composition according to the present invention may comprise an antioxidant. Examples of the antioxidant include, but are not limited to, ascorbic acid and sodium sulfite.

The non-lamellar liquid crystal-forming composition according to the present invention can comprise water or form a gel in the presence of water. This gel formation indicates that non-lamellar liquid crystals (liquid crystal gel) are formed. The non-lamellar liquid crystals are capable of retaining a substance such as a drug in the inside and perform sustained release. The present invention also relates to a gel comprising the non-lamellar liquid crystal-forming composition according to the present invention (gel composition).

The non-lamellar liquid crystals are a liquid crystal structure that is not a lamellar liquid crystal and, specifically, may be, for example, cubic liquid crystals, reverse hexagonal liquid crystals (HII), an inverse micellar cubic phase (Fd3m), or a sponge phase (L3). The non-lamellar liquid crystals may comprise two or more types of non-lamellar liquid crystal phases.

The cubic liquid crystals may be cubic liquid crystals belonging to the crystallographic space group Ia3d (hereinafter, referred to as Ia3d cubic liquid crystals), cubic liquid crystals belonging to the crystallographic space group Pn3m (hereinafter, referred to as Pn3m cubic liquid crystals), or cubic liquid crystals belonging to the crystallographic space group Im3m (hereinafter, referred to as Im3m cubic liquid crystals).

The liquid crystal structure can be analyzed by conventional methods and can be analyzed by, for example, small-angle X-ray scattering (SAXS) measurement using the following method.

When a measurement sample is an emulsion, the sample can be filled into an X-ray capillary tube such as one made of soda glass or quartz, for example, and the capillary tube can be then sealed with an oxy-fuel burner, and subjected to SAXS measurement. The SAXS measurement can be performed using a commercially available instrument, for example, the NANO-Viewer nano-scale X-ray structure analysis equipment (Rigaku Corp.).

The liquid crystal structure which the non-lamellar liquid crystal-forming composition according to the present invention has formed, or which the non-lamellar liquid crystal-forming composition according to the present invention forms in the presence of water, can be confirmed by determining whether the following scattering peak ratio (peak interval) peculiar to each liquid crystal structure is shown as a result of SAXS measurement.

Ratio of Pn3m cubic liquid crystal:

$$\sqrt{2}:\sqrt{3}:\sqrt{4}:\sqrt{6}:\sqrt{8}:\sqrt{9}:\sqrt{10}: ,,,,$$

Ratio of Ia3d cubic liquid crystal:

$$\sqrt{3}:\sqrt{4}:\sqrt{7}:\sqrt{8}:\sqrt{10}:\sqrt{11}: ,,,,$$

Ratio of Im3m cubic liquid crystal:

$$\sqrt{2}:\sqrt{4}:\sqrt{6}:\sqrt{8}:\sqrt{10}:\sqrt{12}:\sqrt{14}: ,,,,$$

Ratio of Fd3m cubic liquid crystal:

$$\sqrt{3}:\sqrt{8}:\sqrt{11}:\sqrt{12}:\sqrt{16}:\sqrt{19}:\sqrt{24}:\sqrt{27}: ,,,,$$

Ratio of reverse hexagonal liquid crystal:

$$1:\sqrt{3}:2: ,,,,$$

The space group and the lattice constant can be easily determined by calculating a peak value from SAXS

intensity distribution data and then calculating the reciprocal ratio therefrom according to a method well known to persons skilled in the art.

On the other hand, a broad scattering peak is observed in the SAXS measurement of the sponge phase (L3 phase).

Not only the types of liquid crystal phases but their lattice spacings or lattice constants can be determined by analyzing the scattering vector values of the peaks of the measurement sample, including scattering vector value q1 [nm-1] of a peak positioned on the smallest angle side. The liquid crystal phases or the size of a unit lattice can be altered by changing the composition of the non-lamellar liquid crystal-forming composition on the basis of such analysis results. As a result, the sustained release (sustained release rate) of a drug from a composition or formulation (e.g., a precursor formulation and an emulsion) comprising the non-lamellar liquid crystal-forming composition and the drug can be controlled. The liquid crystal phases or the size of a unit lattice can be altered depending on, for example, the amphipathic compound represented by the general formula (I), phospholipid, and other components as well as their ratio (weight ratio), thereby obtaining a sustained release rate depending thereon. When amphipathic compounds represented by the general formula (I) having analogous R and the same carbon chain lengths (the values of n and m are the same therebetween), or close carbon chain lengths (only the values of m are the same therebetween) and the same R, are separately used in combination with the same phospholipid, at equivalent weight ratios to the phospholipid in non-lamellar liquid crystal-forming compositions, these compositions exhibit the same or analogous liquid crystal phases and exhibit similar sustained release characteristics (sustained release rate, etc.). Usually, the sustained release rate increases in the order of an inverse micellar cubic phase (Fd3m), reverse hexagonal liquid crystals (HII), and cubic liquid crystals, from lowest to highest.

The non-lamellar liquid crystal-forming composition according to the present invention can be used as a medical base material for *in vivo* application. The non-lamellar liquid crystal-forming composition according to the present invention has an adhesion preventing effect on living tissue and as such, can be used for adhesion prevention of living tissue. Thus, the non-lamellar liquid crystal-forming composition according to the present invention may be for use in adhesion prevention of living tissue. The non-lamellar liquid crystal-forming composition according to the present invention can be applied (administered) as an adhesion preventing agent for living tissue into a living body.

The non-lamellar liquid crystal-forming composition according to the present invention can exhibit an effect of preventing living tissue adhesion by applying it to living tissue that is at risk of adhesion. In the present invention, the term "adhesion preventing effect" refers to an effect of preventing a tissue that is at risk of adhesion from adhering to another tissue or organ to result in a state in which it is difficult to peel adhesions, so that adhesion is completely prevented or reduced to low adhesion levels. Thus, the non-lamellar liquid crystal-forming composition according to the present invention can be used for adhesion prevention of living tissue.

The adhesion preventing effect of the non-lamellar liquid crystal-forming composition according to the present invention is provided by the formation of a coating due to the formation of non-lamellar liquid crystal of the amphipathic compound contained in the non-lamellar liquid crystal-forming composition on the tissue surface to which the composition has been applied. The formed coating prevents the tissue from being in contact with other tissues or organs, thereby reducing adhesion.

The adhesion preventing effect of the non-lamellar liquid crystal-forming composition according to the present invention can be confirmed by, for example, applying the non-lamellar liquid crystal-forming composition to a tissue incision of an animal model subjected to laparotomy, closing the abdominal incision, and carrying out follow-up observation. Specifically, an abdominal median incision (e.g., approximately 30 mm) is made to a rat and an incision of approximately 20 mm in length is further made on each of the right and left upper parietal peritoneums. After complete hemostasis, the peritoneum incision is closed with continuous suture (using, e.g., Silk Suture 5-0). Then, a non-lamellar liquid crystal-forming composition sample is applied to either the right or left peritoneum incision so as to cover the sutured incision. When the non-lamellar liquid crystal-forming composition is a liquid crystal precursor composition, an aqueous medium (injectable water, etc.) may be further added to the sample applied site by e.g., spraying or the like so as to induce liquid crystal formation. None is applied to the other peritoneum incision. Subsequently, two layers of the abdominal wall are each closed. Laparotomy can be performed on the rat again after a certain period of time (e.g., 7 days) from surgery for evaluating whether adhesion is observed at the sutured incision.

The non-lamellar liquid crystal-forming composition may be applied in a manner appropriate for a dosage form used therefor. The amount of the non-lamellar liquid crystal-forming composition applied for this evaluation is typically preferably an amount corresponding to 5 to 50 mg of the amphipathic compound.

Adhesion can be evaluated by, for example, scoring adhesion in accordance with the following evaluation scores regarding adhesion severity.

- Grade 0: No adhesion
- Grade 1: Peelable adhesion with mild traction (involving no tissue damage)

(continued)

- Grade 2: Peelable adhesion with strong traction (involving no tissue damage)
- Grade 3: Adhesion involving tissue damage by peeling with strong traction

If the evaluation score for the incision to which the non-lamellar liquid crystal-forming composition sample has been applied is lower than that for the non-applied incision of the same animal individual, it can be determined that the non-lamellar liquid crystal-forming composition exhibits an adhesion preventing effect.

For example, the adhesion range percentage of each incision is calculated as the ratio (%) of an adhesion length to the sutured incision of approximately 20 mm in length. Based on the adhesion range percentage, the following rating can be made: A, when the ratio of the adhesion range of the incision to which the non-lamellar liquid crystal-forming composition sample has been applied to that of the non-applied incision (i.e., adhesion range percentage on the sample-applied side / adhesion range percentage on the non-applied side $\times$ 100) is 40% or less; B+, when the ratio is 41 to 60%; B-, when the ratio is 61 to 80%; and C, when the ratio is 81% or more. In this evaluation, the rating of A or B+ is preferred as indicating the adhesion preventing effect.

In the present invention, the "adhesion prevention" can be indicated by a decrease in frequency and/or degree of adhesion at an applied site by the application of (treatment with) the non-lamellar liquid crystal-forming composition, as compared with an untreated control.

In Patent Literature 3, etc., most of the amphipathic compounds represented by the general formula (I) have been demonstrated to exhibit an adhesion preventing effect.

The present invention provides a method for preventing adhesion of living tissue, comprising applying the non-lamellar liquid crystal-forming composition according to the present invention to living tissue. More specifically, the present invention also provides a method for preventing tissue adhesion at an affected site, comprising applying an effective amount of the non-lamellar liquid crystal-forming composition according to the present invention to an affected site of a patient, specifically a site at risk of adhesion, in particular, a site at which tissue repair is expected to occur (e.g., an *in vivo* inflammatory site or injured site). Specific examples of such site at risk of adhesion include exogenously- or endogenously-induced inflammatory sites, wound sites such as surgical incisions, and sites at which the tissue surface has been damaged due to artificial treatment involving, e.g., contact during surgery. In the context of the present invention, the term "injured site" refers to a site of a tissue or organ damaged as a result of surgery, trauma, diseases, or the like. Examples of a tissue or organ to which the adhesion preventing agent is applied include, but are not limited to, peritoneum, small intestine, large intestine, rectum, stomach, duodenum, cecum, liver, uterus, fallopian tube, lymphatic vessels, heart, pericardium, lung, brain, ovary, and tendons. In typical cases, the non-lamellar liquid crystal-forming composition according to the present invention is applied to an incision, an area surrounding an incision, or a whole organ having an incision, upon surgery. The non-lamellar liquid crystal-forming composition according to the present invention may be applied to an *in vivo* site to be in contact with a wound site or an inflammatory site.

The application of the non-lamellar liquid crystal-forming composition to an affected site such as an injured site (e.g., a wound site) or an inflammatory site can be done in a manner suitable for a dosage form. For example, the adhesion preventing agent can be sprayed onto an affected site such as an injured site (e.g., a wound site) or an inflammatory site using a gas propellant aerosol container. Alternatively, in the case of a pump spray formulation, the non-lamellar liquid crystal-forming composition can be sprayed onto an affected site such as an injured site (e.g., a wound site) or an inflammatory site using a general-use non-gas- propellant (e.g., manual) spray container, for example. In the case of endoscopic surgery or laparoscopic surgery, the non-lamellar liquid crystal-forming composition can be sprayed onto an affected site such as an injured site (e.g., a wound site) using, for example, a spray nozzle used during endoscopic surgery or laparoscopic surgery. In the context of the present invention, the term "spray" refers to ejecting (atomizing and/or squirting) a substance of interest in the form of droplets, mist, fine particles, foam or the like by pressure. If the non-lamellar liquid crystal-forming composition is a topical formulation, an adequate amount of the non-lamellar liquid crystal-forming composition can be applied by spreading it to an affected site such as an injured site (e.g., a wound site) or an inflammatory site. If the non-lamellar liquid crystal-forming composition is an injection, the non-lamellar liquid crystal-forming composition can be injected into an affected site such as an injured site (e.g., a wound site) or an inflammatory site.

It is preferred to apply the non-lamellar liquid crystal-forming composition according to the present invention to an affected site such as an injured site (e.g., a wound site) or an inflammatory site in a sufficient amount to cover the affected site. In a preferred embodiment, a specific dosage amount of the non-lamellar liquid crystal-forming composition according to the present invention is 10 mg to 100 g, or 50 mg to 50 g (and more preferably 0.1 g to 10 g) for humans.

When the non-lamellar liquid crystal-forming composition according to the present invention contains a sufficient amount of an aqueous medium (e.g., the non-lamellar liquid crystal-forming composition being a liquid crystal emulsion), the non-lamellar liquid crystal-forming composition can form non-lamellar liquid crystals on the tissue surface to which the non-lamellar liquid crystal-forming composition has been applied. When the non-lamellar liquid crystal-forming composition according to the present invention does not contain a sufficient amount of an aqueous medium (e.g., the non-lamellar liquid crystal-forming composition being a liquid crystal precursor composition), it is preferred to apply an aqueous medium in addition to the non-lamellar liquid crystal-forming composition to an affected site such as an injured site (e.g., a wound site) or an inflammatory site in order to promote coating formation, although non-lamellar liquid crystals are also formed with water in the body. The aqueous medium may be, for example, water such as sterile water, purified water, distilled water, ion exchanged water, ultrapure water, or injectable water, or may be a physiologically acceptable aqueous solution. Examples of the physiologically acceptable aqueous solution include physiological saline; aqueous electrolyte solutions such as aqueous sodium chloride solution, aqueous calcium chloride solution, aqueous magnesium chloride solution, aqueous sodium sulfate solution, aqueous potassium sulfate solution, aqueous sodium carbonate solution, and aqueous sodium acetate solution; buffers such as phosphate buffer and Tris-HCl buffer; aqueous solutions containing sugar molecules such as glucose, sucrose, maltose, and hyaluronic acid; and aqueous solutions containing water soluble polymers such as polyethylene glycol and polyvinyl alcohol. Preferred examples of the physiologically acceptable aqueous solution include a hyaluronic acid aqueous solution containing hyaluronic acid or a salt thereof (e.g., sodium hyaluronate).

It is preferred to apply the non-lamellar liquid crystal-forming composition which is a liquid crystal precursor composition and then apply an aqueous medium on the applied non-lamellar liquid crystal-forming composition, but there is no limitation thereto. It is possible to apply an aqueous medium in a manner similar to that for the non-lamellar liquid crystal-forming composition, for example, by spraying, spreading, or injecting. After applying an aqueous medium to a tissue or organ in the above manner, it is preferred to allow it to stand for a certain period of time (which may be, but is not limited to, for example, 1 to 30 minutes and preferably 5 to 10 minutes) to promote coating formation.

A subject (or patient) to which the method for preventing adhesion using the non-lamellar liquid crystal-forming composition of the present invention is applied is typically a mammal such as a human, livestock, pet animal, and laboratory animal. A subject who has received or is expected to receive an injury to a tissue (or organ) due to surgery, trauma, diseases, or the like is particularly preferred. Examples of surgery include endoscopic surgery and laparoscopic surgery as well as laparotomy.

Since the non-lamellar liquid crystal-forming composition of the present invention has a marked reduction or disappearance of a toxicity, the method for preventing adhesion according to the present invention is highly safe for patients to be treated therewith.

The non-lamellar liquid crystal-forming composition according to the present invention may comprise a drug in addition to the amphipathic compound represented by the general formula (I) and optionally the above-mentioned further components including a phospholipid. In the context of the present invention, the "drug" is any substance (active ingredient) to be administered to a living body, which is intended to be retained in the non-lamellar liquid crystal structure for allowing sustained release (controlled release) by incorporating the drug into the non-lamellar liquid crystal-forming composition. The drug is not the amphipathic compound itself represented by the general formula (I). The drug may be an organic compound or may be an inorganic compound. The drug may be a water soluble drug or may be a lipid soluble (lipophilic, water insoluble or poorly water soluble) drug. The drug may be a physiologically active substance. The drug may be, but not limited to, for example, a protein, a peptide, an amino acid, or a nucleic acid. The drug may be, but not limited to, for example, a gonadotropin-releasing hormone (GnRH) agonist. The gonadotropin-releasing hormone (GnRH) agonist may be, but not limited to, for example, leuprolide or a salt thereof. The salt of leuprolide may be any type of pharmaceutically acceptable salt. Examples thereof include, but are not limited to, carboxylic acid salts including acetic acid salt (i.e., leuprolide acetate). Leuprolide acetate is also called leuprorelin acetate or the like. Such a non-lamellar liquid crystal-forming composition can be used for sustained release of the drug.

The present invention also provides a pharmaceutical formulation comprising the non-lamellar liquid crystal-forming composition according to the present invention. The pharmaceutical formulation according to the present invention preferably comprises a non-lamellar liquid crystal-forming composition comprising an amphipathic compound represented by the general formula (I) and a phospholipid and having a biocompatibility increased by the phospholipid. In one embodiment, the pharmaceutical formulation according to the present invention comprises a non-lamellar liquid crystal-forming composition comprising an amphipathic compound represented by the general formula (I), a phospholipid, and a drug. The "pharmaceutical formulation" used in the present invention may be a pharmaceutical composition. The pharmaceutical formulation or the pharmaceutical composition according to the present invention may further comprise other substances such as pharmaceutically acceptable additives (e.g., carriers, excipients, lubricants, disintegrants, wetting agents, buffers, corrigents, preservatives, colorants, flavoring agents, and propellants) as long as the ability to form non-lamellar liquid crystals can be maintained.

The pharmaceutical formulation according to the present invention may be formulated in any dosage form, for example, a spray formulation, an aerosol formulation, an injection, or a depot formulation.

The pharmaceutical formulation according to the present invention may be for use in adhesion prevention of living tissue. The pharmaceutical formulation according to the present invention may be a sustained release formulation, such as a depot formulation, further comprising the drug as described above.

The present invention also provides a method for delivering a drug in a sustained release manner into a living body (into the body) or to live cells or live tissue, comprising applying the pharmaceutical formulation according to the present invention comprising the drug or the non-lamellar liquid crystal-forming composition according to the present invention comprising the drug to a living body such as a subject (or patient), for example, into the body (particularly, living tissue in the body) or to a body surface, or to live cells or live tissue. In the case of using the non-lamellar liquid crystal-forming composition that is a liquid crystal precursor composition, or the pharmaceutical formulation comprising it, an aqueous medium may be applied onto the non-lamellar liquid crystal-forming composition or the pharmaceutical formulation after its application, though the approach is not limited thereto. Alternatively, in that case, liquid crystal formation by water in a living body can also be utilized. In this context, the application to a living body (into the body or to a body surface, etc.) or to live cells or live tissue is preferably performed by parenteral administration (e.g., intravenous, intra-arterial, intraperitoneal, intramuscular, subcutaneous, or intracutaneous). The method of the present invention can deliver a drug in a sustained release manner into the body or to live cells or live tissue with a high safety.

Examples

Hereinafter, the present invention will be described further specifically with reference to Examples. However, the technical scope of the present invention is not limited to these Examples.

[Example 1] Synthesis of amphipathic compound - (1)

(1) Synthesis of mono-O-(5,9,13-trimethyltetradec-4-enoyl)sorbitan

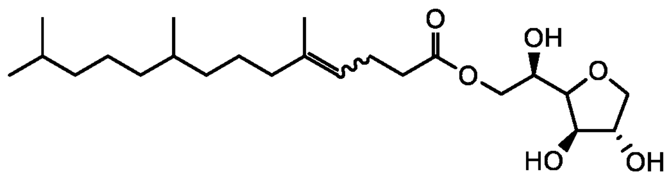

14.1 g (50.0 mmol) of methyl 5,9,13-trimethyltetradec-4-enoate and 10.9 g of 90 % by weight sorbitan aqueous solution (60.0 mmol, sorbitan M-90, Sanko Chemical Industry Co., Ltd., containing 90% solid and 10% water, content in the solid: 79 to 84% sorbitan, 15 to 18% isosorbide) were added to a reaction container at room temperature, and then stirred at 120°C at 8 kPa for 1 hour. The reduced pressure was canceled with nitrogen, and 0.27 g (5.0 mmol) of sodium methoxide and 0.01 g of sodium phosphinate monohydrate were added thereto, followed by stirring at 160°C at 8 kPa for 1 hour. The reduced pressure was canceled with nitrogen, and 0.27 g (5.0 mmol) of sodium methoxide was added thereto, followed by further stirring at 160°C at 8 kPa for 1 hour. After the reaction mixture was cooled to 60°C, 50 mL of ethyl acetate and 50 mL of 0.5 M hydrochloric acid were added thereto with stirring. The resulting reaction solution was subjected to extraction by the addition of 150 mL of ethyl acetate. The extract was washed with saturated sodium bicarbonate aqueous solution and saturated brine, successively, dried over magnesium sulfate, filtered, and concentrated. The resulting residue was purified by silica gel column chromatography (mobile phase: ethyl acetate/hexane mixture) to obtain 8.96 g of a fraction containing the title compound (43% yield) as a light brown transparent liquid. The obtained fraction contained mono-O-(5,9,13-trimethyltetradec-4-enoyl)sorbitan and mono-O-(5,9,13-trimethyltetradec-4-enoyl)isosorbide at a ratio of approximately 8:2 (weight ratio) (as calculated from a TIC area value based on GC-MS measurement in the ion mode EI+ (positive)). The obtained fraction further contained a small amount of a diester derivative from sorbitan (as estimated by GC-MS measurement and TLC analysis). Results of measuring $^1$H-NMR of the obtained fraction are as follows.

$^1$H-NMR spectrum (300 MHz, $CDCl_3$, TMS) δ: 0.7-0.9 (m, 9H), 0.9-1.7 (m, 15H), 1.8-2.0 (m, 2H), 2.2-2.5 (m, 4H), 3.5-4.9 (m, 6.1H), 5.04 (m, 1H), 5.0-5.2 (m, 0.6H)
Mono-O-(5,9,13-trimethyltetradec-4-enoyl)sorbitan is also referred to as C17 sorbitan ester.

The obtained fraction was used as a mono-O-(5,9,13-trimethyltetradec-4-enoyl)sorbitan fraction (C17 sorbitan ester fraction) in Examples later.

(2) Synthesis of mono-O-(5,9,13-trimethyltetradecanoyl)sorbitan

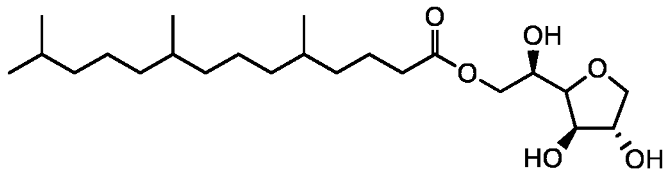

Under a nitrogen atmosphere, 0.48 g of 5% palladium carbon was added to a solution of 4.14 g (10.0 mmol) of mono-O-(5,9,13-trimethyltetradec-4-enoyl)sorbitan in ethyl acetate (12 mL). After nitrogen in the system was replaced with hydrogen, the reaction mixture was stirred at room temperature for 2 days under an atmospheric pressure hydrogen atmosphere. After hydrogen in the system was replaced with nitrogen, the 5% palladium carbon was filtered off. The filtrate was purified by silica gel column chromatography (mobile phase: ethyl acetate) to obtain 4.02 g of a fraction containing the title compound (97% yield) as a colorless transparent liquid. The obtained fraction contained mono-O-(5,9,13-trimethyltetradecanoyl)sorbitan and mono-O-(5,9,13-trimethyltetradecanoyl)isosorbide at a ratio of approximately 8:2 (weight ratio) (as calculated from a TIC area value based on GC-MS measurement in the ion mode EI+). The obtained fraction further contained a small amount of a diester derivative from sorbitan (as estimated by GC-MS measurement and TLC analysis). Results of measuring $^1$H-NMR of the obtained fraction are as follows.

$^1$H-NMR spectrum (300 MHz, $CDCl_3$, TMS) $\delta$: 0.7-0.9 (m, 12H), 0.9-1.7 (m, 19H), 2.2-2.4 (m, 2H), 3.5-4.9 (m, 6.4H), 5.0-5.2 (m, 0.6H)

Mono-O-(5,9,13-trimethyltetradecanoyl)sorbitan is also referred to as saturated C17 sorbitan ester.

(3) Synthesis of mono-O-(5,9,13,17-tetramethyloctadec-4-enoyl)sorbitan

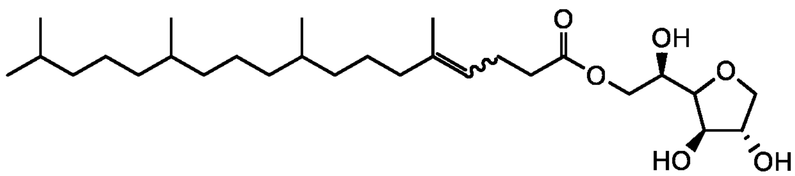

70.5 g (200 mmol) of methyl 5,9,13,17-tetramethyloctadec-4-enoate and 54.7 g of 90 % by weight sorbitan aqueous solution (300 mmol, sorbitan M-90, Sanko Chemical Industry Co., Ltd., containing 90% solid and 10% water, content in the solid: 79 to 84% sorbitan, 15 to 18% isosorbide) were added to a reaction container at room temperature, and then stirred at 120°C at 8 kPa for 1 hour. The reduced pressure was canceled with nitrogen, and 2.2 g (40 mmol) of sodium methoxide and 0.04 g of sodium phosphinate monohydrate were added thereto, followed by stirring at 160°C at 8 kPa for 1 hour. The reduced pressure was canceled with nitrogen, and 1.1 g (20 mmol) of sodium methoxide was added thereto, followed by further stirring at 160°C at 8 kPa for 1 hour. The reduced pressure was canceled with nitrogen, and 1.1 g (20 mmol) of sodium methoxide was added thereto again, followed by further stirring at 160°C at 8 kPa for 1.5 hours. After the reaction mixture was cooled to 60°C, 200 mL of ethyl acetate and 200 mL of 0.5 M hydrochloric acid were added thereto with stirring. The resulting reaction solution was subj ected to extraction by the addition of 600 mL of ethyl acetate. The extract was washed with saturated sodium bicarbonate aqueous solution and saturated brine, successively, dried over magnesium sulfate, filtered, and concentrated. The resulting residue was purified by silica gel column chromatography (mobile phase: ethyl acetate/hexane mixture) to obtain 36.1 g of a fraction containing the title compound (37% yield) as a light brown transparent liquid. The obtained fraction contained mono-O-(5,9,13,17-tetramethyloctadec-4-enoyl)sorbitan and mono-O-(5,9,13,17-tetramethyloctadec-4-enoyl)isosorbide at a ratio of approximately 8:2 (weight ratio) (as calculated from a TIC area value based on GC-MS measurement in the ion mode EI+). The obtained fraction further contained a small amount of a diester derivative from sorbitan (as estimated by GC-MS measurement and TLC analysis). Results of measuring $^1$H-NMR of the obtained fraction are as follows.

$^1$H-NMR spectrum (300 MHz, $CDCl_3$, TMS) $\delta$: 0.7-0.9 (m, 12H), 0.9-1.8 (m, 22H), 1.85-2.0 (m, 2H), 2.0-2.5 (m, 4H), 3.5-4.9 (m, 6.4H), 5.04 (m, 1H), 5.0-5.2 (m, 0.6H)

Mono-O-(5,9,13,17-tetramethyloctadec-4-enoyl)sorbitan is also referred to as C22 sorbitan ester.

The obtained fraction was used as a mono-O-(5,9,13,17-tetramethyloctadec-4-enoyl)sorbitan fraction (C22 sorbitan ester fraction) in Examples later.

Further, 13.00 g of the C22 sorbitan ester fraction was subjected to purification through a silica gel column (mobile phase: ethyl acetate/hexane mixture) so that low polar components such as diester derivative from sorbitan, and mono-O-(5,9,13,17-tetramethyloctadec-4-enoyl)isosorbide were removed from the C22 sorbitan ester fraction to obtain 6.78 g of a purified fraction as a pale yellow transparent liquid. Its purity was no less than 99% of mono-O-(5,9,13-trimethyltetradecanoyl)sorbitan (as calculated from a TIC area value based on GC-MS measurement in the ion mode EI+). The obtained fraction contained few low polar components such as diester derivative from sorbitan (as estimated by GC-MS measurement and TLC analysis). Results of measuring $^1$H-NMR of the obtained compound are as follows. $^1$H-NMR spectrum (300 MHz, $CDCl_3$, TMS) $\delta$: 0.8-0.9 (m, 12H), 0.9-1.7 (m, 22H), 1.9-2.0 (m, 2H), 2.2-2.5 (m, 4H), 2.93 (brs, OH), 3.6-4.5 (m, 7.5H), 5.06 (brd, J = 5.0 Hz, 1H), 4.9-5.2 (m, 0.5H)

The purified fraction obtained as described above is referred to as highly pure mono-O-(5,9,13,17-tetramethyloctadec-4-enoyl)sorbitan or highly pure C22 sorbitan ester.

(4) Synthesis of mono-O-(5,9,13,17-tetramethyloctadecanoyl)sorbitan

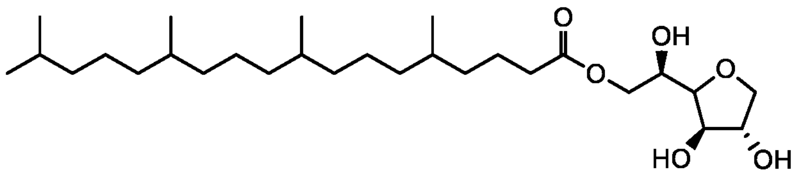

Under a nitrogen atmosphere, 0.70 g of 5% palladium carbon was added to a solution of 5.82 g (12.0 mmol) of the C22 sorbitan ester fraction in ethyl acetate (17.4 mL). After nitrogen in the system was replaced with hydrogen, the reaction mixture was stirred at room temperature for 2 days under an atmospheric pressure hydrogen atmosphere. After hydrogen in the system was replaced with nitrogen, the 5% palladium carbon was filtered off. The filtrate was purified by silica gel column chromatography (mobile phase: ethyl acetate) to obtain 5.69 g of a fraction containing the title compound (97% yield) as a colorless transparent liquid. The obtained fraction contained mono-O-(5,9,13,17-tetramethyloctadecanoyl)sorbitan and mono-O-(5,9,13,17-tetramethyloctadecanoyl)isosorbide at a ratio of approximately 8:2 (weight ratio) (as calculated from a TIC area value based on GC-MS measurement in the ion mode EI+). The obtained fraction further contained a small amount of a diester derivative from sorbitan (as estimated by GC-MS measurement and TLC analysis). Results of measuring $^1$H-NMR of the obtained fraction are as follows.

$^1$H-NMR spectrum (300 MHz, $CDCl_3$, TMS) $\delta$: 0.7-0.9 (m, 15H), 0.9-1.7 (m, 26H), 2.2-2.4 (m, 2H), 3.5-4.9 (m, 6.4H), 5.0-5.2 (m, 0.6H)

Mono-O-(5,9,13,17-tetramethyloctadecanoyl)sorbitan is also referred to as saturated C22 sorbitan ester.

The obtained fraction was used as a mono-O-(5,9,13,17-tetramethyloctadecanoyl)sorbitan fraction (saturated C22 sorbitan ester fraction) in Examples later.

(5) Synthesis of mono-O-(5,9,13,17-tetramethyloctadec-4-enoyl)isosorbide

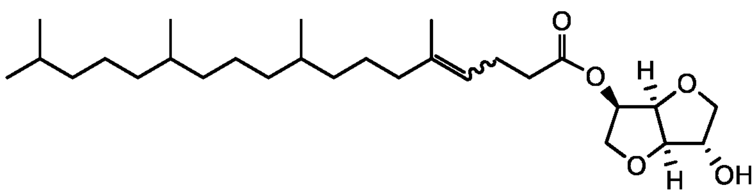

35.3 g (100 mmol) of methyl 5,9,13,17-tetramethyloctadec-4-enoate and 29.2 g of isosorbide (200 mmol, Sanko

Chemical Industry Co., Ltd., 98.0% or higher purity, 1.0% or less water) were added to a reaction container at room temperature. After the mixture was heated to 120°C with stirring at 8 kPa, the reduced pressure was canceled with nitrogen, and 1.1 g (20 mmol) of sodium methoxide and 0.02 g of sodium phosphinate monohydrate were added thereto. Under a nitrogen atmosphere, after the reaction mixture was heated to 180°C, the pressure was reduced to 8 kPa, followed by stirring for 1 hour. The reduced pressure was canceled with nitrogen, and 0.54 g (10 mmol) of sodium methoxide was added thereto, followed by further stirring at 180°C at 8 kPa for 1 hour. The reduced pressure was canceled with nitrogen, and 0.54 g (10 mmol) of sodium methoxide was added thereto again, followed by further stirring at 180°C at 8 kPa for 1 hour. After the reaction mixture was cooled to 60°C, 100 mL of ethyl acetate and 100 mL of 0.5 M hydrochloric acid were added thereto with stirring. The resulting reaction solution was subjected to extraction by the addition of 300 mL of ethyl acetate. The extract was washed with saturated sodium bicarbonate aqueous solution and saturated brine, successively, dried over magnesium sulfate, filtered, and concentrated. The resulting residue was purified by silica gel column chromatography (mobile phase: ethyl acetate/hexane mixture) to obtain 17.9 g of the title compound (38% yield) as a light brown transparent liquid.

The compound was further purified by silica gel column chromatography (with ethyl acetate/hexane mixture), which results in separation into 2-O-(5,9,13,17-tetramethyloctadec-4-enoyl)isosorbide and 5-O-(5,9,13,17-tetramethyloctadec-4-enoyl)isosorbide at a ratio of 60:40 (weight ratio). Results of measuring $^1$H-NMR of the obtained two compounds are as follows.

< 2-O-(5,9,13,17-Tetramethyloctadec-4-enoyl)isosorbide >

$^1$H-NMR spectrum (300 MHz, $CDCl_3$, TMS) δ: 0.7-0.9 (m, 12H), 0.9-1.7 (m, 22H), 1.85-2.0 (m, 2H), 2.2-2.4 (m, 4H), 2.56 (d, J = 7.2 Hz, OH), 3.55 (dd, J = 6.0, 9.5 Hz, 1H), 3.87 (dd, J = 6.0, 9.5 Hz, 1H), 3.99 (m, 2H), 4.29 (m, 1H), 4.44 (d, J = 4.3 Hz, 1H), 4.60 (t, J = 4.9 Hz, 1H), 5.04 (m, 1H), 5.21 (s, 1H)

< 5-O-(5,9,13,17-Tetramethyloctadec-4-enoyl)isosorbide >

$^1$H-NMR spectrum (300 MHz, $CDCl_3$, TMS) δ: 0.7-0.9 (m, 12H), 0.9-1.7 (m, 22H), 1.83 (brs, OH), 1.85-2.0 (m, 2H), 2.2-2.4 (m, 4H), 2.2-2.4 (m, 4H), 3.74 (ddd, J = 2.1.5.2, 9.8 Hz, 1H), 3.82-3.93 (m, 3H), 4.31 (s, 1H), 4.38 (d, J = 4.6 Hz, 1H), 4.83 (t, J = 5.0 Hz, 1H), 5.07 (m, 1H), 5.13 (m, 1H)

Mono-O-(5,9,13,17-tetramethyloctadec-4-enoyl)isosorbide is also referred to as C22 isosorbide ester.

(6) Synthesis of mono-O-(5,9,13,17-tetramethyloctadecanoyl)isosorbide

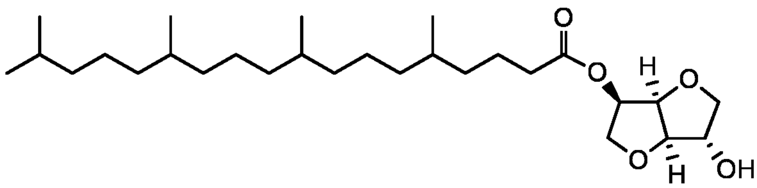

Under a nitrogen atmosphere, 0.56 g of 5% palladium carbon was added to a solution of 4.66 g (10.0 mmol) of mono-O-(5,9,13,17-tetramethyloctadec-4-enoyl)isosorbide in ethyl acetate (14 mL). After nitrogen in the system was replaced with hydrogen, the reaction mixture was stirred at room temperature for 2 days under an atmospheric pressure hydrogen atmosphere. After hydrogen in the system was replaced with nitrogen, the 5% palladium carbon was filtered off. The filtrate was purified by silica gel column chromatography (mobile phase: ethyl acetate) to obtain 4.65 g of the title compound (99% yield) as a colorless transparent liquid.

Mono-O-(5,9,13,17-tetramethyloctadecanoyl)isosorbide is also referred to as saturated C22 isosorbide ester.

(7) Synthesis of mono-O-(5,9,13-trimethyltetradec-4-enoyl)glycerol

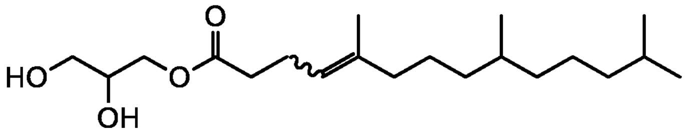

1.0 g (3.5 mmol) of methyl 5,9,13-trimethyltetradec-4-enoate (i.e., methyl tetrahydrofarnesylacetate) was slowly added dropwise to a solution of 0.65 g (7.1 mmol) of glycerol and 0.59 g (4.3 mmol) of potassium carbonate in dry N,N-dimethylformamide (3.5 mL) at 80°C. After the reaction mixture was stirred at 100°C for 18 hours, 1 M hydrochloric acid was added to the reaction solution, followed by extraction with ether. The extract was washed with saturated sodium bicarbonate aqueous solution and saturated brine, successively, and dried over anhydrous sodium sulfate, filtered, and concentrated. The resulting residue was purified by silica gel column chromatography (mobile phase: ethyl acetate/hexane mixture) to obtain the title compound as a colorless transparent liquid. Results of measuring $^1$H-NMR and viscosity of the obtained compound are as follows.

$^1$H-NMR spectrum (300 MHz, $CDCl_3$, TMS) $\delta$: 0.80-0.90 (m, 9H), 1.00-1.70 (m, 15H), 1.97 (td, J = 7.8, 17.0 Hz, 2H), 2.13 (t, J = 6.1 Hz, 1H, OH), 2.25-2.45 (m, 4H), 2.55 (d, J = 5.2 Hz, 1H, OH), 3.50-4.00 (m, 3H), 4.10-4.25 (m, 2H), 5.08 (t, J = 6.7 Hz, 1H)
Viscosity: 0.48 Pa·s (at shear velocity of 92 1/s)
Mono-O-(5,9,13-trimethyltetradec-4-enoyl)glycerol is also referred to as C17 glycerin ester.

(8) Synthesis of mono-O-(5,9,13,17-tetramethyloctadec-4-enoyl)glycerol

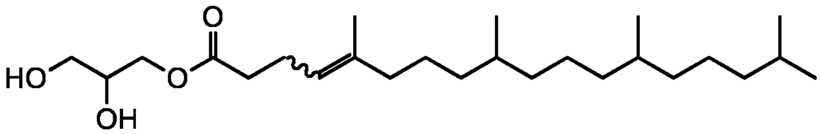

Under reduced pressure of 60 to 70 mmHg and nitrogen gas stream, 28.2 g (80.0 mmol) of methyl 5,9,13,17-tetramethyloctadec-4-enoate was slowly added dropwise at 80°C to a solution of 23.5 g (255 mmol) of glycerol and 0.55 g (4.0 mmol) of potassium carbonate in dry N,N-dimethylformamide (48 mL), followed by stirring at the same temperature for 3 hours. The resulting reaction solution was diluted with a mixed solvent of ethyl acetate/hexane (1:1, 200 mL), washed with water, saturated sodium bicarbonate aqueous solution, and saturated brine (twice), and then dried over magnesium sulfate. After filtration, the filtrate was concentrated, and the resulting residue was purified by silica gel column chromatography (mobile phase: hexane/ethyl acetate = 100:0 to 30:70) to obtain 13.3 g of the title compound (40% yield) as a pale yellow transparent liquid. Results of measuring $^1$H-NMR of the obtained compound are as follows.

$^1$H-NMR spectrum (300 MHz, $CDCl_3$, TMS) $\delta$: 0.80-0.95 (m, 12H), 1.00-1.70 (m, 22H), 1.85-2.15 (m, 2H), 2.15-2.55 (m, 4H), 3.53-3.78 (m, 3H), 3.80-4.00 (m, 1H), 4.10-4.25 (m, 2H), 5.08 (dd, J = 6.9 Hz, J = 6.9 Hz, 1H)
Mono-O-(5,9,13,17-tetramethyloctadec-4-enoyl)glycerol is also referred to as C22 glycerin ester.

(9) Synthesis of mono-O-(5,9,13,17-tetramethyloctadecanoyl)glycerol

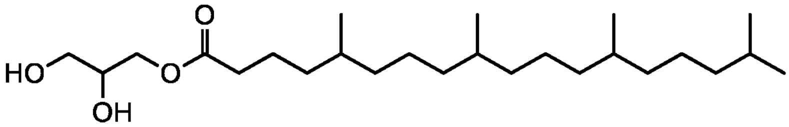

Under a nitrogen atmosphere, 2.5 g of 5% palladium carbon was added to a solution of 20.6 g (50.0 mmol) of mono-O-(5,9,13,17-tetramethyloctadec-4-enoyl)glycerol in ethyl acetate (62 mL). After nitrogen in the system was replaced with hydrogen, the reaction mixture was stirred at room temperature for 42 hours under an atmospheric pressure hydrogen atmosphere. After hydrogen in the system was replaced with nitrogen, the 5% palladium carbon was filtered

off. The filtrate was purified by silica gel column chromatography (with ethyl acetate) to obtain 20.2 g of the title compound (98% yield) as a colorless transparent liquid. Results of measuring $^1$H-NMR of the obtained compound are as follows.

$^1$H-NMR spectrum (300 MHz, $CDCl_3$, TMS) δ: 0.7-0.9 (m, 15H), 0.95-1.75 (m, 26H), 2.13 (t, J = 6.0 Hz, OH), 2.34 (t, J = 7.7 Hz, 2H), 2.56 (d, J = 5.1 Hz, OH), 3.55-3.75 (m, 2H), 3.94 (m, 1H), 4.15 (dd, J = 6.0, 11.7 Hz, 1H), 4.20 (dd, J = 4.7, 11.7 Hz, 1H)

Mono-O-(5,9,13,17-tetramethyloctadecanoyl)glycerol is also referred to as saturated C22 glycerin ester.

(10) Synthesis of mono-O-(5,9,13,17-tetramethyloctadeca-4,8,12,16-tetraenoyl) glycerol

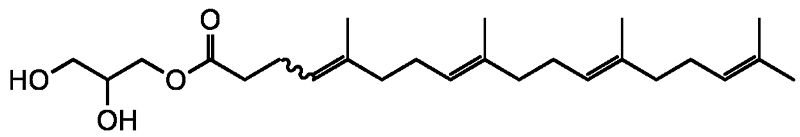

Under a nitrogen atmosphere, a solution of 53 mL (0.42 mol) of trimethyl orthoacetate and 5.0 mL (40 mmol) of n-hexanoic acid was added dropwise at 135°C over 8 hours to a solution of 58.1 g (200 mmol) of 3,7,11,15-tetramethylhexadeca-1,6,10,14-tetraen-3-ol (i.e., geranyl linalool) and 19 mL (0.15 mol) of trimethyl orthoacetate. After the reaction mixture was stirred for 6 hours at the same temperature, a solution of 5.3 mL (42 mmol) of trimethyl orthoacetate and 0.5 mL (4 mmol) of n-hexanoic acid was further added dropwise, and the mixture was further stirred for 2 hours at the same temperature. The resulting reaction solution was diluted with a mixed solvent of ethyl acetate/hexane (3:1, 300 mL), washed with saturated sodium bicarbonate aqueous solution (twice) and saturated brine, and then dried over magnesium sulfate. After filtration, the filtrate was concentrated to obtain 67.24 g of methyl 5,9,13,17-tetramethyloctadeca-4,8,12,16-tetraenoate (i.e., methyl geranylgeranylacetate) as a crude liquid product. The crude product was directly used for the next reaction.

Under reduced pressure of 200 to 250 mmHg, 13.9 g (40.0 mmol) of methyl 5,9,13,17-tetramethyloctadeca-4,8,12,16-tetraenoate (i.e., methyl geranylgeranylacetate) was slowly added dropwise at 85°C to a solution of 7.4 g (80 mmol) of glycerol and 5.5 g (40 mmol) of potassium carbonate in dry N,N-dimethylformamide (16 mL), followed by stirring at the same temperature for 6 hours. Methanol produced during this reaction was distilled off. The resulting reaction solution was diluted with a mixed solvent of ethyl acetate/hexane (1:1, 200 mL), washed with water, saturated sodium bicarbonate aqueous solution, and saturated brine (twice), and then dried over magnesium sulfate. After filtration, the filtrate was concentrated, and the resulting residue was purified by silica gel column chromatography (mobile phase: hexane/ethyl acetate = 100:0 to 0:100) to obtain 5.44 g of the title compound (33% yield) as a transparent liquid. Results of measuring $^1$H-NMR and viscosity of the obtained compound are as follows.

$^1$H-NMR spectrum (270 MHz, $CDCl_3$, TMS) δ: 1.55-1.72 (m, 15H), 1.9-2.2 (m, 13H), 2.27-2.45 (m, 4H), 2.53 (brs, 1H, OH), 3.59 (dd, J = 5.4, 11.4 Hz, 1H), 3.68 (dd, J = 3, 11.4 Hz, 1H), 3.92 (m, 1H), 4.15 (dd, J = 6.0, 11.6 Hz, 1H), 4.21 (dd, J = 4.8, 11.6 Hz, 1H), 5.05-5.15 (m, 4H)

Viscosity: 0.37 Pa·s (at shear velocity of 92 1/s)

Mono-O-(5,9,13,17-tetramethyloctadeca-4,8,12,16-tetraenoyl)glycerol is also referred to as glyceryl geranylgeranylacetate.

(11) Synthesis of mono-O-(5,9,13,17-tetramethyloctadecanoyl)erythritol

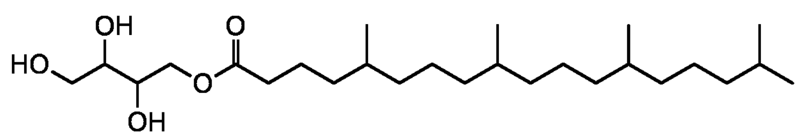

Under a nitrogen atmosphere, one drop of pyridine was added to 10 g of 5,9,13,17-tetramethyloctadecanoic acid and 20 ml of methylene chloride, and 5.2 g of thionyl chloride was added dropwise thereto at room temperature. After the completion of dropwise addition, the mixture was refluxed for 1 hour and concentrated under reduced pressure

to obtain 10.5 g of 5,9,13,17-tetramethyloctadecanoic acid chloride.

2.56 g of erythritol, 2.21 g of pyridine, and 70 ml of dry DMF were mixed and dissolved with heating. The resultant was cooled to room temperature, and a solution of 5 g of 5,9,13,17-tetramethyloctadecanoic acid chloride obtained above in 10 ml of methylene chloride was added dropwise thereto, and the mixture was then stirred at room temperature for 1 hour. 100 ml of methylene chloride was added to the resulting reaction solution, and the mixture was washed 3 times with saturated brine, and dried over anhydrous sodium sulfate. After performing filtration and vacuum concentration, the resultant was purified by silica gel column chromatography to obtain 2.83 g of the title compound as a transparent semi-solid. As a result of HPLC analysis, the obtained title compound contained 1-O-(5,9,13,17-tetramethyloctadecanoyl)erythritol (91.6%) and 2-0-(5,9,13,17-tetramethyloctadecanoyl)erythritol (8.4%). Results of measuring $^1$H-NMR of the obtained compound are as follows.

$^1$H-NMR spectrum (270 MHz, $CDCl_3$, TMS) $\delta$: 0.8-0.9 (m, 15H), 1.0-1.7 (m, 26H), 2.11 (br.s, 1H), 2.33 (t, J=7.9Hz, 2H), 2.66 (br.s, 1H), 2.75 (br.s, 1H), 3.6-3.9 (m, 4H), 4.29-4.36 (m, 2H)

Mono-O-(5,9,13,17-tetramethyloctadecanoyl)erythritol was also referred to as saturated C22 erythritol ester.

(12) Synthesis of mono-O-(5,9,13,17-tetramethyloctadec-4-enoyl)pentaerythritol

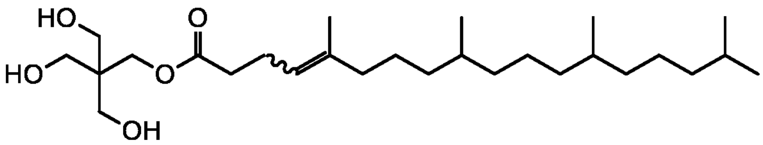

Under reduced pressure of 60-70 mmHg and nitrogen gas stream, 250 g (0.71 mol) of methyl 5,9,13,17-tetramethyloctadec-4-enoate was slowly added dropwise at 78-83°C to a solution of 157 g (1.15 mol) of pentaerythritol and 1.58 g (1.15 mmol) of potassium carbonate in dry N,N-dimethylformamide (700 mL). After the reaction mixture was stirred at the same temperature for 10 hours, formic acid was added at 75°C to adjust the pH to 4. After the resulting solution was subjected to vacuum concentration, the resultant was diluted with t-butylmethylether (1.5 L), and the resulting insoluble residue was filtered off. The filtrate was washed with 10% sodium bicarbonate aqueous solution twice, and then decolorized with activated carbon (8 g). After filtration, the filtrate was concentrated, and the resulting residue was purified by silica gel column chromatography (mobile phase: hexane/ethyl acetate mixture) to obtain the title compound.

$^1$H-NMR, Infrared (IR) spectrum by infrared spectroscopy and viscosity of the obtained compound were measured. The results are as follows.

$^1$H-NMR spectrum (300 MHz, $CDCl_3$, TMS) $\delta$: 0.80-0.95 (m, 12H), 1.00-1.70 (m, 22H), 1.90-2.05 (m, 2H), 2.25-2.45 (m, 4H), 3.64 (s, 6H), 4.24 (s, 2H), 5.07 (brs, 1H)

IR spectrum (NaCl thin film method): 3387, 2926, 2866, 1739, 1461, 1378, 1267, 1139, 1051.

Viscosity: 1.7 Pa·s.

Mono-O-(5,9,13,17-tetramethyloctadec-4-enoyl)pentaerythritol is also referred to as C22 pentaerythritol ester.

(13) Synthesis of mono-O-(5,9,13,17-tetramethyloctadecanoyl)pentaerythritol

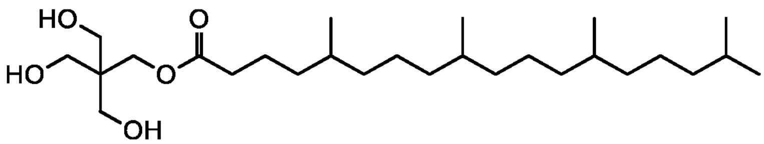

3.81 g of pentaerythritol, 2.21 g of pyridine, and 120 mL of dry DMF were mixed and dissolved with heating. The resultant was cooled to room temperature, a solution of 5 g of 5,9,13,17-tetramethyloctadecanoic acid chloride obtained in the synthesis process of mono-O-(5,9,13,17-tetramethyloctadecanoyl)erythritol (see, (11)) dissolved in 5 mL of methylene chloride was added dropwise thereto, and the mixture was then stirred at room temperature for 1 hour. 100 mL of methylene chloride was added to the resulting reaction solution, and the mixture was washed 3 times with saturated brine, and dried over anhydrous sodium sulfate. After performing filtration and vacuum concentration, the

resultant was purified by silica gel column chromatography to obtain 2.50 g of mono-O-(5,9,13,17-tetramethyloctadecanoyl)pentaerythritol having the following physical properties. As a result of HPLC analysis of the obtained product, the purity was no less than 99.5%. Results of NMR measurement were as follows.

$^1$H-NMR spectrum (270 MHz, $CDCl_3$, TMS) δ: 0.8-0.9 (m, 15H), 1.0-1.7 (m, 26H), 2.34 (t, J = 7.4 Hz, 2H), 3.06 (brs, 3H), 3.63 (s, 6H), 4.17 (s, 2H)
Mono-O-(5,9,13,17-tetramethyloctadecanoyl)pentaerythritol is also referred to as saturated C22 pentaerythritol ester.

(14) Synthesis of mono-O-(5,9,13,17-tetramethyloctadec-4-enoyl)diglycerol

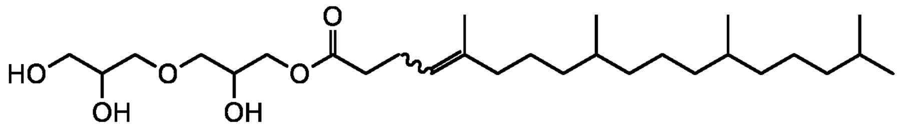

Under reduced pressure of 60-70 mmHg and nitrogen gas stream, 199 g (0.564 mol) of methyl 5,9,13,17-tetramethyloctadec-4-enoate was slowly added dropwise at 78-83°C to a solution of 259 g (1.56 mol) of diglycerol and 1.58 g (1.15 mmol) of potassium carbonate in dry N,N-dimethylformamide (700 mL). After the reaction mixture was stirred at the same temperature for 10 hours, formic acid was added at 75°C to adjust the pH to 4. After the resulting solution was subjected to vacuum concentration, the resultant was diluted with t-butylmethylether (1.5L), and the resulting insoluble residue was filtered off. The filtrate was washed with 10% sodium bicarbonate aqueous solution twice, and then decolorized with activated carbon (8 g). After filtration, the filtrate was concentrated, and the resulting residue was dissolved in ethanol, followed by filtration through cellulose powder. After the filtrate was concentrated, the resulting residue was purified by silica gel column chromatography (mobile phase: hexane/ethyl acetate mixture) to obtain the title compound as a transparent viscous liquid. Results of measuring $^1$H-NMR of the obtained compound are as follows.

$^1$H-NMR spectrum (300 MHz, $CDCl_3$, TMS) δ: 0.80-0.90 (m, 12H), 1.00-1.70 (m, 22H), 1.97 (ddd, J=6.9, 7.8, 17.4Hz, 2H), 2.20-2.45 (m, 4H), 3.50-4.10 (m, 8H), 4.10-4.25 (m, 2H), 5.08 (dd, J=6.6, 6.6Hz,1H)
Mono-O-(5,9,13,17-tetramethyloctadec-4-enoyl)diglycerol was also referred to as C22 diglycerin ester.

(15) Synthesis of mono-O-(3,7,11,15-tetramethylhexadec-2-enoyl)glycerol

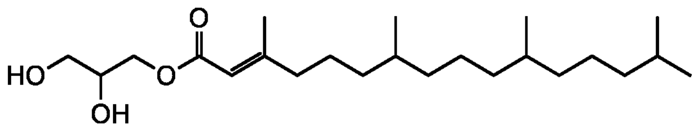

1.0 g (3.1 mmol) of methyl 3,7,11,15-tetramethylhexadec-2-enoate was slowly added dropwise to a solution of 0.57 g (6.2 mmol) of glycerol and 0.85 g (6.2 mmol) of potassium carbonate in dry N,N-dimethylformamide (3 mL) at 80°C. After the reaction mixture was stirred at 100°C for 12 hours, 1M hydrochloric acid was added to the reaction solution, followed by extraction with ether. The extract was washed with saturated sodium bicarbonate aqueous solution and saturated brine, successively, and dried over anhydrous sodium sulfate, filtered, and concentrated. The resulting residue was purified by silica gel column chromatography (mobile phase: ethyl acetate/hexane mixture) to obtain 459 mg of the title compound (35% yield) as a colorless viscous product. Results of measuring $^1$H-NMR of the obtained compound are as follows.

$^1$H-NMR spectrum (300 MHz, $CDCl_3$, TMS) δ: 0.80-0.95 (m, 12H), 1.00-1.80 (m, 19H), 1.90-2.20 (m, 5H), 3.50-4.00 (m, 3H), 4.10-4.30 (m, 2H), 5.71 (brs, 1H)
Mono-O-(3,7,11,15-tetramethylhexadec-2-enoyl)glycerol is also referred to as C20 glycerin ester, or glyceryl phytenate.

(16) Synthesis of mono-O-(3,7,11,15-tetramethylhexadecanoyl)glycerol

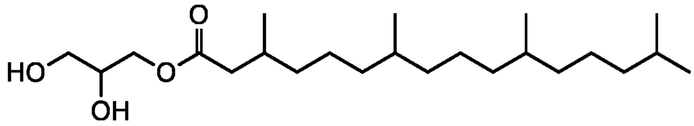

Under a nitrogen atmosphere, 0.26 g of 5% palladium carbon was added to a solution of 2.52 g (6.10 mmol) of C20 glycerin ester in ethyl acetate (15 mL). After nitrogen in the system was replaced with hydrogen, the reaction mixture was stirred at room temperature for 1 day under an atmospheric pressure hydrogen atmosphere. After hydrogen in the system was replaced with nitrogen, the 5% palladium carbon was filtered off. The filtrate was purified by silica gel column chromatography (mobile phase: ethyl acetate) to obtain 2.50 g of the title compound (99% yield) as a colorless transparent liquid. Results of measuring $^1$H-NMR of the obtained compound are as follows.

$^1$H-NMR spectrum (300 MHz, $CDCl_3$, TMS) $\delta$: 0.8-0.9 (m, 12H), 0.9-1.0 (m, 3H), 1.0-2.0 (m, 22H), 2.1-2.4 (m, 2H), 3.4-4.0 (m, 3H), 4.05-4.25 (m, 2H)

Mono-O-(3,7,11,15-tetramethylhexadecanoyl)glycerol is also referred to as saturated C20 glycerin ester, or glyceryl phytanate.

[Example 2] Preparation of precursor formulation, gel formation test, and liquid crystal structure analysis - (1)

An amphipathic compound having an isoprenoid fatty acid chain (isoprenoid lipid) having 22 carbon atoms, which is a self-organizing lipid (hereinafter, also referred to as SOL; the abbreviation SOL is used in Tables), synthesized in Example 1, soybean phosphatidylcholine (SPC) (LIPOID S100, Lipoid GmbH; hereinafter and in Tables, the abbreviation SPC is also used) as a phospholipid, an oil, and an alcohol were mixed according to the combination ratios shown in Table 1 below. The "self-organizing lipid" (SOL) is a lipid capable of forming a gel by itself when mixed with water. In Tables, "EtOH" denotes ethanol, and "PG" denotes propylene glycol (the same holds true for Tables 1 to 5 and 8 to 12). Regarding some formulations, the surfactant P80 (polyoxyethylene sorbitan monooleate (20E.O.), NIKKOL TO-10MV, Nikko Chemicals Co., Ltd.) and the water soluble polymer HPC (hydroxypropylcellulose, HPC-SSL, Nippon Soda Co., Ltd.) were further added. The obtained mixtures were dissolved at 40°C or lower in a water bath to prepare precursor formulations of Nos. 1 to 61 shown in Table 1.

Also, precursor formulations of Nos. 62 to 64 were prepared in the same way as above according to the combination ratios shown in Table 2 using, as a phospholipid, EPC (phosphatidylcholine, egg yolk-derived, 163-21181, FUJIFILM Wako Pure Chemical Corp.), DMPC (dimyristoyl phosphatidylcholine, COATSOME MC-4040, NOF Corp.), or DPPC (dipalmitoyl phosphatidylcholine, COATSOME MC-6060, NOF Corp.), instead of SPC.

Further, precursor formulations of Nos. 65 to 75 were prepared in the same way as above according to the combination ratios shown in Table 3 using, instead of the amphipathic compound having an isoprenoid fatty acid chain having 22 carbon atoms, an amphipathic compound having an isoprenoid fatty acid chain having 17 carbon atoms, synthesized in Example 1, or glyceryl monooleate (Rikemal XO-100, NOF Corp.), which has linear oleic acid as a fatty acid chain, or without the use of an amphipathic compound having an isoprenoid fatty acid chain.

The precursor formulations of Nos. 1 to 75 thus prepared were subjected to a gel formation test. An aliquot (about 100 to 300 mg) of each precursor formulation was added to an excess aqueous medium (about 0.5 to 2 mL of injectable water for Nos. 1 to 61 and 64 to 75 or phosphate buffered saline (PBS) of pH 7.4 for Nos. 62 and 63) in a vial, and mixed at room temperature (25°C) with a spatula or a vortex mixer. As a result, all the precursor formulations of Nos. 1 to 75 produced gel compositions having colorless transparent to white turbid appearance separated in the excess aqueous medium.

The gel compositions prepared from the precursor formulations of Nos. 1 to 70 were embedded directly in a pinhole slit and analyzed for their non-lamellar liquid crystal structures by small-angle X-ray scattering diffractometry using a small-angle X-ray scattering (SAXS) apparatus (manufactured by Rigaku Corp., Nano-Viewer).

Tables 1 to 3 show the obtained liquid crystal phases, and scattering vector values q1 [nm-1] positioned on the smallest angle side. HII means reverse hexagonal liquid crystals, Pn3m means reverse cubic liquid crystals belonging to the crystallographic space group Pn3m, Im3m means reverse cubic liquid crystals belonging to the crystallographic space group Im3m, and Fd3m means reverse cubic liquid crystals belonging to the crystallographic space group Fd3m. There were also some gel compositions having two sizes (scattering vector values q1) in the same liquid crystal phase, or having two types of different liquid crystal phases.

As shown in Tables 1 and 2, in the case of using the SOL amphipathic compound having an isoprenoid fatty acid chain having 22 carbon atoms, synthesized in Example 1, the SOL-containing precursor formulations were able to form non-lamellar liquid crystals in a wide range of SOL:phospholipid ratios (weight ratios) from 100:0 to at least 30:70, regardless of the types of the amphipathic compound, the phospholipid, and the oil. Also, the SOL-containing precursor formulations were able to form non-lamellar liquid crystals in a wide range of SOL + phospholipid (the total amount of SOL and the phospholipid):oil ratios (weight ratios) from 100:0 to at least 20:80. Even when a given amount of P80 or HPC was added as an additive, the SOL-containing precursor formulations favorably formed liquid crystal gels. These results revealed that, depending on various compositions of the amphipathic compound, the phospholipid, and the oil, diverse liquid crystal structures such as reverse hexagonal liquid crystals or reverse cubic liquid crystals belonging to the crystallographic space group Pn3m, Im3m, or Fd3m are formed and their q1 values attributed to the lattice constant have a wide range of values.

In the case of using SPC as the phospholipid and sesame oil (triglyceride) as the oil, no liquid crystal gel was formed at a phospholipid:oil ratio = 85:15 or 75:25 (weight ratio) without SOL (not indicated in Tables). Therefore, for forming a liquid crystal gel containing a phospholipid by using sesame oil (triglyceride) at these ratios, it seemed necessary to add SOL or an amphipathic compound having an isoprenoid fatty acid chain (isoprenoid lipid).

Table 1

| Formulation No. | SOL used | Composition | | | | | | Liquid crystal formation by water | |
|---|---|---|---|---|---|---|---|---|---|
| | | Lipid and oil | | | Additive [%] | Alcohol | | SAXS | |
| | | SOL+SPC +oil [%] | SOL:SPC [weight ratio] | (SOL+ SPC):oil [weight ratio]* | | EtOH [%] | PG [%] | Liquid crystal phase | q1 [nm-1] |
| 1 | C22 glycerin ester | 85 | 100:0 | 100:0 | | 10 | 5 | HII | 1.43 |
| 2 | | 92.6 | 80:20 | 100:0 | | 7.4 | | HII | 1.29 |
| 3 | | 92.6 | 60:40 | 100:0 | | 7.4 | | HII | 1.14 |
| 4 | | 92.6 | 40:60 | 100:0 | | 7.4 | | Pn3m | 0.77 |
| 5 | | 85 | 40:60 | 85:15 sesame oil | | 10 | 5 | HII | 0.82 |
| 6 | | 80.75 | 40:60 | 85:15 sesame oil | P80 4.25% | 10 | 5 | HII | 0.71 |
| 7 | Saturated C22 glycerin ester | 92.6 | 60:40 | 100:0 | | 7.4 | | HII | 1.08 |
| 8 | | 92.6 | 40:60 | 100:0 | | 7.4 | | Pn3m | 0.59 |
| 9 | Glyceryl geranylgeranyl acetate | 92.6 | 100:0 | 100:0 | | 7.4 | | HII | 1.52 |
| 10 | | 92.6 | 40:60 | 100:0 | | 7.4 | | Pn3m | 0.72 |
| 11 | Saturated C22 erythritol ester | 92.6 | 60:40 | 100:0 | | 7.4 | | Im3m | 0.35 |
| 12 | | 92.6 | 60:40 | 90:10 soybean oil | | 7.4 | | HII | 0.76 |
| 13 | | 92.6 | 100:0 | 100:0 | | 7.4 | | Pn3m+ | 0.79+ |
| | C22 pentaerythritol ester | | | | | | | Im3m | 0.62 |
| 14 | | 92.6 | 80:20 | 100:0 | | 7.4 | | Im3m | 0.57 |
| 15 | | 92.6 | 60:40 | 100:0 | | 7.4 | | Im3m | 0.46 |
| 16 | | 92.6 | 50:50 | 100:0 | | 7.4 | | Im3m | 0.32 |
| 17 | | 85 | 60:40 | 90:10 sesame oil | | 10 | 5 | Pn3m | 0.79 |
| 18 | | 80.75 | 60:40 | 90:10 sesame oil | P80 4.25% | 10 | 5 | Pn3m | 0.69 |
| 19 | Saturated C22 pentaerythritol ester | 85 | 60:40 | 90:10 sesame oil | | 10 | 5 | Pn3m | 0.74 |

(continued)

| Formulation No. | SOL used | Composition | | | | | | Liquid crystal formation by water | |
|---|---|---|---|---|---|---|---|---|---|
| | | Lipid and oil | | | Additive [%] | Alcohol | | SAXS | |
| | | SOL+SPC +oil [%] | SOL:SPC [weight ratio] | (SOL+ SPC):oil [weight ratio]* | | EtOH [%] | PG [%] | Liquid crystal phase | q1 [nm-1] |
| 20 | C22 diglycerin ester | 85 | 40:60 | 85:15 IPM | | 10 | 5 | HII | 0.69 |
| 21 | | 85 | 40:60 | 85:15 tocopherol acetate | | 10 | 5 | HII (two sizes) | 0.65+ (small) 0.54 |

(continued)

| Formulation No. | SOL used | Composition | | | | | | Liquid crystal formation by water | |
|---|---|---|---|---|---|---|---|---|---|
| | | Lipid and oil | | | Additive [%] | Alcohol | | SAXS | |
| | | SOL+SPC +oil [%] | SOL:SPC [weight ratio] | (SOL+ SPC):oil [weight ratio]* | | EtOH [%] | PG [%] | Liquid crystal phase | q1 [nm-1] |
| 22 | C22 sorbitan ester fraction | 92.6 | 100:0 | 100:0 | | 7.4 | | HII | 1.36 |
| 23 | | 92.6 | 70:30 | 100:0 | | 7.4 | | HII | 1.17 |
| 24 | | 92.6 | 50:50 | 100:0 | | 7.4 | | Pn3m+ HII | 0.81+ 1.06 |
| 25 | | 92.6 | 40:60 | 100:0 | | 7.4 | | Pn3m | 0.74 |
| 26 | | 93.7 | 70:30 | 90:10 sesame oil | | 6.3 | | HII | 1.20 |
| 27 | | 93.7 | 70:30 | 85:15 sesame oil | | 6.3 | | Fd3m | 0.57 |
| 28 | | 93.7 | 60:40 | 90:10 sesame oil | | 6.3 | | HII | 1.15 |
| 29 | | 93.7 | 50:50 | 80:20 sesame oil | | 6.3 | | HII | 0.99 |
| 30 | | 93.7 | 50:50 | 60:40 sesame oil | | 6.3 | | HII | 0.96 |
| 31 | | 85 | 40:60 | 85:15 sesame oil | | 10 | 5 | HII | 0.80 |
| 32 | | 84 | 40:60 | 85:15 sesame oil | HPC 1% | 10 | 5 | HII | 0.80 |
| 33 | | 80.75 | 40:60 | 85:15 sesame oil | P80 4.25% | 10 | 5 | HII | 0.72 |
| 34 | | 79.75 | 40:60 | 85:15 sesame oil | HPC 1% P80 4.25% | 10 | 5 | HII | 0.72 |
| 35 | | 92.6 | 40:60 | 70:30 sesame oil | | 7.4 | | HII | 0.77 |
| 36 | | 92.6 | 40:60 | 50:50 sesame oil | | 7.4 | | HII | 0.74 |
| 37 | | 92.6 | 40:60 | 20:80 sesame oil | | 7.4 | | HII | 0.68 |
| 38 | | 85 | 30:70 | 85:15 sesame oil | | 10 | 5 | Pn3m+ HII | 0.55+ 0.68 |
| 39 | | 93.7 | 70:30 | 90:10 squalene | | 6.3 | | HII | 1.16 |
| 40 | | 93.7 | 70:30 | 70:30 | | 6.3 | | Fd3m | 0.55 |

(continued)

| Formulation No. | SOL used | Composition | | | | | | Liquid crystal formation by water | |
|---|---|---|---|---|---|---|---|---|---|
| | | Lipid and oil | | | Additive [%] | Alcohol | | SAXS | |
| | | SOL+SPC +oil [%] | SOL:SPC [weight ratio] | (SOL+ SPC):oil [weight ratio]* | | EtOH [%] | PG [%] | Liquid crystal phase | q1 [nm-1] |
| | | | | squalene | | | | | |
| 41 | | 93.7 | 50:50 | 85:15 squalene | | 6.3 | | HII | 0.93 |
| 42 | | 93.7 | 70:30 | 90:10 squalane | | 6.3 | | HII | 1.16 |
| 43 | | 93.7 | 70:30 | 85:15 IPM | | 6.3 | | Fd3m | 0.62 |
| 44 | | 93.7 | 70:30 | 90:10 tocopherol acetate | | 6.3 | | HII | 1.21 |
| 45 | | 93.7 | 70:30 | 80:20 tocopherol acetate | | 6.3 | | Fd3m | 0.58 |
| 46 | | 93.7 | 50:50 | 85:15 tocopherol acetate | | 6.3 | | HII | 1.07 |
| 47 | | 93.7 | 50:50 | 70:30 tocopherol acetate | | 6.3 | | Fd3m | 0.49 |
| 48 | | 92.6 | 40:60 | 93.2:6.8 cholesterol | | 7.4 | | HII | 1.03 |
| 49 | Highly pure C22 sorbitan ester | 92.6 | 100:0 | 100:0 | | 7.4 | | Pn3m | 0.69 |
| 50 | | 92.6 | 60:40 | 90:10 sesame oil | | 7.4 | | Pn3m | 0.72 |
| 51 | | 92.6 | 50:50 | 80:20 sesame oil | | 7.4 | | Pn3m | 0.63 |

(continued)

| Formulation No. | SOL used | Composition | | | | | | Liquid crystal formation by water | |
|---|---|---|---|---|---|---|---|---|---|
| | | Lipid and oil | | | Additive [%] | Alcohol | | SAXS | |
| | | SOL+SPC +oil [%] | SOL:SPC [weight ratio] | (SOL+ SPC):oil [weight ratio]* | | EtOH [%] | PG [%] | Liquid crystal phase | q1 [nm-1] |
| 52 | Saturated C22 sorbitan ester fraction | 92.6 | 100:0 | 100:0 | | 7.4 | | HII | 1.32 |
| 53 | | 92.6 | 90:10 | 100:0 | | 7.4 | | HII | 1.25 |
| 54 | | 92.6 | 70:30 | 100:0 | | 7.4 | | HII | 1.13 |
| 55 | | 92.6 | 60:40 | 100:0 | | 7.4 | | HII | 1.06 |
| 56 | | 92.6 | 50:50 | 100:0 | | 7.4 | | Pn3m+ HII | 0.81+ 1.02 |
| 57 | | 92.6 | 40:60 | 100:0 | | 7.4 | | Pn3m+ HII | 0.63+0 . 98 |
| 58 | | 85 | 40:60 | 85:15 sesame oil | | 10 | 5 | HII | 0.74 |
| 59 | | 80.75 | 40:60 | 85:15 sesame oil | P80 4.25% | 10 | 5 | HII | 0.68 |
| 60 | C22 isosorbide ester | 92.6 | 100:0 | 100:0 | | 7.4 | | HII | 1.58 |
| 61 | | 85 | 40:60 | 85:15 sesame oil | | 10 | 5 | HII | 0.74 |

*The oil used is indicated following the weight ratio.

Table 2

| Formulation No. | SOL used | Composition | | | | | Liquid crystal formation by PBS or water | |
|---|---|---|---|---|---|---|---|---|
| | | Lipid and oil | | | Additive [%] | Alcohol | SAXS | |
| | | SOL+ phospholipid+oil [%] | SOL: phospholipid [weight ratio]* | (SOL+ phospholipid) :oil [weight ratio] | | EtOH [%] | Liquid crystal phase | q1 [nm-1] |
| 62 | C22 glycerin ester | 88 | 70:30 DMPC | 100:0 | P80 2% | 10 | HII | 1.11 |
| 63 | | 88 | 60:40 DPPC | 100:0 | P80 2% | 10 | HII | 1.05 |
| 64 | Saturated C22 sorbitan ester fraction | 92.6 | 60:40 EPC | 100:0 | | 7.4 | HII | 1.03 |

*The phospholipid used is indicated following the weight ratio.

Table 3

| Formulation No. | SOL used | Composition | | | | | | Liquid crystal formation by water | |
|---|---|---|---|---|---|---|---|---|---|
| | | Lipid and oil | | | Additive [%] | Alcohol | | SAXS | |
| | | SOL+SPC +oil [%] | SOL:SPC [weight ratio] | (SOL+ SPC):oil [weight ratio]* | | EtOH [%] | PG [%] | Liquid crystal phase | q1 [nm-1] |
| 65 | C17 glycerin ester | 85 | 100:0 | 100:0 | | 10 | 5 | Pn3m | 1.31 |
| 66 | | 85 | 60:40 | 85:15 sesame oil | | 10 | 5 | HII | 1.17 |
| 67 | | 85 | 40:60 | 85:15 sesame oil | | 10 | 5 | HII | 0.82 |
| 68 | C17 sorbitan ester fraction | 85 | 100:0 | 100:0 | | 10 | 5 | HII | 1.61 |
| 69 | | 85 | 40:60 | 92.8:7.2 sesame oil | | 10 | 5 | HII | 0.93 |
| 70 | | 85 | 40:60 | 92.8:7.2 IPM | | 10 | 5 | HII | 1.01 |
| 71 | Glyceryl monooleate | 85 | 100:0 | 100:0 | | 10 | 5 | Gel formation | |
| 72 | - | 85 | 0:100 | 35:65 GDO | | 10 | 5 | Gel formation | |
| 73 | | 85 | 0:100 | 45:55 GDO | | 10 | 5 | Gel formation | |
| 74 | | 85 | 0:100 | 30:70 tocopherol | | 10 | 5 | Gel formation | |
| 75 | | 85 | 0:100 | 70:30 IPM | | 10 | 5 | Gel formation | |

*The oil used is indicated following the weight ratio.

[Example 3] Preparation of emulsion (dispersion) and liquid crystal structure analysis - (1)

An amphipathic compound having an isoprenoid fatty acid chain having 22 carbon atoms, which is a self-organizing lipid (the abbreviation SOL is used in Tables), synthesized in Example 1, soybean phosphatidylcholine (LIPOID S100, Lipoid GmbH; the abbreviation SPC is used in Tables) as a phospholipid, an oil, and an alcohol were mixed according to the combination ratios shown in Table 4 below to obtain an oil solution. Meanwhile, Pluronic F127 (Unilube(R) 70DP-950B, NOF Corp.) as a surfactant and injectable water (Otsuka distilled water) were mixed to prepare an aqueous Pluronic solution. For some formulations, the surfactant P80 was added to the oil solution, or the antioxidants ascorbic acid and sodium sulfite were added to the aqueous Pluronic solution. The oil solution and the Pluronic aqueous solution thus prepared were each completely dissolved at 30°C or lower in a water bath, then mixed together at room temperature, and stirred with a spatula or a stirrer tip to prepare a suspension. This suspension was further dispersed with a high-pressure homogenizer (Star Burst minimo, manufactured by Sugino Machine Ltd.) to prepare a white emulsion containing fine particles (formulation Nos. 76 to 105). These emulsions were each prepared in an amount of 7 to 30 g.

Further, emulsions of formulation Nos. 106 to 116 were prepared in the same way as above according to the combination ratios shown in Table 5 using an amphipathic compound having an isoprenoid fatty acid chain having 17 or 20 carbon atoms, synthesized in Example 1, sorbitan monooleate having linear oleic acid as a fatty acid chain (NIKKOL SO-IOV, Nikko Chemicals Co., Ltd.), or purified sorbitan monooleate (obtained by removing low polar components such as oleic acid and sorbitan dioleate from NIKKOL SO-10V by silica gel column purification), instead of the amphipathic compound having an isoprenoid fatty acid chain having 22 carbon atoms, or without the use of SOL.

The emulsions of formulation Nos. 76 to 116 thus obtained were subjected to structural analysis by small-angle X-ray scattering (SAXS) using the NANO-Viewer nano-scale X-ray structure analysis equipment (manufactured by Rigaku Corp.). Each emulsion was introduced to a capillary under atmospheric pressure, and measured in the apparatus having a reduced pressure state (the sample itself was placed under atmospheric pressure). At least two scattering peaks were observed in the scattering intensity distributions obtained from the emulsions of formulation Nos. 76, 77, 81 to 86, 88, 89, 92 to 103, 105 to 109, and 115. The peak value ratios indicated the ratio of 1:√3:2 peculiar to reverse hexagonal liquid crystals. Therefore, these emulsions were found to be liquid crystal emulsions containing fine particles of reverse hexagonal liquid crystals dispersed in an aqueous phase (hexasome). A scattering peak peculiar to reverse hexagonal liquid crystals as well as a broad scattering peak were observed in the scattering intensity distributions obtained from the emulsions of formulation Nos. 77, 83 to 86, 88, 89, 92, 103, and 105 among them. These emulsions were considered as liquid crystal emulsions comprising a sponge phase (L3 phase). A broad scattering peak was observed in the scattering intensity distributions obtained from the emulsions of formulation Nos. 79, 87, 90, 91, 104, and 110 to 114. These emulsions were considered as liquid crystal emulsions containing fine particles dispersed in a sponge phase (L3 phase). At least three scattering peaks were observed in the scattering intensity distribution obtained from the emulsion of formulation No. 78, and the peak value ratio indicated the ratio of √2:√4:√6 peculiar to cubic liquid crystals belonging to the crystallographic space group Im3m. Therefore, this emulsion was found to be a liquid crystal emulsion containing fine particles of cubic liquid crystals belonging to the crystallographic space group Im3m, dispersed in an aqueous phase. At least six scattering peaks were observed in the scattering intensity distributions obtained from the emulsions of formulation Nos. 80 and 116, and the peak value ratios indicated the ratio of √3:√8:√11:√12:√16:√19 peculiar to cubic liquid crystals belonging to the crystallographic space group Fd3m. Therefore, these emulsions were found to be liquid crystal emulsions containing fine particles of cubic liquid crystals belonging to the crystallographic space group Fd3m, dispersed in an aqueous phase.

In the case of using the SOL amphipathic compound having an isoprenoid fatty acid chain having 22 carbon atoms, synthesized in Example 1, liquid crystal emulsions having non-lamellar liquid crystals were successfully prepared in a wide range of SOL:phospholipid ratios and (SOL + phospholipid):oil ratios shown in Table 4, as with the liquid crystal gels and fluids obtained from the precursor formulations described in Example 2. Even when a given amount of P80 or an antioxidant was added as an additive, liquid crystal emulsions were favorably prepared.

Table 4

| Formulation No. | SOL used | Composition | | | | | | | |
|---|---|---|---|---|---|---|---|---|---|
| | | Lipid and oil | | | Additive [%] | Alcohol | | Pluronic F127 [%] | Injectable water [%] |
| | | SOL+SPC +oil [%] | SOL:SPC [weight ratio] | (SOL+ SPC):oil [weight ratio]* | | EtOH [%] | PG [%] | | |
| 76 | C22 glycerin ester | 27.5 | 100:0 | 91:9 IPM | | 2 | | 6.25 | 64.25 |
| 77 | | 25 | 40:60 | 85:15 sesame oil | | 2 | | 5.5 | 67.5 |
| 78 | C22 pentaerythritol ester | 20 | 100:0 | 100:0 | | 4 | | 4.4 | 71.6 |
| 79 | | 20 | 50:50 | 85:15 sesame oil | | 4 | | 4.4 | 71.6 |
| 80 | C22 sorbitan ester fraction | 25 | 100:0 | 90:10 IPM | | 2 | | 5.5 | 67.5 |
| 81 | | 25 | 90:10 | 100:0 | | 2 | | 5.5 | 67.5 |
| 82 | | 25 | 70:30 | 100:0 | | 2 | | 5.5 | 67.5 |
| 83 | | 25 | 40:60 | 85:15 sesame oil | | 2 | | 4.125 | 68.875 |
| 84 | | 25 | 40:60 | 85:15 sesame oil | | 2 | | 5.5 | 67.5 |
| 85 | | 25 | 40:60 | 85:15 sesame oil | | 2 | | 6.875 | 66.125 |
| 86 | | 30 | 40:60 | 85:15 sesame oil | | 2 | | 6.6 | 61.4 |
| 87 | | 25 | 40:60 | 70:30 sesame oil | | 2 | | 5.5 | 67.5 |
| 88 | | 21 | 30:70 | 85:15 sesame oil | | 2 | | 4.62 | 72.38 |
| 89 | | 21 | 30:70 | 85:15 sesame oil | | 4 | | 4.62 | 70.38 |
| 90 | | 21 | 30:70 | 85:15 sesame oil | | 2 | 3 | 4.62 | 69.38 |
| 91 | | 21 | 30:70 | 85:15 sesame oil | P80 1% | 2 | | 4.62 | 71.38 |
| 92 | | 21 | 30:70 | 85:15 sesame oil | Ascorbic acid 0.1% Sodium sulfite 0.05% | 2 | | 4.62 | 72.23 |
| 93 | | 25 | 40:60 | 85:15 IPM | | 2 | | 4.125 | 68.875 |

(continued)

| Formulation No. | SOL used | Composition | | | | | | | |
|---|---|---|---|---|---|---|---|---|---|
| | | Lipid and oil | | | Additive [%] | Alcohol | | Pluronic F127 [%] | Injectable water [%] |
| | | SOL+SPC +oil [%] | SOL:SPC [weight ratio] | (SOL+ SPC):oil [weight ratio]* | | EtOH [%] | PG [%] | | |
| 94 | | 25 | 40:60 | 85:15 IPM | | 2 | | 4.8125 | 68.1875 |
| 95 | | 25 | 40:60 | 85:15 IPM | | 2 | | 5.5 | 67.5 |
| 96 | | 25 | 40:60 | 70:30 IPM | | 2 | | 5.5 | 67.5 |
| 97 | | 30 | 70:30 | 90:10 squalene | | 2 | | 6.6 | 61.4 |
| 98 | | 25 | 40:60 | 70:30 squalene | | 2 | | 5.5 | 67.5 |
| 99 | | 25 | 40:60 | 85:15 tocopherol acetate | | 2 | | 5.5 | 67.5 |
| 100 | | 30 | 40:60 | 85:15 tocopherol acetate | | 2 | | 6.6 | 61.4 |
| 101 | | 25 | 40:60 | 93.2:6.8 cholesterol | | 2 | | 5.5 | 67.5 |
| 102 | Saturated C22 sorbitan ester fraction | 20 | 70:30 | 100:0 | | 2 | | 5.5 | 67.5 |
| 103 | | 25 | 40:60 | 85:15 sesame oil | | 2 | | 5.5 | 67.5 |
| 104 | | 21 | 30:70 | 85:15 sesame oil | | 4 | | 4.62 | 70.38 |
| 105 | C22 isosorbide ester | 25 | 40:60 | 70:30 sesame oil | | 2 | | 5.5 | 67.5 |
| *The oil used is indicated following the weight ratio. | | | | | | | | | |

Table 5

| Formulation No. | SOL used | Composition | | | | | | |
|---|---|---|---|---|---|---|---|---|
| | | Lipid and oil | | | Additive [%] | Alcohol | Pluronic F127 [%] | Injectablewater [%] |
| | | SOL+SPC +oil [%] | SOL:SPC [weight ratio] | (SOL+ SPC):oil [weight ratio]* | | EtOH [%] | | |
| 106 | C17 glycerin ester | 27.5 | 100:0 | 91:9 IPM | | 2 | 6.25 | 64.25 |
| 107 | C17 sorbitan ester fraction | 25 | 40:60 | 92.8:7.2 sesame oil | | 2 | 5.5 | 67.5 |
| 108 | C20 glycerin ester | 27.5 | 100:0 | 91:9 IPM | | 2 | 6.25 | 64.25 |
| 109 | Saturated C20 glycerin ester | 27.5 | 100:0 | 91:9 IPM | | 2 | 6.25 | 64.25 |
| 110 | Sorbitan monooleate (NIKKOL SO-10V) | 25 | 40:60 | 92.8:7.2 sesame oil | | 2 | 5.5 | 67.5 |
| 111 | | 25 | 40:60 | 80:20 sesame oil | | 2 | 5.5 | 67.5 |
| 112 | | 25 | 40:60 | 60:40 sesame oil | | 2 | 5.5 | 67.5 |
| 113 | | 23 | 30:70 | 85:15 sesame oil | | 2 | 3.795 | 71.205 |
| 114 | Purified sorbitan monooleate | 21 | 30:70 | 85:15 sesame oil | | 2 | 4.62 | 72.38 |
| 115 | - | 25 | 0:100 | 60:40 GDO | | 2 | 5.5 | 67.5 |
| 116 | | 25 | 0:100 | 30:70 tocopherol | | 2 | 5.5 | 67.5 |

*The oil used is indicated following the weight ratio.

[Example 4] *In vivo* safety evaluation - (1)

The precursor formulations of formulation Nos. 1 to 6, 8 to 10, 12 to 16, 19, 21 to 24, 27, 31, 34, 38, 50, 52 to 54, 57, 59, 61, 65 to 67, 69, and 71 prepared in Example 2, and the emulsions of formulation Nos. 76 to 85, 88, 89, 91 to 93, 102, 103, 106, 108, and 109 prepared in Example 3 were evaluated for their safeties in the body by intraperitoneal administration. 8- to 10-week-old female Wistar rats were used in the evaluation.

The precursor formulations become bulk gels by forming non-lamellar liquid crystals after being administered into the body. Therefore, if a precursor formulation raise a foreign body response, a phenomenon (e.g., abscess having white pus) related to the foreign body response can be observed at a directly visible level. The abscess that appears in the present invention is a cystic tissue having accumulated leukocytes, etc. based on a noninfectious aseptic foreign body response. The emulsions are in a solution state containing fine particles of non-lamellar liquid crystals, and therefore can spread to the entire peritoneal cavity. Therefore, if it induces tissue damage, the tissue damage can be observed throughout the peritoneal cavity. Furthermore, absorption and transfer of the emulsions into blood through the peritoneum, etc. are faster, because of the fine particles, than those of the bulk gels, and systemic toxicity (mainly, hepatotoxicity) are much easier to observe.

(1) Safety evaluation of precursor formulation

First, the rats underwent general anesthesia with pentobarbital, and underwent laparotomy with an approximately 30 mm abdominal midline incision in the supine position. Approximately 20 mm incisions were made on the upper parietal peritoneums, and complete hemostasis was achieved. The peritoneum incisions were closed with a continuous suture of 6 stitches using Silk Suture 5-0. Approximately 23 mg (using a pipetter with a calibration set to 30 $\mu$L) of each precursor formulation was added dropwise and developed along the sutured incision wound. After injectable water (Otsuka distilled water) was sprayed (5 pushes, approximately 180 mg) to the sample applied site using a manual simple spray bottle (Spray Vial No. 2, manufactured by Maruemu Corp.), two layers of the abdominal walls were immediately closed with a suture to finish the surgery.

After 7 days of the surgery, the rats underwent general anesthesia and underwent laparotomy again. The safety of the applied precursor formulation was evaluated by intraperitoneal observations.

Abscess developed so as to surround the formed gel was observed for the precursor formulations of formulation Nos. 1, 2, 9, 13, 14, 22, 52, 53, 65, 66, and 71 containing no phospholipid or having a small phospholipid content, regardless of the number of carbon atoms of the amphipathic compound or whether the amphipathic compound has an isoprenoid fatty acid chain or a linear fatty acid chain. This abscess indicates leukocyte infiltration irrespective of infection, and was white pus resulting from a foreign body response to the administered precursor formulation. Exemplary photographs showing the developed abscess are shown in Figure 1 (Figures 1A to 1C: the precursor formulation No. 1 was applied; Figure 1D: the precursor formulation No. 71 was applied). The arrows in Figures 1A to ID depict abscess.

On the other hand, any intraperitoneal observation related to side effects including abscess was not observed for the precursor formulations of Nos. 3 to 6, 8, 10, 12, 15, 16, 19, 21, 23, 24, 27, 31, 34, 38, 50, 54, 57, 59, 61, 67, and 69 containing a given amount or more of the phospholipid.

Not only the abscess mentioned above but slight enlargement of the liver and adhesion between the liver and the greater omentum were observed for the precursor formulation of No. 65 containing C17 glycerin ester and containing no phospholipid.

The results described above revealed that, when using a given amount or more of phospholipid in the precursor formulations prepared in Example 2, neither a foreign body response nor a side effect attributed thereto is caused, regardless of the presence or absence of an oil, a surfactant, and a water soluble polymer. Particularly, regarding the case of using C22 sorbitan ester or saturated C22 sorbitan ester, the formulations containing at least 30% of phospholipid in terms of SOL:phospholipid ratio did not cause any side effect including foreign body responses.

Thus, the precursor formulations were confirmed to become more highly safe by adding phospholipid to the amphipathic compound having an isoprenoid fatty acid chain having 17 and 22 carbon atoms, synthesized in Example 1. Also, it was considered that the formulations comprising the amphipathic compound having an isoprenoid fatty acid chain having 22 carbon atoms cause few side effects even at a high dose.

(2) Safety evaluation of emulsion

First, the rats underwent general anesthesia with pentobarbital, and were placed in the supine position. A given amount of each emulsion prepared in Example 3 was intraperitoneally administered using a syringe (Terumo Syringe 1 mL) equipped with a 26 G needle. In the case of a highly viscous emulsion that found the difficulty in passing through the 26 G needle, a given amount of the emulsion was intraperitoneally administered from an approximately 1 cm abdominal midline incision site by laparotomy using a needleless syringe (Terumo Syringe 1 mL). After the administration, two

layers of the abdominal walls were closed with a suture to finish the surgery.

After 7 days of the surgery, the rats underwent general anesthesia and underwent laparotomy again. The safety of the applied emulsion was evaluated by intraperitoneal observations.

As a result, observations related to side effects were obtained for the emulsions of formulation Nos. 76, 78, 80, and 81 containing the amphipathic compound having an isoprenoid fatty acid chain having 22 carbon atoms, and containing no phospholipid or having a small phospholipid content. For example, slight ascites was observed in one out of 3 cases given the emulsion of formulation No. 76 containing C22 glycerin ester and containing no phospholipid, at a dose of 360 μL. One out of 3 cases given this emulsion at a dose of 720 μL died 3 days later, and slight enlargement of the liver was observed in the remaining two cases. Also, a slightly enlarged liver to an extent similar to that of the emulsion of No. 76 containing C22 glycerin ester, and/or perihepatic adhesion was observed for the emulsions of formulation Nos. 78, 80, and 81 containing C22 pentaerythritol ester or C22 sorbitan ester as the amphipathic lipid having an isoprenoid fatty acid chain having 22 carbon atoms, and containing no phospholipid or having a small phospholipid content.

Observations related to side effects were also obtained for the emulsions of formulation Nos. 108 and 109 containing C20 glycerin ester or saturated C20 glycerin ester having an isoprenoid fatty acid chain having 20 carbon atoms, and containing no phospholipid. For example, all of 3 cases given the emulsion of formulation No. 108 containing C20 glycerin ester and containing no phospholipid, at a dose of 220 μL died 1 day later. One out of 3 cases given the emulsion of formulation No. 109 containing saturated C20 glycerin ester and containing no phospholipid, at a dose of 220 μL also died 3 days later, and observations related to a plurality of side effects, such as ascites, enlargement of the liver, perihepatic adhesion, and liver surface whitening were obtained in the remaining two cases. The emulsions obtained using C20 glycerin ester or saturated C20 glycerin ester, even if containing phospholipid, were found to be evidently low safe. On the other hand, the emulsions of formulation Nos. 77, 79, 82 to 85, 88, 89, 91 to 93, 102, and 103 containing the amphipathic compound having an isoprenoid fatty acid chain having 22 carbon atoms, and containing a given amount or more of phospholipid were found to have no observation related to side effects throughout the peritoneal cavity even at a dose of 720 μL at least. For example, the emulsions of formulation Nos. 82 and 102 had a SOL:phospholipid ratio of 70:30. The total weight of the lipids (SOL and phospholipid) and the oil contained in 720 μL of the emulsion is 0.18 g for formulation Nos. 77, 82 to 85, 93, and 103, 0.15 g for formulation Nos. 88, 89, 91, and 92, and 0.14 g for formulation Nos. 79 and 102, which correspond to 60 g, 50 g, and 48 g, respectively, in terms of dose to humans (supposing a rat body weight of 150 g and a human body weight of 50 kg).

Observations related to side effects were found for the first time at a dose of 72 μL for the emulsion of formulation No. 106 containing C17 glycerin ester having an isoprenoid fatty acid chain having 17 carbon atoms, and containing no phospholipid. An enlarged liver and perihepatic adhesion were observed in all of 3 cases given this emulsion at a dose of 220 μL, and slight ascites was observed in 1 out thereof. All of 3 cases given this emulsion at a dose of 720 μL died 1 day later. These results revealed that emulsions obtained using C17 glycerin ester, if containing no phospholipid, have stronger side effects on the liver than those of emulsions obtained using the amphipathic compound having an isoprenoid fatty acid chain having 22 carbon atoms.

The results described above revealed that, by containing a given amount or more of phospholipid, the emulsions prepared in Example 3 were more highly safe, regardless of the presence or absence of an oil, a surfactant, and an antioxidant. This tendency was marked in the emulsions containing the amphipathic compound having an isoprenoid fatty acid chain having 22 carbon atoms, and these emulsions were very safe even at a high dose. No superiority in safety during *in vivo* application was found in the emulsions containing the amphipathic lipid having an isoprenoid fatty acid chain having 20 carbon atoms as compared with the emulsions containing the amphipathic compound having an isoprenoid fatty acid chain having 17 or 22 carbon atoms.

No improvement in safety was found in the formulations supplemented with an additive selected from the group consisting of the oil, the surfactant, the antioxidant, and the water soluble polymer (HPC) exemplified in this Example without containing phospholipid, as compared with the formulations not supplemented therewith.

It has been reported that, in the case of using glyceryl monooleate as SOL, the addition of Citrem (e.g., Grindsted(R) Citrem LR10 or BC-FS SG commercially available from Danisco A/S (Denmark)) known as a negatively charged surfactant increases safeties (e.g., Non Patent Literature 2). The safety evaluation mentioned above was carried out on emulsions derived from formulation No. 106 containing C17 glycerin ester by the replacement of the C17 glycerin ester with a mixture of C17 glycerin ester and Citrem at a C17 glycerin ester:Citrem ratio of 80:20, 60:40, or 40:60. As a result, observations related to side effects equivalent to those for the emulsion of formulation No. 106 were obtained. Observations related to side effects were obtained also in the safety evaluation on a precursor formulation having a C17 glycerin ester:Citrem ratio of 60:40, as with the precursor formulation of No. 65 containing no phospholipid. Thus, the addition of Citrem was found to not improve the safeties of formulations containing the amphipathic lipid having an isoprenoid fatty acid chain according to the present invention.

The results described above revealed that formulations containing the amphipathic compound having an isoprenoid fatty acid chain, and containing a given amount or more of phospholipid are highly safe, regardless of dosage

forms (e.g., precursor formulations and emulsions). Particularly, the results demonstrated that by containing a given amount or more of phospholipid, formulations containing the amphipathic compound having an isoprenoid fatty acid chain having 22 carbon atoms become very highly safe and can be used safely in the body even at a high dose, though such formulations have a high risk of causing side effects if containing no phospholipid.

[Example 5] Particle size of emulsion and stability evaluation thereof - (1)

The particle size distributions of the emulsions of formulation Nos. 77, 79, 81 to 105, 107, and 110 to 116 containing the phospholipid, prepared in Example 3 were measured by the dynamic light scattering method using Zetasizer Nano-ZS (manufactured by Malvern Panalytical Ltd.). Measurement samples were prepared by diluting each emulsion 200-fold with distilled water immediately after preparation (within 2 days at room temperature) or after a given period at room temperature from preparation. Table 6 shows the average particle size (nm) (Z-Average) obtained from each measurement sample, the period from the preparation day as to the emulsions after a given period at room temperature from preparation, and the amount of change in average particle size thereof (nm) (i.e., average particle size of the emulsion after a given period from preparation - average particle size of the emulsion immediately after preparation).

As for stability over time at room temperature, the average particle size had little change over a long period in the emulsions of formulation Nos. 83, 84, 86 to 88, 92, 94, 95, and 98 to 100 containing the amphipathic compound having an isoprenoid fatty acid chain having 22 carbon atoms, synthesized in Example 1. Their appearances also had little change.

By contrast, the average particle size was largely elevated in the emulsions of Nos. 110 to 114 containing sorbitan monooleate (NIKKOL SO-IOV) having linear oleic acid as a fatty acid chain, or purified sorbitan monooleate. These emulsions became strongly white in appearance, and aggregates evidently occurred in some samples. Thus, the formulations containing sorbitan monooleate were found to be instable, regardless of the purity of sorbitan monooleate.

These results revealed that emulsions containing the amphipathic compound having an isoprenoid fatty acid chain having 22 carbon atoms, and phospholipid are much superior in stability over time to emulsions containing the amphipathic compound having linear oleic acid as a fatty acid chain, and phospholipid.

Table 6

| Formulation No. | Particle size immediately after preparation | Particle size after a given period at room temperature from preparation | | |
|---|---|---|---|---|
| | Average particle size (nm) | Average particle size (nm) | Period elapsed after preparation | Amount of change (nm) |
| 77 | 133 | | | |
| 79 | 95 | | | |
| 81 | 149 | | | |
| 82 | 135 | | | |
| 83 | 145 | 143 | 5.5 months | -2 |
| 84 | 136 | 138 | 12 months | 2 |
| 85 | 109 | | | |
| 86 | 127 | 128 | 12 months | 1 |
| 87 | 105 | 109 | 9 months | 4 |
| 88 | 115 | 113 | 3.5 months | -2 |
| 89 | 103 | | | |
| 90 | 87 | | | |
| 91 | 89 | | | |
| 92 | 97 | 105 | 6.5 months | 8 |
| 93 | 128 | | | |
| 94 | 113 | 116 | 6 months | 3 |

(continued)

| Formulation No. | Particle size immediately after preparation | Particle size after a given period at room temperature from preparation | | |
|---|---|---|---|---|
| | Average particle size (nm) | Average particle size (nm) | Period elapsed after preparation | Amount of change (nm) |
| 95 | 110 | 108 | 8 months | -2 |
| 96 | 108 | | | |
| 97 | 147 | | | |
| 98 | 119 | 124 | 1.5 months | 5 |
| 99 | 144 | 148 | 6.5 months | 4 |
| 100 | 122 | 122 | 4 months | 0 |
| 101 | 115 | | | |
| 102 | 201 | | | |
| 103 | 141 | | | |
| 104 | 93 | | | |
| 105 | 101 | | | |
| 107 | 117 | | | |
| 110 | 112 | 181 | 8 months | 69 |
| 111 | 107 | 169 | 7 months | 62 |
| 112 | 114 | 219 | 7 months | 105 |
| 113 | 128 | 146 | 1 months | 18 |
| 114 | 123 | 144 | 1 week | 21 |
| 115 | 139 | | | |
| 116 | 213 | | | |

[Example 6] Confirmation of spray performance

Manual simple spray bottles (Spray Vial No. 2, manufactured by Maruemu Corp.) were filled with 1 to 5 mL each of the emulsions of formulation Nos. 77, 79, 81 to 84, 87 to 90, 92 to 99, and 101 to 105 containing the amphipathic compound having an isoprenoid fatty acid chain having 22 carbon atoms, and the phospholipid, prepared in Example 3 to prepare pump spray formulations.

The emulsions in all the formulations described above were able to be mist-sprayed or stream-sprayed. Each sample was sprayed once onto a test surface from a distance of about 2 cm. The ranges coated with the emulsions all became almost circular shapes.

[Example 7] Efficacy evaluation regarding adhesion preventing effect

The precursor formulations of Nos. 31 and 72 to 75 and the emulsions of Nos. 83 to 97, 99 to 101, 104, 105, 115, and 116 were evaluated for their adhesion preventing effects using 10-week-old female Wistar rats.

First, the rats underwent general anesthesia with pentobarbital and were placed in the supine position, and underwent laparotomy with an approximately 30 mm abdominal midline incision. Approximately 20 mm incisions were made on the right and left upper parietal peritoneums, and complete hemostasis was achieved. The right and left peritoneum incisions were closed with a continuous suture of 6 stitches using Silk Suture 5-0.

Subsequently, approximately 23 mg (using a pipetter with a calibration set to 30 μL) of the precursor formulation or approximately 72 mg of the emulsion was added dropwise and developed to the sutured right peritoneum incision to cover the sutured site (sample-applied side). For the precursor formulation, injectable water (Otsuka distilled water) was further sprayed (5 pushes, approximately 180 mg) to the sample-applied site using a manual simple spray bottle (Spray

Vial No. 2, manufactured by Maruemu Corp.) in order to induce liquid crystal formation. None was applied to the sutured left peritoneum incision (non-applied side; control). Immediately after the application of the sample to the sutured right peritoneum incision, two layers of the abdominal walls were closed (abdominal incisions were closed) with a suture to finish the surgery.

After 7 days of the surgery, the rats underwent general anesthesia and underwent laparotomy again. Adhesions at the sutured incisions on the sample-applied side and the non-applied side were evaluated according to the adhesion severity scoring and the definition of adhesion range percentage given below. In this evaluation, no observation related to side effects was obtained in all the formulations described above.

Adhesion severity:

- Grade 0: No adhesion
- Grade 1: Peelable adhesion with mild traction (involving no tissue damage)
- Grade 2: Peelable adhesion with strong traction (involving no tissue damage)
- Grade 3: Adhesion involving tissue damage by peeling with strong traction

Adhesion range percentage:

Ratio (%) of an adhesion length to the sutured incision of approximately 20 mm in length
Rating:
A, when the ratio of the adhesion range of the sample-applied incision to that of the non-applied incision (i.e., adhesion range percentage on the sample-applied side / adhesion range percentage on the non-applied side × 100) is 40% or less; B+, when the ratio is 41 to 60%; B-, when the ratio is 61 to 80%; and C, when the ratio is 81% or more.

Results of evaluating the adhesion preventing effect are shown in Table 7.

All the precursor formulation of No. 31 and the emulsions of Nos. 83 to 97, 99 to 101, 104, and 105 containing both the SOL sorbitan ester compound and phospholipid exhibited a higher adhesion preventing (reducing) effect on the sample-applied side than that on the non-applied side. On the other hand, the precursor formulations of Nos. 72 to 75 and the emulsions of Nos. 115 and 116 containing no SOL had a low adhesion preventing (reducing) effect.

These results revealed that precursor formulations and emulsions containing both SOL and phospholipid are free of side effects observable in appearance in the peritoneal cavity, and have an adhesion preventing (reducing) effect.

Table 7

| Formulation No. | Number of rats | Non-applied side | | Sample-applied side | | Rating |
|---|---|---|---|---|---|---|
| | | Adhesion severity mean±S.D. | Adhesion range percentage (%) mean±S.D. | Adhesion severity mean±S.D. | Adhesion range percentage (%) mean±S.D. | |
| 31 | 3 | 2.7±0.6 | 30±17 | 0.0±0.0 | 0±0 | A |
| 72 | 3 | 3.0±0.0 | 60±36 | 3.0±0.0 | 87±23 | C |
| 73 | 3 | 3.0±0.0 | 100±0 | 3.0±0.0 | 80±20 | C |
| 74 | 3 | 3.0±0.0 | 100±0 | 2.8±0.3 | 80±35 | C |
| 75 | 3 | 2.0±1.7 | 20±17 | 2.7±0.6 | 57±42 | C |
| 83 | 5 | 1.5±1.4 | 56±52 | 1.0±1.4 | 28±44 | B+ |
| 84 | 10 | 2.1+1.2 | 54±40 | 2.0±1.4 | 24±26 | B+ |
| 85 | 5 | 2.7±0.4 | 68±28 | 2.6±0.5 | 40±37 | B+ |
| 86 | 5 | 2.2±1.3 | 58±40 | 0.4±0.9 | 2±4 | A |

(continued)

| Formulation No. | Number of rats | Non-applied side | | Sample-applied side | | Rating |
|---|---|---|---|---|---|---|
| | | Adhesion severity mean±S.D. | Adhesion range percentage (%) mean±S.D. | Adhesion severity mean±S.D. | Adhesion range percentage (%) mean±S.D. | |
| 87 | 5 | 3.0±0.1 | 82±19 | 1.2±1.6 | 24±36 | A |
| 88 | 5 | 2.8±0.4 | 68±23 | 1.0±1.4 | 12±18 | A |
| 89 | 5 | 2.8±0.4 | 78±29 | 1.2±1.6 | 12±18 | A |
| 90 | 5 | 3.0±0.0 | 66±24 | 2.2±1.3 | 30±30 | B+ |
| 91 | 5 | 2.6±0.5 | 66±47 | 1.2±1.6 | 22±30 | A |
| 92 | 5 | 2.8±0.4 | 74±17 | 2.4±1.3 | 38±33 | B+ |
| 93 | 4 | 2.5±0.6 | 68±33 | 2.2±1.5 | 35±44 | B+ |
| 94 | 5 | 3.0±0.0 | 70±16 | 1.8±1.6 | 20±21 | A |
| 95 | 5 | 2.9±0.3 | 66±36 | 1.8±1.6 | 34±36 | B+ |
| 96 | 5 | 2.2±1.3 | 50±43 | 1.8±1.6 | 30±31 | B+ |
| 97 | 5 | 2.7±0.6 | 84±18 | 2.2±1.3 | 40±34 | B+ |
| 99 | 3 | 3.0±0.0 | 73±21 | 2.0±1.7 | 37±47 | B+ |
| 100 | 5 | 3.0±0.0 | 76±34 | 2.1+1.2 | 42±38 | B+ |
| 101 | 3 | 3.0±0.0 | 67±35 | 3.0±0.0 | 40±30 | B+ |
| 104 | 5 | 2.6±0.5 | 82±11 | 1.2±1.6 | 30±41 | A |
| 105 | 5 | 2.8±0.3 | 88±16 | 2.4±1.3 | 38±37 | B+ |
| 115 | 3 | 3.0±0.0 | 73±17 | 2.0±1.7 | 53±50 | B- |
| 116 | 3 | 2.0±1.7 | 53±50 | 2.8±0.3 | 67±31 | C |

The above-mentioned non-lamellar liquid crystal formulations containing SOL and phospholipid were further evaluated for their adhesion preventing effects using New Zealand White rabbits (female, 13 weeks old at the time of surgery) instead of the Wistar rats. After an abdominal incision was made in each rabbit under anesthesia, approximately 4 × 5 cm peritoneum and right internal oblique muscle on the right abdominal wall was excised (side-wall excision site). Also, the 6th segment of the cecal haustra was scratched using gauze, and dried for approximately 60 minutes under an incandescent lamp (cecal scratch site). In this way, cecal/peritoneal adhesion models were prepared. For a test group, 800 μL of the non-lamellar liquid crystal formulation was applied to each of the side-wall excision site and the cecal scratch site, followed by closing of the abdominal incision. For a physiological saline group as a control, 5 mL of physiological saline was applied to each of the side-wall excision site and the cecal scratch site, followed by closing of the abdominal incision. During the experimental period, the general conditions of the individual rabbits were observed once a day (before and after the surgery on the surgery day), and their body weights were measured on the surgery day and the anatomy day. The rabbits were euthanized 7 days after the surgery, and subjected to laparotomy. The formulation applied sites and intra-abdominal organs were macroscopically observed, and adhesions in the formulation applied sites were evaluated. An adhesion preventing effects were shown, as with the case of using the rats. For example, the formulations such as formulation No. 88 exhibited a much higher adhesion preventing effect than that in the control physiological saline group. Neither evidently abnormal changes in body weight of each animal individual nor systemic toxicity was found for any of the administered formulations. Also, abnormal observations (inflammation, enlargement, organ adhesion, etc.) were not found in the formulation applied sites or the intra-abdominal organs (the liver, the spleen, etc.), and neither ascites nor pleural effusion accumulation was found.

[Example 8] *In vitro* release test of leuprolide acetate - (1)

C17 glycerin ester having an isoprenoid fatty acid chain having 17 carbon atoms, which is a self-organizing lipid (the abbreviation SOL is used in Tables), synthesized in Example 1, the surfactant P80 (polyoxyethylene sorbitan

monooleate (20E.O.)), and ethanol were mixed according to the combination ratios shown in Table 8 below in the same way as in Example 2, followed by stirring at 60°C for 5 minutes. As a phospholipid, DMPC (dimyristoyl phosphatidylcholine, COATSOME MC-4040, NOF Corp.), DOPC (dioleyl phosphatidylcholine, COATSOME MC-8181, NOF Corp.), DOPE (dioleyl phosphatidylethanolamine, COATSOME ME-8181, NOF Corp.), or DOPG-Na (sodium dioleyl phosphatidylglycerin, COATSOME MG-8181LS, NOF Corp.) was added to the obtained solution, and the mixture was stirred at 60°C for 1 hour. In this way, precursor formulations of Nos. 117 to 120 were prepared.

All the precursor formulations of Nos. 117 to 120 produced gel compositions when added to large excess PBS of pH 7.4. Each gel composition was subjected to small-angle X-ray scattering diffractometry in the same way as in Example 2 to confirm that all the gel compositions had a non-lamellar liquid crystal structure. Table 8 shows the obtained liquid crystal phases, and scattering vector values q1 [nm-1] of peaks positioned on the smallest angle side.

Table 8

| Formulation No. | SOL used | Composition | | | | | Liquid crystal formation by PBS | |
|---|---|---|---|---|---|---|---|---|
| | | Lipid and oil | | | Additive [%] | Alcohol | SAXS | |
| | | SOL+phospholipid+ oil [%] | SOL: phospholipid [weight ratio]* | (SOL+ phospholipid):oil [weight ratio] | | EtOH [%] | Liquid crystal phase | q1 [nm-1] |
| 117 | C17 glycerin ester | 88 | 40:60 DMPC | 100:0 | P80 2% | 10 | Pn3m | 0.90 |
| 118 | | 88 | 40:60 DOPC | 100:0 | P80 2% | 10 | Pn3m | 0.95 |
| 119 | | 88 | 40:60 DOPE | 100:0 | P80 2% | 10 | HII | 1.20 |
| 120 | | 88 | 73:27 DOPG-Na | 100:0 | P80 2% | 10 | Im3m | 0.57 |

*The phospholipid used is indicated following the weight ratio.

Subsequently, precursor formulation Nos. 121 to 124 (prepared from Nos. 117 to 120, respectively) containing leuprolide acetate were prepared as follows. First, 3.75 mg of leuprolide acetate (L0249, Tokyo Chemical Industry Co., Ltd.) was added to a dialysis tube (Pur-A-Lyzer™ MINI 12000, Sigma-Aldrich Co. LLC), and dissolved in 5 mg of dimethyl sulfoxide, followed by addition of 91.25 mg each of the precursor formulations of Nos. 117 to 120. Each mixture thus obtained was stirred for 2 minutes with a pellet pestle to obtain 100 mg each of precursor formulation Nos. 121 to 124 containing leuprolide acetate. For a comparative control, 3.75 mg of leuprolide acetate was added to a dialysis tube, and dissolved in 96.25 mg of PBS of pH 7.4 to obtain 100 mg of aqueous solution No. 125 containing leuprolide acetate.
Precursor formulation Nos. 121 to 124 (100 mg each) and aqueous solution No. 125 (100 mg) containing leuprolide acetate were subjected to an *in vitro* release test. One dialysis tube connected at its upper portion with a floating rack was placed in one vial (25 mL size) containing 20 mL of a PBS solution of pH 7.4 containing 0.02% P80 such that the precursor formulation contained in the dialysis tube was sufficiently immersed in the PBS solution. The vial was left standing at room temperature (25°C). Subsequently, 500 μL aliquots were collected from the PBS solution in the vial after 0, 1, 3, 6, 12, 24, 48, 72, 120, and 168 hours from the start of the test, over 7 days. Precursor formulation Nos. 121 to 124 each added to the PBS solution were all confirmed to become gel compositions by visual observation.
Leuprolide acetate in each sample collected from the PBS solution was quantified by LC/MS/MS analysis using a calibration curved prepared in advance. The analysis conditions are as follows.

- Analysis column: Shodex ODP2HP-2B 2.0 mm I.D × 50 mm (Showa Denko K.K.)
- Mobile phase: water (containing 0.1% formic acid):acetonitrile = 70:30
- Flow rate: 0.1 mL, column temperature: 40°C, injection volume: 10 μL
- Precursor ion: 605.3 m/z, product ion: 249.0 m/z

Figure 2 shows data on *in vitro* release from precursor formulation Nos. 121 to 124 and aqueous solution No. 125 containing leuprolide acetate. In Figure 2, the abscissa depicts time [days], and the ordinate depicts the release rate [%] (a mean from n = 3 to 6) of leuprolide acetate into the PBS solution at each time point based on the amount of leuprolide acetate contained in each precursor formulation at the start of the test. Aqueous solution No. 125 rapidly released the whole amount of leuprolide acetate within 1 or 2 days, whereas precursor formulation Nos. 121 to 124 were all found to perform sustained release of leuprolide acetate without initial burst release. Among them, precursor formulation No. 122 had a slightly fast sustained release rate, and precursor formulation No. 124 had a slightly slow sustained release rate. Thus, the sustained release rate of leuprolide acetate was also found to be controllable by changing the type of phospholipid. From the results, the formulation of the present invention including precursor formulation Nos. 121 to 124 was found to be useful as a depot formulation.

[Example 9] Subcutaneous implantation test - (1)

7- to 8-week-old male Wistar rats underwent general anesthesia with three types of mixed anesthetic agents (medetomidine hydrochloride + midazolam + butorphanol tartrate), and their backs were shaved. Then, 100 μL each of precursor formulation Nos. 117 and 118 (containing C17 glycerin ester and the phospholipid DMPC or DOPC) prepared in the same way as in Example 8 was subcutaneously administered to the back of each rat using a syringe (Terumo Syringe 1 mL) equipped with a 23 G needle.
After 2 days of the administration, the rats underwent general anesthesia again, and the skin including the subcutaneous formulation administered site was excised. After subcutaneous fats were roughly removed, the formulation administered site was macroscopically observed.
Figure 3 shows photographs of the formulation administered sites under observation (Figure 3A: precursor formulation No. 117, Figure 3B: precursor formulation No. 118). Both the administered formulations existed as gel compositions without discoloration in the formulation administered sites. Observations attributed to formulation-derived inflammation in tissue surrounding the formulation administered site were not obtained in any of the rats given the formulations. Abnormal observations such as bleeding, loculation, or discoloration were not obtained neither.

[Example 10] Synthesis of amphipathic compound - (2)

(1) Synthesis of mono-O-(5,9,13-trimethyltetradec-4-enoyl)propylene glycol

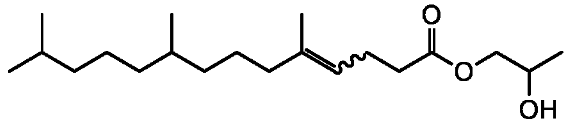

Under reduced pressure of 60 to 70 mmHg and nitrogen gas stream, 28.2 g (100 mmol) of methyl 5,9,13-trimethyltetradec-4-enoate was slowly added dropwise at 85°C to a solution of 24.3 g (317 mmol) of propylene glycol and 0.30 g (2.2 mmol) of potassium carbonate in dry N,N-dimethylformamide (70 mL), followed by stirring at the same temperature for 5 hours. The resulting reaction solution was diluted with a mixed solvent of ethyl acetate/hexane (1:1, 160 mL), washed with water, saturated sodium bicarbonate aqueous solution, and saturated brine (twice), and dried over magnesium sulfate. After filtration, the filtrate was concentrated, and the obtained residue was purified by silica gel column chromatography (mobile phase: hexane/ethyl acetate = 100:0 to 70:30) to obtain 26.1 g of the title compound (80% yield) as a pale yellow transparent liquid. Results of measuring $^1$H-NMR of the obtained compound are as follows.

$^1$H-NMR spectrum (300 MHz, $CDCl_3$, TMS) $\delta$: 0.75-0.90 (m, 9H), 0.95-1.70 (m, 18H), 1.97 (td, J = 7.6, 18 Hz, 2H), 2.07 (brs, OH), 2.25-2.42 (m, 4H), 3.55-3.70 (m, 0.7H), 3.85-4.15 (m, 1.95H), 4.98 (m, 0.35H), 5.08 (t, J = 6.2 Hz, 1H)
Mono-O-(5,9,13-trimethyltetradec-4-enoyl)propylene glycol is also referred to as C17 propylene glycol ester.

(2) Synthesis of mono-O-(5,9,13,17-tetramethyloctadec-4-enoyl)propylene glycol

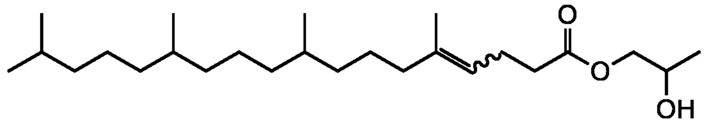

Under reduced pressure of 60 to 70 mmHg and nitrogen gas stream, 30.0 g (85.0 mmol) of methyl 5,9,13,17-tetramethyloctadec-4-enoate was slowly added dropwise at 85°C to a solution of 20.6 g (271 mmol) of propylene glycol and 0.211 g (1.53 mmol) of potassium carbonate in dry N,N-dimethylformamide (60 mL), followed by stirring at the same temperature for 3 hours. The resulting reaction solution was diluted with a mixed solvent of ethyl acetate/hexane (1:1, 120 mL), washed with water, saturated sodium bicarbonate aqueous solution, and saturated brine (twice), and dried over magnesium sulfate. After filtration, the filtrate was concentrated, and the obtained residue was purified by silica gel column chromatography (mobile phase: hexane/ethyl acetate = 100:0 to 70:30) to obtain 21.6 g of the title compound (63% yield) as a pale yellow transparent liquid. Results of measuring $^1$H-NMR of the obtained compound are as follows.

$^1$H-NMR spectrum (300 MHz, $CDCl_3$, TMS) $\delta$: 0.80-0.95 (m, 12H), 0.95-1.70 (m, 25H), 1.96 (td, J = 7.5, 18 Hz, 2H), 2.1 (brs, OH), 2.25-2.42 (m, 4H), 3.55-3.70 (m, 0.7H), 3.85-4.15 (m, 1.95H), 4.98 (m, 0.35H), 5.08 (t, J = 6.8 Hz, 1H)
Mono-O-(5,9,13,17-tetramethyloctadec-4-enoyl)propylene glycol is also referred to as C22 propylene glycol ester.

(3) Synthesis of mono-O-(5,9,13,17-tetramethyloctadecanoyl)propylene glycol

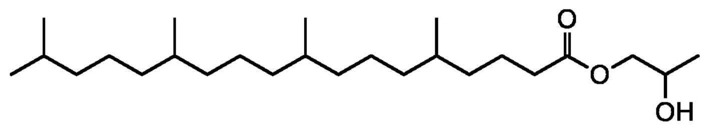

Under a nitrogen atmosphere, 0.48 g of 5% palladium carbon was added to a solution of 3.96 g (10.0 mmol) of mono-O-(5,9,13,17-tetramethyloctadec-4-enoyl)propylene glycol in ethyl acetate (12 mL). After nitrogen in the system was replaced with hydrogen, the reaction mixture was stirred at room temperature for 48 hours under an atmospheric pressure hydrogen atmosphere. After hydrogen in the system was replaced with nitrogen, the 5% palladium carbon was filtered off. The filtrate was purified by silica gel column chromatography (with ethyl acetate) to obtain 3.94 g of the title compound (99% yield) as a colorless transparent liquid. Results of measuring $^1$H-NMR of the obtained compound are as follows.

$^1$H-NMR spectrum (300 MHz, $CDCl_3$, TMS) $\delta$: 0.75-0.9 (m, 15H), 0.95-1.7 (m, 29H), 2.28 (brs, OH), 2.30 (m, 2H),

3.54-3.67 (m, 0.62H), 3.88-4.1 (m, 2.07H), 4.97 (m, 0.31H)
Mono-O-(5,9,13,17-tetramethyloctadecanoyl)propylene glycol is also referred to as saturated C22 propylene glycol ester.

[Example 11] Preparation of precursor formulation, gel formation test, and liquid crystal structure analysis - (2)

The amphipathic compound synthesized in Example 1 or 10, which is an isoprenoid lipid, the phospholipid SPC, DMPC, DPPC, DOPC, DOPE, DOPG-Na (all were the same as the products used in Example 2 or 8) or EPC (purified egg yolk lecithin, PL-100M, Kewpie Corp.), an oil, and optionally an alcohol were mixed according to the combination ratios shown in Table 9 below. The obtained mixtures were dissolved at 40°C or lower in a water bath to prepare precursor formulations of Nos. 126 to 152 shown in Table 9. Among the isoprenoid lipids used, C17 propylene glycol ester and C22 propylene glycol ester were low viscous liquids and were capable of preparing precursor formulations without being mixed with an alcohol (e.g., formulation No. 147).

C17 propylene glycol ester, C22 propylene glycol ester, and saturated C22 propylene glycol ester are isoprenoid lipids, though these lipids, when each used alone, do not form a gel by mixing with water and thus are not self-organizing lipids (SOL).

The precursor formulations of Nos. 126 to 152 were subjected to a gel formation test. An aliquot (about 100 to 300 mg) of each precursor formulation was added to excess injectable water (about 0.5 to 2 mL) in a vial, and mixed at room temperature (25°C) with a spatula and/or a vortex mixer. As a result, all the precursor formulations of Nos. 126 to 152 produced gel compositions having colorless transparent to white turbid appearance separated in the excess injectable water (aqueous medium).

The gel compositions prepared from the precursor formulations of Nos. 126 to 152 were analyzed for their non-lamellar liquid crystal structures by small-angle X-ray scattering diffractometry in the same way as in Example 2 to determine liquid crystal phases, and scattering vector values q1 [nm-1] of peaks positioned on the smallest angle side. As shown in Table 9, using the isoprenoid lipid that is a self-organizing lipid (SOL) as well as non-SOL isoprenoid lipid, the precursor formulations containing the isoprenoid lipid and phospholipid in combination were found to be able to form non-lamellar liquid crystals in the presence of water.

Precursor formulations prepared in the same way as above using propylene glycol monostearate (NIKKOL PMS-1CV, Nikko Chemicals Co., Ltd.), which is a pharmaceutical additive and has linear stearic acid as a fatty acid chain, instead of the isoprenoid lipid described above, formed no liquid crystal gel in the entire range of propylene glycol monostearate:phospholipid ratios (weight ratio) from 100:0 to 0:100.

Table 9

| Formulation No. | Isoprenoid lipid used | Composition | | | | | Liquid crystal formation by water | |
|---|---|---|---|---|---|---|---|---|
| | | Lipid and oil | | | Alcohol | | SAXS | |
| | | Isoprenoid lipid+ phospholipid +oil [%] | Isoprenoid lipid: phospholipid [weight ratio]* | (Isoprenoid lipid+ phospholipid) :oil [weight ratio]** | EtOH [%] | PG [%] | Liquid crystal phase | q1 [nm-1] |
| 126 | C22 glycerin ester | 92.6 | 50:50 SPC | 100:0 | 7.4 | | Pn3m+HII | 0.84+ 1.05 |
| 127 | C22 pentaerythritol ester | 86.2 | 80:20 SPC | 75:25 sesame oil | 13.8 | | HII | 1.00 |
| 128 | C22 sorbitan ester fraction | 92.6 | 80:20 SPC | 100:0 | 7.4 | | HII | 1.24 |
| 129 | C17 glycerin ester | 92.6 | 50:50 SPC | 100:0 | 7.4 | | Pn3m | 0.91 |
| 130 | | 85 | 50:50 SPC | 85:15 sesame oil | 10 | 5 | HII | 1.03 |
| 131 | Saturated C22 sorbitan ester fraction | 92.6 | 60:40 EPC | 100:0 | 7.4 | | HII | 1.06 |
| 132 | | 92.6 | 40:60 EPC | 100:0 | 7.4 | | Im3m+HII | 0.42+ 0.99 |
| 133 | | 85 | 40:60 EPC | 85:15 sesame oil | 10 | 5 | HII | 0.80 |
| 134 | | 86.2 | 60:40 DMPC | 100:0 | 13.8 | | Pn3m | 0.76 |
| 135 | | 86.2 | 60:40 DPPC | 100:0 | 13.8 | | Pn3m | 0.76 |
| 136 | | 86.2 | 60:40 DOPC | 100:0 | 13.8 | | HII | 1.06 |
| 137 | | 86.2 | 60:40 DOPE | 100:0 | 13.8 | | HII | 1.22 |
| 138 | | 86.2 | 60:40 SPC+DOPG-Na (9:1 mixture) | 100:0 | 13.8 | | HII | 1.05 |

(continued)

| Formulation No. | Isoprenoid lipid used | Composition | | | | | Liquid crystal formation by water | |
|---|---|---|---|---|---|---|---|---|
| | | Lipid and oil | | | Alcohol | | SAXS | |
| | | Isoprenoid lipid+ phospholipid +oil [%] | Isoprenoid lipid: phospholipid [weight ratio]* | (Isoprenoid lipid+phospholipid) :oil [weight ratio]** | EtOH [%] | PG [%] | Liquid crystal phase | q1 [nm-1] |
| 139 | C17 propylene glycol ester | 92.6 | 60:40 SPC | 100:0 | 7.4 | | Fd3m | 0.67 |
| 140 | | 92.6 | 40:60 SPC | 100:0 | 7.4 | | HII | 1.12 |
| 141 | | 92.6 | 30:70 SPC | 100:0 | 7.4 | | Im3m+HII | 0.56+ 1.07 |
| 142 | | 92.6 | 40:60 SPC | 70:30 sesame oil | 7.4 | | HII | 0.89 |
| 143 | | 92.6 | 30:70 SPC | 85:15 sesame oil | 7.4 | | HII | 0.74 |
| 144 | C22 propylene glycol ester | 92.6 | 70:30 SPC | 100:0 | 7.4 | | Fd3m | 0.71 |
| 145 | | 92.6 | 60:40 SPC | 100:0 | 7.4 | | Fd3m | 0.64 |
| 146 | | 92.6 | 50:50 SPC | 100:0 | 7.4 | | HII | 1.19 |
| 147 | | 100 | 50:50 SPC | 100:0 | 0 | | HII | 1.19 |
| 148 | | 92.6 | 40:60 SPC | 100:0 | 7.4 | | HII | 1.04 |
| 149 | | 92.6 | 30:70 SPC | 100:0 | 7.4 | | Pn3m+HII | 0.65+ 0.99 |
| 150 | | 92.6 | 40:60 SPC | 70:30 sesame oil | 7.4 | | HII | 0.81 |
| 151 | | 92.6 | 30:70 SPC | 85:15 sesame oil | 7.4 | | HII | 0.70 |
| 152 | Saturated C22 propylene glycol ester | 92.6 | 50:50 SPC | 100:0 | 7.4 | | HII | 1.15 |

*The phospholipid used is indicated following the weight ratio.
**The oil used is indicated following the weight ratio.

[Example 12] Preparation of emulsion (dispersion) and liquid crystal structure analysis - (2)

White emulsion Nos. 153 and 154 (8 g each) containing fine particles were prepared in the same way as in Example 3 using C17 glycerin ester and C22 propylene glycol ester synthesized in Examples 1 and 10, respectively, according to the combination ratios shown in Table 10 below.

The emulsions of formulation Nos. 153 and 154 were subjected to structural analysis by small-angle X-ray scattering (SAXS) in the same way as in Example 3. A broad scattering peak was observed in the scattering intensity distribution obtained from the emulsion of formulation No. 153. Therefore, this emulsion was considered as a liquid crystal emulsion containing fine particles of a sponge phase (L3 phase) dispersed in an aqueous phase. At least three scattering peaks were observed in the scattering intensity distribution obtained from the emulsion of formulation No. 154 (the scattering vector value q1 of the peak positioned on the smallest angle side was 1.08 nm-1), and the peak value ratio indicated the ratio of 1:√3:2 peculiar to reverse hexagonal liquid crystals. Therefore, this emulsion was found to be a liquid crystal emulsion containing fine particles of reverse hexagonal liquid crystals dispersed in an aqueous phase (hexasome).

Table 10

| Formulation No. | Isoprenoid lipid used | Composition | | | | | |
|---|---|---|---|---|---|---|---|
| | | Lipid and oil | | | Alcohol | Pluronic F127 [%] | Injectable water [%] |
| | | Isoprenoid lipid+SPC +oil [%] | Isoprenoid lipid:SPC [weight ratio] | (Isoprenoid lipid+SPC):oil [weight ratio]* | EtOH [%] | | |
| 153 | C17 glycerin ester | 25 | 40:60 | 85:15 sesame oil | 2 | 5.5 | 67.5 |
| 154 | C22 propylene glycol ester | 25 | 40:60 | 100:0 | 2 | 5.5 | 67.5 |
| *The oil used is indicated following the weight ratio. | | | | | | | |

[Example 13] *In vivo* safety evaluation - (2)

The precursor formulations of formulation Nos. 127, 128, 130, 131, 134, 136, 137, 139, 143, 145, 146, and 152 prepared in Example 11, and the emulsions of formulation Nos. 153 and 154 prepared in Example 12 were evaluated for their safeties in the body by intraperitoneal administration in the same way as in Example 4.

(1) Results of evaluating safety of precursor formulation

Abscess developed to surround the formed gel was observed for the precursor formulations of formulation Nos. 127 and 139 having a small phospholipid content. On the other hand, any intraperitoneal observation related to side effects including abscess as a foreign body response was not observed for the precursor formulations of formulation Nos. 128, 130, 131, 134, 136, 137, 143, 145, 146, and 152 having a larger phospholipid content.

The composition of the precursor formulation of formulation No. 127 which caused abscess corresponds to a composition in which 62.5% of the phospholipid contained in formulation No. 15 which caused no abscess in Example 4 is replaced with an oil (isoprenoid lipid (SOL in Table 1):phospholipid:oil = 60:15:25), and had the same isoprenoid lipid (SOL in Table 1):phospholipid ratio = 80:20 as that of formulation No. 14 which caused abscess in Example 4. From these results, a certain amount or more of the phospholipid, not the oil, relative to the isoprenoid lipid was found anew to be necessary for securing an *in vivo* safety.

Even by the intraperitoneal administration which requires a higher safety than that of subcutaneous implantation (Example 9), the precursor formulations caused neither a foreign body response nor other side effects, regardless of the type of phospholipid. Particularly, it is important that the results were obtained which showed that formulation Nos. 131, 134, 136, and 137 in which SPC in formulation No. 55 was replaced with any of other various phospholipids (EPC, DMPC, DOPC, or DOPE) had no side effect in safety evaluation.

Not only in the case of using an isoprenoid lipid wherein R is derived from isosorbide, as an isoprenoid lipid of the general formula (I) wherein R is a hydrophilic group having one hydroxyl group, but in the case of using an isoprenoid

lipid wherein R is derived from propylene glycol, the combination of the isoprenoid lipid with a given amount or more of phospholipid was found to cause neither a foreign body response nor other side effects (see formulation Nos. 143, 145, 146, and 152).

An isoprenoid lipid wherein R is derived from propylene glycol ester was less likely to cause side effects when having a larger number of carbon atoms, i.e., having 22 carbon atoms, than when having 17 carbon atoms (formulation No. 139 vs. formulation No. 145), as in an isoprenoid lipid wherein R is derived from glycerin ester.

(2) Results of evaluating safety of emulsion

The emulsion of formulation No. 153 containing C17 glycerin ester and having the proportion of the phospholipid as large as 60% in terms of an isoprenoid lipid:phospholipid ratio was found to have no observation related to side effects throughout the peritoneal cavity when administered at a dose of 43 μL to a rat. The total weight of the isoprenoid lipid, the phospholipid and the oil contained in the dose 43 μL was converted to 3.6 g in terms of a dose in a human so as to attain equal doses per body weight supposing a rat body weight of 150 g and a human body weight of 50 kg. However, formulation No. 153 caused a slightly enlarged liver at a larger dose of 360 μL (= a dose of 30 g in a human) and caused a higher severity of the enlarged liver at a dose of 720 μL (= a dose of 60 g in a human). Accordingly, the dose at which an emulsion containing the amphipathic compound having an isoprenoid fatty acid chain having 17 carbon atoms, and containing a given amount or more of phospholipid was able to be intraperitoneally administered in safety was found to be lower than that of an emulsion containing the amphipathic compound having an isoprenoid fatty acid chain having 22 carbon atoms, and containing a given amount or more of phospholipid.

The emulsion of formulation No. 154 containing C22 propylene glycol ester was found to have no observation related to side effects throughout the peritoneal cavity even at a dose of 720 μL (corresponding to a dose of 60 g in a human calculated as described above), as in other emulsions containing the amphipathic compound having an isoprenoid fatty acid chain having 22 carbon atoms, and containing a given amount or more of phospholipid (e.g., formulation Nos. 77, 79, 82 to 85, 88, 89, 91 to 93, 102, and 103) shown in Example 4.

[Example 14] *In vitro* release test of FD-4

The C22 glycerin ester having an isoprenoid fatty acid chain having 22 carbon atoms, synthesized in Example 1, the surfactant P80 (polyoxyethylene sorbitan monooleate (20E.O.)), and ethanol were mixed according to the combination ratios shown in Table 11 below in the same way as in Example 2, followed by stirring at 60°C for 5 minutes. As a phospholipid, DMPC, DPPC, or DOPC (all were the same as the products used in Examples 2 or 8) was added to the obtained solution, and the mixture was stirred at 60°C for 1 hour. In this way, precursor formulations of Nos. 155 to 158 were prepared.

All the precursor formulations of Nos. 155 to 158 produced gel compositions when added to large excess PBS of pH 7.4. Each gel composition was subjected to small-angle X-ray scattering diffractometry in the same way as in Example 2 to confirm that all the gel compositions had a non-lamellar liquid crystal structure. Table 11 shows the obtained liquid crystal phases, and scattering vector values q1 [nm-1] of peaks positioned on the smallest angle side.

Table 11

| Formulation No. | Isoprenoid lipid used | Composition | | | | | Liquid crystal formation by PBS | |
|---|---|---|---|---|---|---|---|---|
| | | Lipid and oil | | | Additive [%] | Alcohol | SAXS | |
| | | Isoprenoid lipid+ phospholipid +oil [%] | Isoprenoid lipid: phospholipid [weight ratio]* | (Isoprenoid lipid+ phospholipid) :oil [weight ratio] | | EtOH [%] | Liquid crystal phase | q1 [nm-1] |
| 155 | C22 glycerin ester | 88 | 86:14 DMPC | 100:0 | P80 2% | 10 | HII | 1.36 |
| 156 | | 88 | 59:41 DMPC | 100:0 | P80 2% | 10 | Pn3m | 0.85 |
| 157 | | 88 | 59:41 DPPC | 100:0 | P80 2% | 10 | HII | 1.06 |
| 158 | | 88 | 45:55 DOPC | 100:0 | P80 2% | 10 | Pn3m | 0.82 |

*The phospholipid used is indicated following the weight ratio.

Subsequently, FD-4 (fluorescein isothiocyanate-dextran of average molecular weight 4,000, Sigma-Aldrich Co. LLC, product No. 46944) was added at 15.6 mg/mL formulation to the precursor formulations of Nos. 155 to 158, and each mixture was stirred for 3 minutes with a pellet pestle to obtain precursor formulation Nos. 159 to 162 containing FD-4. For a comparative control, FD-4 was added at 15.6 mg/mL formulation to C22 glycerin ester alone and dissolved to obtain precursor formulation No. 163 of C22 glycerin ester containing FD-4 and containing no phospholipid.

Precursor formulation Nos. 159 to 163 containing FD-4 were subjected to an *in vitro* release test. 100 μL of each formulation was added to a dialysis tube (Pur-A-Lyzer™ MINI 12000, Sigma-Aldrich Co. LLC) hydrated with PBS in advance. One dialysis tube connected at its upper portion with a floating rack was placed in one vial (25 mL size) containing 20 mL of a PBS solution of pH 7.4 containing 0.01% sodium azide such that the precursor formulation contained in the dialysis tube was sufficiently immersed in the PBS solution. The vial was left standing at room temperature (25°C). Subsequently, 500 μL aliquots were collected from the PBS solution in the vial over time from immediately after the start of the test through 48 hours later (the same amount of PBS was added during each collection). Precursor formulation Nos. 159 to 163 each added to the PBS solution were all confirmed to become gel compositions by visual observation.

FD-4 in each sample collected from the PBS solution was quantified using a fluorescence spectrophotometer (RF-5300PC; Shimadzu Corp., Japan) (excitation wavelength of 490 nm, fluorescence wavelength of 515 nm).

Figure 4 shows a graph of *in vitro* release from precursor formulation Nos. 159 to 163 containing FD-4. In Figure 4, the abscissa depicts time [hours], and the ordinate depicts the release rate [%] (a mean from n = 3) of FD-4 into the PBS solution at each time point based on the amount of FD-4 contained in each precursor formulation at the start of the test. The release rate of FD-4 at 48 hours after the start of the test was 1% for formulation No. 159, 35% for No. 160, 24% for No. 161, 39% for No. 162, and 1% for No. 163.

All the precursor formulations containing FD-4 started gradual release of FD-4 without initial burst release. Precursor formulation No. 163 containing no phospholipid and formulation No. 159 having a small phospholipid content had a slow release rate, whereas precursor formulation Nos. 160 to 162 having a larger phospholipid content had a faster release rate.

From these results, drug release was found to be controllable according to the type and the content of the phospholipid in precursor formulations containing different phospholipids added to the isoprenoid lipid C22 glycerin ester having an isoprenoid fatty acid chain having 22 carbon atoms.

[Example 15] *In vitro* release test of leuprolide acetate - (2)

Precursor formulations of Nos. 164 to 169 were prepared according to the combination ratios shown in Table 12 below in the same way as in Example 2 using, as the isoprenoid lipid, C17 glycerin ester having an isoprenoid fatty acid chain having 17 carbon atoms, or C22 propylene glycol ester (R in the general formula (I) has one hydroxyl group), C22 glycerin ester (R in the general formula (I) has two hydroxyl groups), C22 sorbitan ester fraction (R in the general formula (I) has three hydroxyl groups), or saturated C22 sorbitan ester fraction (R in the general formula (I) has three hydroxyl groups) having an isoprenoid fatty acid chain having 22 carbon atoms, and the phospholipid soybean phosphatidylcholine (LIPOID S100, Lipoid GmbH; the abbreviation SPC is used in Tables), the surfactant P80 (polyoxyethylene sorbitan monooleate (20E.O.)), and ethanol.

All the precursor formulations of Nos. 164 to 169 produced gel compositions when added to large excess PBS of pH 7.4. Each gel composition was subjected to small-angle X-ray scattering diffractometry in the same way as in Example 2 to confirm that all the gel compositions had a non-lamellar liquid crystal structure. Table 12 shows the obtained liquid crystal phases, and scattering vector values q1 [nm-1] of peaks positioned on the smallest angle side.

Table 12

| Formulation No. | Isoprenoid lipid used | Composition | | | | | Liquid crystal formation by PBS | |
|---|---|---|---|---|---|---|---|---|
| | | Lipid and oil | | | Additive [%] | Alcohol | SAXS | |
| | | Isoprenoid lipid+SPC+ oil [%] | Isoprenoid lipid: SPC [weight ratio] | (Isoprenoid lipid+SPC):oil [weight ratio] | | EtOH [%] | Liquid crystal phase | q1 [nm-1] |
| 164 | C17 glycerin ester | 88 | 45:55 | 100:0 | P80 2% | 10 | Pn3m | 0.76 |
| 165 | C22 propylene glycol ester | 88 | 45:55 | 100:0 | P80 2% | 10 | HII | 1.22 |
| 166 | C22 glycerin ester | 88 | 45:55 | 100:0 | P80 2% | 10 | Pn3m+ HII | 0.81+ 1.06 |
| 167 | C22 sorbitan ester fraction | 88 | 45:55 | 100:0 | P80 2% | 10 | Pn3m+ HII | 0.73+ 1.05 |
| 168 | Saturated C22 sorbitan ester fraction | 88 | 45:55 | 100:0 | P80 2% | 10 | Pn3m | 0.73 |
| 169 | Saturated C22 sorbitan ester fraction | 88 | 73:27 | 100:0 | P80 2% | 10 | HII | 1.12 |

Subsequently, the precursor formulations of Nos. 164 to 169 were each added to a dimethyl sulfoxide solution of leuprolide acetate in the same way as in Example 8 to prepare precursor formulation Nos. 170 to 175 (100 mg each) containing leuprolide acetate, which were then subjected to an *in vitro* release test of leuprolide acetate over 7 days. Precursor formulation Nos. 170 to 175 each added to the PBS solution were all confirmed to become gel compositions by visual observation.

Figure 5 shows data on *in vitro* release from precursor formulation Nos. 170 to 175 containing leuprolide acetate. In Figure 5, the abscissa depicts time [days], and the ordinate depicts the release rate [%] (a mean from n = 3) of leuprolide acetate into the PBS solution at each time point based on the amount of leuprolide acetate contained in each precursor formulation at the start of the test. The release rate of leuprolide acetate on 7 days after the start of the test was 62% for No. 170, 7% for No. 171, 64% for No. 172, 45% for No. 173, 69% for No. 174, and 16% for No. 175.

All the precursor formulations containing leuprolide acetate started gradual release of leuprolide acetate without initial burst release. Among precursor formulation Nos. 170 to 174 having the same composition except for the isoprenoid lipid, precursor formulation No. 171 containing the most hydrophobic C22 propylene glycol ester had a slow release rate, whereas precursor formulation Nos. 170, 172, 173, and 174 containing C17 glycerin ester, C22 glycerin ester, C22 sorbitan ester fraction, and saturated C22 sorbitan ester fraction, respectively, having similar HLB values had a fast release rate. Formulation No. 165 corresponding to formulation No. 171 forms reverse hexagonal liquid crystals by the addition of PBS, formulation Nos. 164 and 168 corresponding to formulation Nos. 170 and 174 form Pn3m cubic liquid crystals by the addition of PBS, and formulation Nos. 166 and 167 corresponding to formulation Nos. 172 and 173 form Pn3m cubic liquid crystals and reverse hexagonal liquid crystals by the addition of PBS. In particular, precursor formulation No. 174 containing a saturated C22 sorbitan ester fraction having a saturated fatty acid chain had the fastest release rate. Furthermore, the release rate of precursor formulation No. 175 containing a saturated C22 sorbitan ester fraction was slower than that of precursor formulation No. 174 having a higher phospholipid ratio. Formulation No. 169 corresponding to formulation No. 175 forms reverse hexagonal liquid crystals by the addition of PBS.

From these results, drug release was found to be controllable according to the type of the isoprenoid lipid and the content of phospholipid in precursor formulations containing phospholipid added to the isoprenoid lipid represented by the general formula (I).

[Example 16] Subcutaneous implantation test - (2)

72 μL of the emulsion of formulation No. 87 prepared in Example 3, or 100 μL of each of 10 types of precursor formulations of formulation Nos. 35, 129, 145, 146, 3, 126, 23, 24, 54, and 56 prepared in Examples 2 and 11 was subcutaneously administered to between the shoulder blades of each 7-week-old male Sprague-Dawley (SD) rat (Crl:CD (SD), SPF) (n = 3 per formulation) using a syringe equipped with a 22 G injection needle.

After 7 days of the administration, the rats were euthanized by blood letting under inhalation anesthesia with sevoflurane (Mylan Seiyaku Ltd.), followed by excision of the skin including the subcutaneous formulation administered site. After subcutaneous fats were roughly removed, the formulation administered site was macroscopically observed.

Figures 6 and 7 show photographs of the formulation administered sites under observation (Figure 6A: emulsion No. 87, Figure 6B: precursor formulation No. 35, Figure 6C: precursor formulation No. 129, Figure 6D: precursor formulation No. 145, Figure 6E: precursor formulation No. 146, Figure 6F: precursor formulation No. 3, Figure 7A: precursor formulation No. 126, Figure 7B: precursor formulation No. 23, Figure 7C: precursor formulation No. 24, Figure 7D: precursor formulation No. 54, Figure 7E: precursor formulation No. 56).

Observations attributed to formulation-derived inflammation in tissue surrounding the formulation administered site were not obtained in any of the rats given the formulations. Abnormal observations such as bleeding or discoloration were not observed neither.

No formulation remained on the site administered with the emulsion of formulation No. 87. A gel composition having the same or smaller volume as or than that brought about by the dose of the formulation existed, in the same state as in the gel composition observed in the gel formation test, at the site administered with precursor formulation No. 35, precursor formulation No. 145, precursor formulation No. 146, precursor formulation No. 23, precursor formulation No. 24, precursor formulation No. 54, or precursor formulation No. 56 obtained using C22 propylene glycol ester, C22 sorbitan ester fraction, or saturated C22 sorbitan ester fraction, among the 10 types of precursor formulations mentioned above. On the other hand, a gel composition having a larger volume than that of the gel composition described above existed, at the site administered with precursor formulation No. 129, precursor formulation No. 3, or precursor formulation No. 126 obtained using C17 glycerin ester or C22 glycerin ester. This indicates that the compositions underwent the infiltration of biological substances.

From the results described above, formulations (e.g., precursor formulations and emulsions) containing the isoprenoid lipid such as C17 glycerin ester, C22 propylene glycol ester, C22 glycerin ester, C22 sorbitan ester fraction, and saturated C22 sorbitan ester fraction, and containing phospholipid such as SPC were also found to be able to be safely used by subcutaneous administration, as shown in the safety evaluation based on intraperitoneal administration

(Examples 4 and 13).

[Example 17] Synthesis of amphipathic compound - (3)

(1) Synthesis of mono-O-(3,7,11,15-tetramethylhexadec-2-enoyl)sorbitan

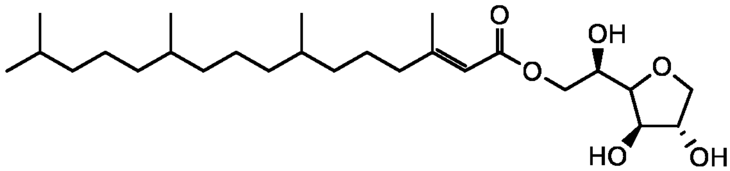

6.49 g (20 mmol) of methyl 3,7,11,15-tetramethylhexadec-2-enoate and 5.47 g of 90 % by weight sorbitan aqueous solution (30.0 mmol, sorbitan M-90, Sanko Chemical Industry Co., Ltd., containing 90% solid and 10% water, content in the solid: 79 to 84% sorbitan, 15 to 18% isosorbide) were added to a reaction container at room temperature, and then stirred at 120°C at 8 kPa for 1 hour. The reduced pressure was canceled with nitrogen, and 0.22 g (4.0 mmol) of sodium methoxide and 4 mg of sodium phosphinate monohydrate were added thereto, followed by stirring at 160°C at 8 kPa for 1 hour. The reduced pressure was canceled with nitrogen, and 0.11 g (2.0 mmol) of sodium methoxide was added thereto, followed by further stirring at 160°C at 8 kPa for 1 hour. The reduced pressure was canceled with nitrogen, and 0.11 g (2.0 mmol) of sodium methoxide was added thereto again, followed by further stirring at 160°C at 8 kPa for 1.5 hours. After the reaction mixture was cooled to 60°C, 20 mL of ethyl acetate and 20 mL of 0.5 M hydrochloric acid were added thereto with stirring. The resulting reaction solution was subj ected to extraction by the addition of 60 mL of ethyl acetate. The extract was washed with saturated sodium bicarbonate aqueous solution and saturated brine, successively, dried over magnesium sulfate, filtered, and concentrated. The resulting residue was purified by silica gel column chromatography (mobile phase: ethyl acetate/hexane mixture) to obtain 3.83 g of a fraction containing the title compound (42% yield) as a light brown transparent liquid. The obtained fraction contained mono-O-(3,7,11,15-tetramethylhexadec-2-enoyl)sorbitan and mono-O-(3,7,11,15-tetramethylhexadec-2-enoyl)isosorbide at a ratio of approximately 8:2 (weight ratio), and further contained a small amount of a diester derivative from sorbitan. Results of measuring $^1$H-NMR of the obtained fraction are as follows.

$^1$H-NMR spectrum (300 MHz, $CDCl_3$, TMS) $\delta$: 0.80-0.95 (m, 12H), 1.00-1.80 (m, 19H), 1.90-2.20 (m, 5H), 3.6-4.9 (m, 6.4H), 5.1-5.3 (m, 0.6H), 5.71 (brs, 1H)

Mono-O-(3,7,11,15-tetramethylhexadec-2-enoyl)sorbitan is also referred to as C20 sorbitan ester.

The obtained fraction was used as a mono-O-(3,7,11,15-tetramethylhexadec-2-enoyl)sorbitan fraction (or C20 sorbitan ester fraction) in Example later.

(2) Synthesis of mono-O-(3,7,11,15-tetramethylhexadecanoyl)sorbitan

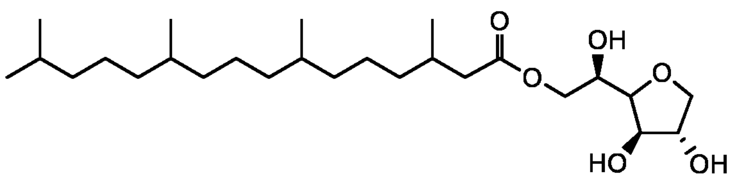

Under a nitrogen atmosphere, 0.30 g of 5% palladium carbon was added to a solution of 2.50 g (5.48 mmol) of the C20 sorbitan ester fraction in ethyl acetate (7.5 mL). After nitrogen in the system was replaced with hydrogen, the reaction mixture was stirred at room temperature for 1 day under an atmospheric pressure hydrogen atmosphere. After hydrogen in the system was replaced with nitrogen, the 5% palladium carbon was filtered off. The filtrate was purified by silica gel column chromatography (mobile phase: ethyl acetate) to obtain 2.49 g of a fraction containing the title compound (99% yield) as a colorless transparent liquid. Results of measuring $^1$H-NMR of the obtained fraction are as follows.

$^1$H-NMR spectrum (300 MHz, $CDCl_3$, TMS) δ: 0.8-0.9 (m, 12H), 0.9-1.0 (m, 3H), 1.0-2.0 (m, 22H), 2.1-2.4 (m, 2H), 3.5-4.9 (m, 6.4H), 5.0-5.2 (m, 0.6H)

Mono-O-(3,7,11,15-tetramethylhexadecanoyl)sorbitan is also referred to as saturated C20 sorbitan ester.

The obtained fraction was used as a mono-O-(3,7,11,15-tetramethylhexadecanoyl)sorbitan fraction (or saturated C20 sorbitan ester fraction) in Example later.

(3) Synthesis of mono-O-(3,7,11,15-tetramethylhexadec-2-enoyl)propylene glycol

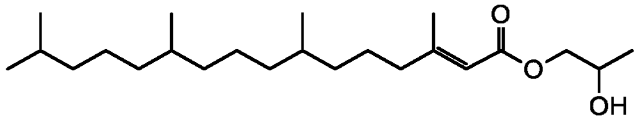

Under reduced pressure of 60 to 70 mmHg and nitrogen gas stream, 10.0 g (30.8 mmol) of methyl 3,7,11,15-tetramethylhexadec-2-enoate was slowly added dropwise at 85°C to a solution of 7.5 g (99 mmol) of propylene glycol and 60 mg (0.44 mmol) of potassium carbonate in dry N,N-dimethylformamide (22 mL), followed by stirring at the same temperature for 3 hours. The resulting reaction solution was diluted with a mixed solvent of ethyl acetate/hexane (1:1, 50 mL), washed with water, saturated sodium bicarbonate aqueous solution, and saturated brine (twice), and dried over magnesium sulfate. After filtration, the filtrate was concentrated, and the resulting residue was purified by silica gel column chromatography (mobile phase: hexane/ethyl acetate = 100:0 to 70:30) to obtain 8.18 g of the title compound (72% yield) as a low viscous pale yellow transparent liquid. Results of measuring $^1$H-NMR of the obtained compound are as follows.

$^1$H-NMR spectrum (300 MHz, $CDCl_3$, TMS) δ: 0.80-0.95 (m, 12H), 1.00-1.80 (m, 22H), 1.90-2.20 (m, 6H), 3.6-3.70 (m, 0.72H), 3.85-4.2 (m, 1.93H), 5.0 (m, 0.36H), 5.71 (brs, 1H)

Mono-O-(3,7,11,15-tetramethylhexadec-2-enoyl)propylene glycol is also referred to as C20 propylene glycol ester.

(4) Synthesis of mono-O-(3,7,11,15-tetramethylhexadecanoyl)propylene glycol

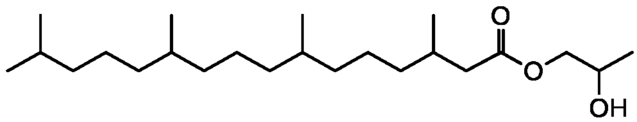

Under a nitrogen atmosphere, 0.36 g of 5% palladium carbon was added to a solution of 3.00 g (8.14 mmol) of mono-O-(3,7,11,15-tetramethylhexadec-2-enoyl)propylene glycol in ethyl acetate (9 mL). After nitrogen in the system was replaced with hydrogen, the reaction mixture was stirred at room temperature for 1 day under an atmospheric pressure hydrogen atmosphere. After hydrogen in the system was replaced with nitrogen, the 5% palladium carbon was filtered off. The filtrate was purified by silica gel column chromatography (with ethyl acetate) to obtain 2.96 g of the title compound (98% yield) as a low viscous colorless transparent liquid. Results of measuring $^1$H-NMR of the obtained compound are as follows.

$^1$H-NMR spectrum (300 MHz, $CDCl_3$, TMS) δ: 0.8-0.9 (m, 12H), 0.9-1.0 (m, 3H), 1.0-2.0 (m, 25H), 2.1-2.4 (m, 2H), 3.55-3.70 (m, 0.72H), 3.85-4.15 (m, 1.93H), 4.98 (m, 0.36H)

Mono-O-(3,7,11,15-tetramethylhexadecanoyl)propylene glycol is also referred to as saturated C20 propylene glycol ester.

(5) Synthesis of mono-O-(4,8,12,16-tetramethylheptadec-3-enoyl)sorbitan

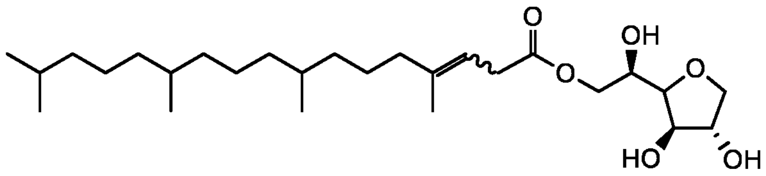

3.39 g (10 mmol) of methyl 4,8,12,16-tetramethylheptadec-3-enoate and 2.74 g of 90 % by weight sorbitan aqueous solution (15.0 mmol, sorbitan M-90, Sanko Chemical Industry Co., Ltd., containing 90% solid and 10% water, content in the solid: 79 to 84% sorbitan, 15 to 18% isosorbide) were added to a reaction container at room temperature, and then stirred at 120°C at 8 kPa for 1 hour. The reduced pressure was canceled with nitrogen, and 0.11 g (2.0 mmol) of sodium methoxide and 4 mg of sodium phosphinate monohydrate were added thereto, followed by stirring at 160°C at 8 kPa for 1 hour. The reduced pressure was canceled with nitrogen, and 54 mg(1.0 mmol) of sodium methoxide was added thereto, followed by further stirring at 160°C at 8 kPa for 1 hour. The reduced pressure was canceled with nitrogen, and 54 mg (1.0 mmol) of sodium methoxide was added thereto again, followed by further stirring at 160°C at 8 kPa for 1.5 hours. After the reaction mixture was cooled to 60°C, 10 mL of ethyl acetate and 10 mL of 0.5 M hydrochloric acid were added thereto with stirring. The resulting reaction solution was subjected to extraction by the addition of 50 mL of ethyl acetate. The extract was washed with saturated sodium bicarbonate aqueous solution and saturated brine, successively, dried over magnesium sulfate, filtered, and concentrated. The resulting residue was purified by silica gel column chromatography (mobile phase: ethyl acetate/hexane mixture) to obtain 1.79 g of a fraction containing the title compound (38% yield) as a light brown transparent liquid. The obtained fraction contained mono-O-(4,8,12,16-tetramethylheptadec-3-enoyl)sorbitan and mono-O-(4,8,12,16-tetramethylheptadec-3-enoyl)isosorbide at a ratio of approximately 8:2 (weight ratio), and further contained a small amount of a diester derivative from sorbitan. Results of measuring $^1$H-NMR of the obtained fraction are as follows.

$^1$H-NMR spectrum (300 MHz, $CDCl_3$, TMS) $\delta$: 0.80-0.90 (m, 12H), 1.00-1.60 (m, 19H), 1.64 and 1.72 (s, 3H), 2.00 (t, J = 7.4 Hz, 2H), 3.10 (d, J = 7.2 Hz, 2H), 3.5-4.9 (m, 6.4H), 5.0-5.2 (m, 0.6H), 5.30 (t, J = 7.2 Hz, 1H)
Mono-O-(4,8,12,16-tetramethylheptadec-3-enoyl)sorbitan is also referred to as C21 sorbitan ester.

The obtained fraction was used as a mono-O-(4,8,12,16-tetramethylheptadec-3-enoyl)sorbitan fraction (or C21 sorbitan ester fraction) in Example later.

(6) Synthesis of mono-O-(4,8,12,16-tetramethylheptadec-3-enoyl)propylene glycol

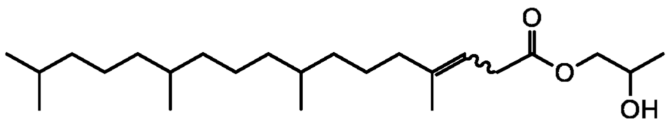

Under reduced pressure of 60 to 70 mmHg and nitrogen gas stream, 3.39 g (10 mmol) of methyl 4,8,12,16-tetramethylheptadec-3-enoate was slowly added dropwise at 85°C to a solution of 2.4 g (32 mmol) of propylene glycol and 19 mg (0.14 mmol) of potassium carbonate in dry N,N-dimethylformamide (7 mL), followed by stirring at the same temperature for 3 hours. The resulting reaction solution was diluted with a mixed solvent of ethyl acetate/hexane (1:1, 30 mL), washed with water, saturated sodium bicarbonate aqueous solution, and saturated brine (twice), and dried over magnesium sulfate. After filtration, the filtrate was concentrated, and the resulting residue was purified by silica gel column chromatography (mobile phase: hexane/ethyl acetate = 100:0 to 70:30) to obtain 2.91 g of the title compound (76% yield) as a low viscous pale yellow transparent liquid. Results of measuring $^1$H-NMR of the obtained compound are as follows.

$^1$H-NMR spectrum (300 MHz, $CDCl_3$, TMS) $\delta$: 0.80-0.90 (m, 12H), 1.00-1.60 (m, 22H), 1.62 and 1.74 (s, 3H), 2.02 (t, J = 7.1 Hz, 2H), 3.10 (d, J = 7.0 Hz, 2H), 3.55-3.70 (m, 0.72H), 3.85-4.15 (m, 1.92H), 4.98 (m, 0.36H), 5.31 (t, J = 7.0 Hz, 1H)
Mono-O-(4,8,12,16-tetramethylheptadec-3-enoyl)propylene glycol is also referred to as C21 propylene glycol ester.

(7) Synthesis of mono-O-(5,9,13,17-tetramethyloctadec-4-enoyl)ethylene glycol

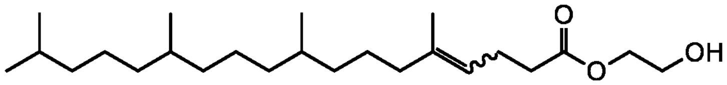

Under reduced pressure of 60 to 70 mmHg and nitrogen gas stream, 3.53 g (10.0 mmol) of methyl 5,9,13,17-tetramethyloctadec-4-enoate was slowly added dropwise at 85°C to a solution of 2.0 g (32 mmol) of ethylene glycol and 19 mg (0.14 mmol) of potassium carbonate in dry N,N-dimethylformamide (7 mL), followed by stirring at the same temperature for 3 hours. The resulting reaction solution was diluted with a mixed solvent of ethyl acetate/hexane (1:1, 30 mL), washed with water, saturated sodium bicarbonate aqueous solution, and saturated brine (twice), and dried over magnesium sulfate. After filtration, the filtrate was concentrated, and the resulting residue was purified by silica gel column chromatography (mobile phase: hexane/ethyl acetate = 100:0 to 70:30) to obtain 2.85 g of the title compound (74% yield) as a low viscous pale yellow transparent liquid. Results of measuring $^1$H-NMR of the obtained compound are as follows.

$^1$H-NMR spectrum (300 MHz, $CDCl_3$, TMS) $\delta$: 0.80-0.95 (m, 12H), 0.95-1.70 (m, 22H), 1.96 (td, J = 7.5, 18 Hz, 2H), 2.25-2.4 (m, 4H), 3.68 (t, J = 6.4 Hz, 2H), 4.10 (t, J = 6.4 Hz, 2H), 5.08 (m, 1H)
Mono-O-(5,9,13,17-tetramethyloctadec-4-enoyl)ethylene glycol is also referred to as C22 ethylene glycol ester.

(8) Synthesis of mono-O-(5,9,13,17-tetramethyloctadec-4-enoyl)1,3-butylene glycol

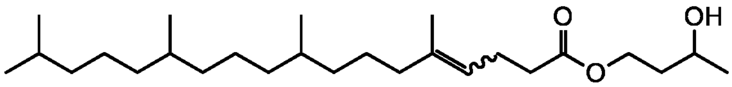

Under reduced pressure of 60 to 70 mmHg and nitrogen gas stream, 3.53 g (10.0 mmol) of methyl 5,9,13,17-tetramethyloctadec-4-enoate was slowly added dropwise at 85°C to a solution of 2.9 g (32 mmol) of 1,3-butylene glycol and 19 mg (0.14 mmol) of potassium carbonate in dry N,N-dimethylformamide (7 mL), followed by stirring at the same temperature for 3 hours. The resulting reaction solution was diluted with a mixed solvent of ethyl acetate/hexane (1:1, 30 mL), washed with water, saturated sodium bicarbonate aqueous solution, and saturated brine (twice), and dried over magnesium sulfate. After filtration, the filtrate was concentrated, and the resulting residue was purified by silica gel column chromatography (mobile phase: hexane/ethyl acetate = 100:0 to 70:30) to obtain 3.02 g of the title compound (73% yield) as a low viscous pale yellow transparent liquid. Results of measuring $^1$H-NMR of the obtained compound are as follows.

$^1$H-NMR spectrum (300 MHz, $CDCl_3$, TMS) $\delta$: 0.80-0.95 (m, 12H), 0.95-1.80 (m, 25H), 1.96 (td, J = 7.6, 18.6 Hz, 2H), 2.25-2.35 (m, 4H), 3.5-3.7 (m, 0.5H), 3.86 (m, 0.75H), 4.12 (m, 0.75H), 4.35 (ddd, J = 5.4, 8.3, 13.7 Hz, 0.75H), 5.08 (m, 1.25H)
Mono-O-(5,9,13,17-tetramethyloctadec-4-enoyl)1,3-butylene glycol is also referred to as C22 butylene glycol ester.

(9) Synthesis of mono-O-(5,9,13,17-tetramethyloctadec-4-enoyl)3-methyl-1,3-butanediol

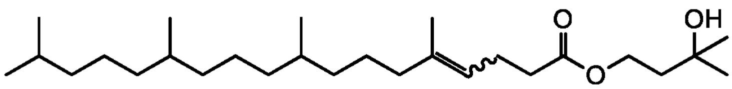

Under reduced pressure of 60 to 70 mmHg and nitrogen gas stream, 3.53 g (10.0 mmol) of methyl 5,9,13,17-tetramethyloctadec-4-enoate was slowly added dropwise at 85°C to a solution of 3.3 g (32 mmol) of 3-methyl-1,3-butanediol and 19 mg (0.14 mmol) of potassium carbonate in dry N,N-dimethylformamide (7 mL), followed by stirring at the same temperature for 3 hours. The resulting reaction solution was diluted with a mixed solvent of ethyl acetate/hexane (1:1, 30 mL), washed with water, saturated sodium bicarbonate aqueous solution, and saturated brine (twice), and dried

over magnesium sulfate. After filtration, the filtrate was concentrated, and the resulting residue was purified by silica gel column chromatography (mobile phase: hexane/ethyl acetate = 100:0 to 70:30) to obtain 2.94 g of the title compound (69% yield) as a low viscous pale yellow transparent liquid. Results of measuring $^1$H-NMR of the obtained compound are as follows.

$^1$H-NMR spectrum (300 MHz, $CDCl_3$, TMS) $\delta$: 0.80-0.95 (m, 12H), 0.95-1.70 (m, 28H), 1.84 (t, J = 6.9 Hz, 2H), 1.96 (td, J = 7.7, 18.3 Hz, 2H), 2.25-2.35 (m, 4H), 4.25 (t, J = 6.9 Hz, 2H), 5.08 (m, 1H)

Mono-O-(5,9,13,17-tetramethyloctadec-4-enoyl)3-methyl-1,3-butanediol is also referred to as C22 isoprene glycol ester.

[Example 18] Preparation of precursor formulation, gel formation test, and liquid crystal structure analysis - (3)

The amphipathic compound that is an isoprenoid lipid, synthesized in Example 17, SPC (LIPOID S100, Lipoid GmbH) as a phospholipid, and an alcohol were mixed according to the combination ratios shown in Table 13 below. The obtained mixtures were dissolved at 40°C or lower in a water bath to prepare precursor formulations of Nos. 176 to 184 shown in Table 13.

C20 propylene glycol ester, saturated C20 propylene glycol ester, C21 propylene glycol ester, C22 ethylene glycol ester, C22 butylene glycol ester, and C22 isoprene glycol ester, when each used alone, do not form a gel by mixing with water and thus are not self-organizing lipids (SOL).

The precursor formulations of Nos. 176 to 184 were subjected to a gel formation test. An aliquot (about 100 to 300 mg) of each precursor formulation was added to excess injectable water (about 0.5 to 2 mL) in a vial, and mixed at room temperature (25°C) with a spatula and/or a vortex mixer. As a result, all the precursor formulations of Nos. 176 to 184 produced gel compositions having colorless transparent to white turbid appearance separated in the excess injectable water (aqueous medium).

The gel compositions obtained from the precursor formulations of Nos. 182 to 184 are considered to exhibit a HII liquid crystal phase, as with formulation Nos. 146 and 147.

Table 13

| Formulation No. | Isoprenoid lipid used | Composition | | | |
|---|---|---|---|---|---|
| | | Lipid and oil | | | Alcohol |
| | | Isoprenoid lipid+SPC+ oil [%] | Isoprenoid lipid: SPC [weight ratio] | (Isoprenoid lipid+SPC):oil [weight ratio] | EtOH [%] |
| 176 | C20 sorbitan ester fraction | 92.6 | 50:50 | 100:0 | 7.4 |
| 177 | Saturated C20 sorbitan ester fraction | 92.6 | 50:50 | 100:0 | 7.4 |
| 178 | C20 propylene glycol ester | 92.6 | 50:50 | 100:0 | 7.4 |
| 179 | Saturated C20 propylene glycol ester | 92.6 | 50:50 | 100:0 | 7.4 |
| 180 | C21 sorbitan ester fraction | 92.6 | 50:50 | 100:0 | 7.4 |
| 181 | C21 propylene glycol ester | 92.6 | 50:50 | 100:0 | 7.4 |
| 182 | C22 ethylene glycol ester | 92.6 | 50:50 | 100:0 | 7.4 |
| 183 | C22 butylene glycol ester | 92.6 | 50:50 | 100:0 | 7.4 |
| 184 | C22 isoprene glycol ester | 92.6 | 50:50 | 100:0 | 7.4 |

All publications, patents and patent applications cited herein are incorporated herein by reference in their entirety.

## Claims

1. A non-lamellar liquid crystal-forming composition comprising an amphipathic compound represented by the following general formula (I) and a phospholipid:

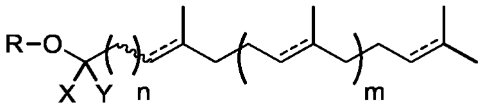

(I)

wherein X and Y each denotes a hydrogen atom or together denote an oxygen atom, n denotes the integer 1 or 2, m denotes the integer 1 or 2, the designation:
denotes a single bond or double bond, and R denotes a hydrophilic group having one or more hydroxyl groups, and
wherein the composition has an increased biocompatibility by the phospholipid.

2. The composition according to claim 1, wherein the amphipathic compound is represented by the following general formula (III) or (IV):

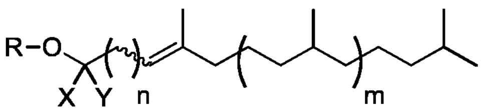

(III)

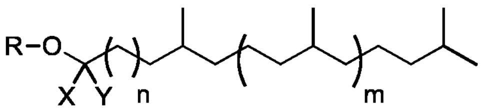

(IV)

wherein X and Y each denotes a hydrogen atom or together denote an oxygen atom, n denotes the integer 1 or 2, and m denotes the integer 1 or 2.

3. The composition according to claim 1 or 2, wherein n = 2 and m = 2 in the general formula.

4. The composition according to any one of claims 1 to 3, wherein a weight ratio between the amphipathic compound and the phospholipid is 80:20 to 20:80, or 70:30 to 30:70.

5. The composition according to claim 4, wherein the weight ratio between the amphipathic compound and the phospholipid is 50:50 to 30:70, or 45:55 to 30:70.

6. The composition according to any one of claims 1 to 5, wherein the phospholipid is phosphatidylcholine or phosphatidylethanolamine.

7. The composition according to any one of claims 1 to 6, wherein the phospholipid is selected from the group consisting of soybean phosphatidylcholine, egg yolk phosphatidylcholine, dimyristoyl phosphatidylcholine, dioleyl phosphatidylcholine, and dioleyl phosphatidylethanolamine.

8. The composition according to any one of claims 1 to 7, wherein R in the general formula (I) denotes a hydrophilic group generated by removal of one hydroxyl group from any one selected from the group consisting of glycerol, erythritol, pentaerythritol, diglycerol, sorbitan, isosorbide, and glycol.

9. The composition according to claim 1 or 2, wherein the amphipathic compound is selected from the group consisting of the following:

mono-O-(5,9,13,17-tetramethyloctadec-4-enoyl)glycerol,
mono-O-(5,9,13,17-tetramethyloctadecanoyl)glycerol,
mono-O-(5,9,13,17-tetramethyloctadeca-4,8,12,16-tetraenoyl)glycerol,
mono-O-(5,9,13,17-tetramethyloctadecanoyl)erythritol,
mono-O-(5,9,13,17-tetramethyloctadec-4-enoyl)pentaerythritol,
mono-O-(5,9,13,17-tetramethyloctadecanoyl)pentaerythritol,
mono-O-(5,9,13,17-tetramethyloctadec-4-enoyl)diglycerol,
mono-O-(5,9,13,17-tetramethyloctadec-4-enoyl)sorbitan,
mono-O-(5,9,13,17-tetramethyloctadecanoyl)sorbitan,
mono-O-(5,9,13,17-tetramethyloctadec-4-enoyl)isosorbide,
mono-O-(5,9,13,17-tetramethyloctadecanoyl)isosorbide,
mono-O-(5,9,13-trimethyltetradec-4-enoyl)glycerol,
mono-O-(5,9,13-trimethyltetradec-4-enoyl)sorbitan,
mono-O-(5,9,13-trimethyltetradecanoyl)sorbitan,
mono-O-(5,9,13-trimethyltetradec-4-enoyl)propylene glycol,
mono-O-(5,9,13,17-tetramethyloctadec-4-enoyl)propylene glycol,
mono-O-(5,9,13,17-tetramethyloctadecanoyl)propylene glycol,
mono-O-(4,8,12,16-tetramethylheptadec-3-enoyl)sorbitan,
mono-O-(4,8,12,16-tetramethylheptadec-3-enoyl)isosorbide,
mono-O-(4,8,12,16-tetramethylheptadec-3-enoyl)propylene glycol,
mono-O-(5,9,13,17-tetramethyloctadec-4-enoyl)ethylene glycol,
mono-O-(5,9,13,17-tetramethyloctadec-4-enoyl)1,3-butylene glycol, and
mono-O-(5,9,13,17-tetramethyloctadec-4-enoyl)3-methyl-1,3-butanediol.

**10.** The composition according to any one of claims 1 to 9, wherein the increased biocompatibility is indicated by reduction or disappearance of a toxicity as compared with a control composition comprising no phospholipid.

**11.** The composition according to any one of claims 1 to 10, further comprising at least one of an oil and an organic solvent.

**12.** The composition according to any one of claims 1 to 11, wherein the composition further comprises an aqueous medium and is a non-lamellar liquid crystal composition.

**13.** The composition according to claim 12, wherein the composition further comprises a surfactant and is a non-lamellar liquid crystal emulsion composition.

**14.** The composition according to any one of claims 1 to 11, wherein the composition comprises no aqueous medium and is a liquid crystal precursor composition capable of forming a non-lamellar liquid crystal in the presence of an aqueous medium.

**15.** A pharmaceutical formulation comprising the composition according to any one of claims 1 to 14.

**16.** The pharmaceutical formulation according to claim 15 for adhesion prevention of living tissue.

**17.** The pharmaceutical formulation according to claim 15, wherein the composition further comprises a drug and is a sustained release formulation.

**18.** The pharmaceutical formulation according to claim 17, wherein the drug is a gonadotropin-releasing hormone (GnRH) agonist.

**19.** The pharmaceutical formulation according to claim 18, wherein the GnRH agonist is leuprolide or a salt thereof.

**20.** The pharmaceutical formulation according to any one of claims 15 to 19, wherein the pharmaceutical formulation is a spray formulation, an aerosol formulation, an injection, or a depot formulation.

**21.** An amphipathic compound represented by the following general formula (I') or a salt thereof:

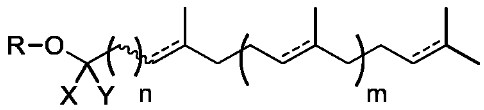

(I')

wherein X and Y each denotes a hydrogen atom or together denote an oxygen atom, n denotes the integer 1 or 2, m denotes the integer 1 or 2, the designation:



denotes a single bond or double bond, and R denotes a hydrophilic group generated by removal of one hydroxyl group from any one selected from the group consisting of sorbitan, isosorbide, and glycol.

**22.** The compound or the salt thereof according to claim 21, wherein n = 2 in the general formula (I').

**23.** The compound or the salt thereof according to claim 21 or 22, wherein the amphipathic compound is selected from the group consisting of the following:

mono-O-(5,9,13,17-tetramethyloctadec-4-enoyl)sorbitan,
mono-O-(5,9,13,17-tetramethyloctadecanoyl)sorbitan,
mono-O-(5,9,13-trimethyltetradec-4-enoyl)sorbitan,
mono-O-(5,9,13-trimethyltetradecanoyl)sorbitan,
mono-O-(5,9,13,17-tetramethyloctadec-4-enoyl)isosorbide,
mono-O-(5,9,13,17-tetramethyloctadecanoyl)isosorbide,
mono-O-(5,9,13-trimethyltetradec-4-enoyl)propylene glycol,
mono-O-(5,9,13,17-tetramethyloctadec-4-enoyl)propylene glycol,
mono-O-(5,9,13,17-tetramethyloctadecanoyl)propylene glycol,
mono-O-(4,8,12,16-tetramethylheptadec-3-enoyl)sorbitan,
mono-O-(4,8,12,16-tetramethylheptadec-3-enoyl)isosorbide,
mono-O-(4,8,12,16-tetramethylheptadec-3-enoyl)propylene glycol,
mono-O-(5,9,13,17-tetramethyloctadec-4-enoyl)ethylene glycol,
mono-O-(5,9,13,17-tetramethyloctadec-4-enoyl)1,3-butylene glycol, and
mono-O-(5,9,13,17-tetramethyloctadec-4-enoyl)3-methyl-1,3-butanediol.

Fig. 1

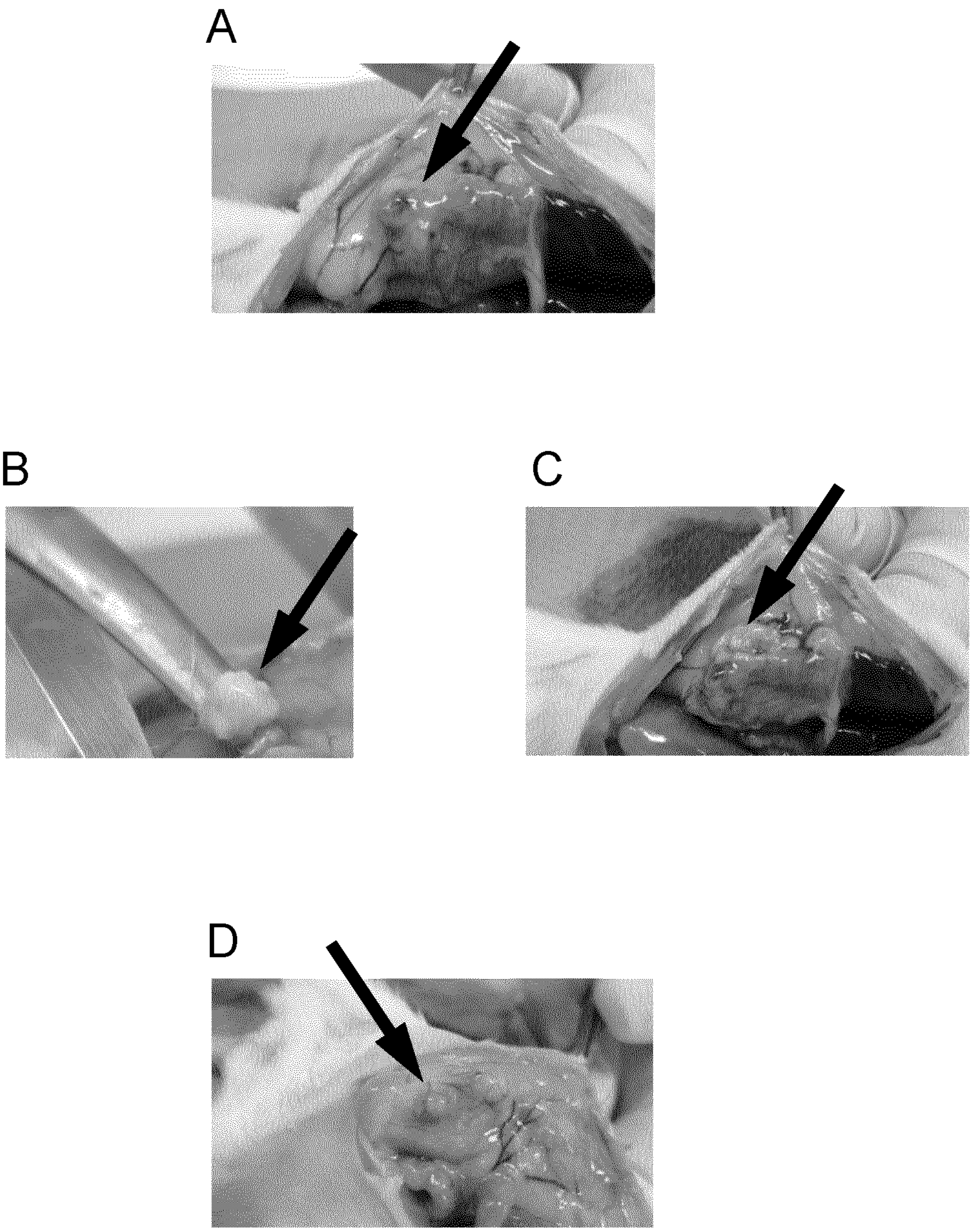

Fig. 2

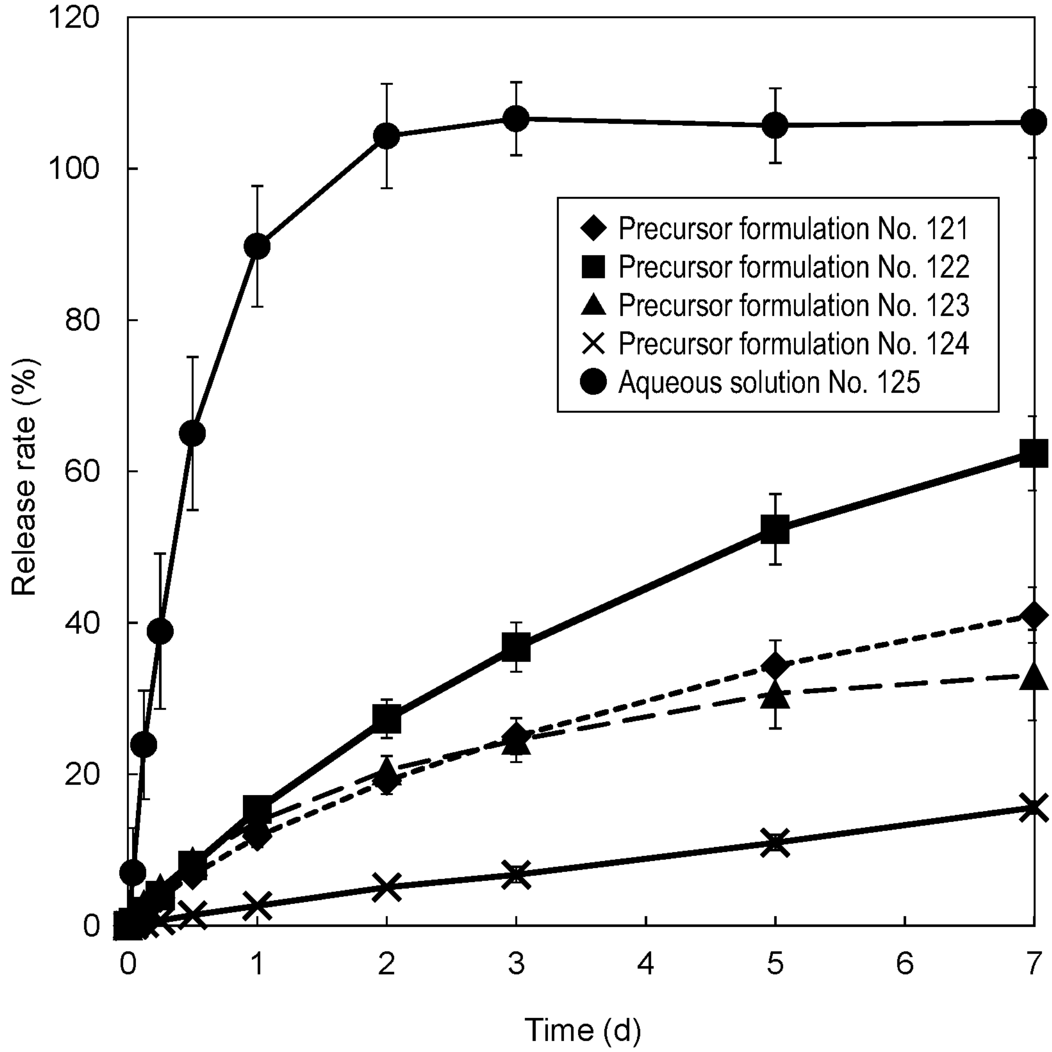
120
100
80
60
40
20
0
Release rate (%)
0
1
2
3
4
5
6
7
Time (d)
Precursor formulation No. 121
Precursor formulation No. 122
Precursor formulation No. 123
Precursor formulation No. 124
Aqueous solution No. 125

Fig. 3

A B

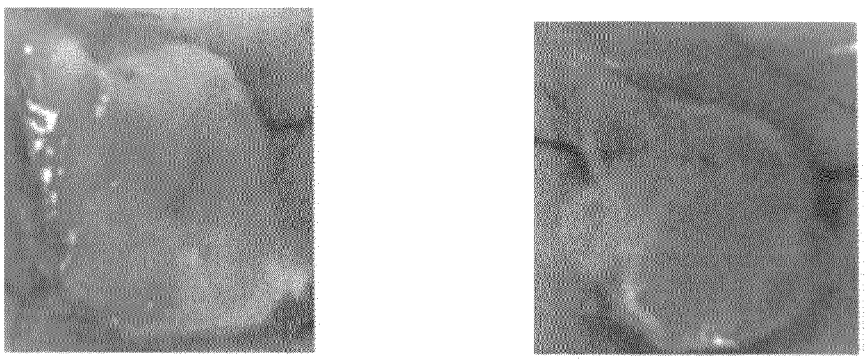

Fig. 4

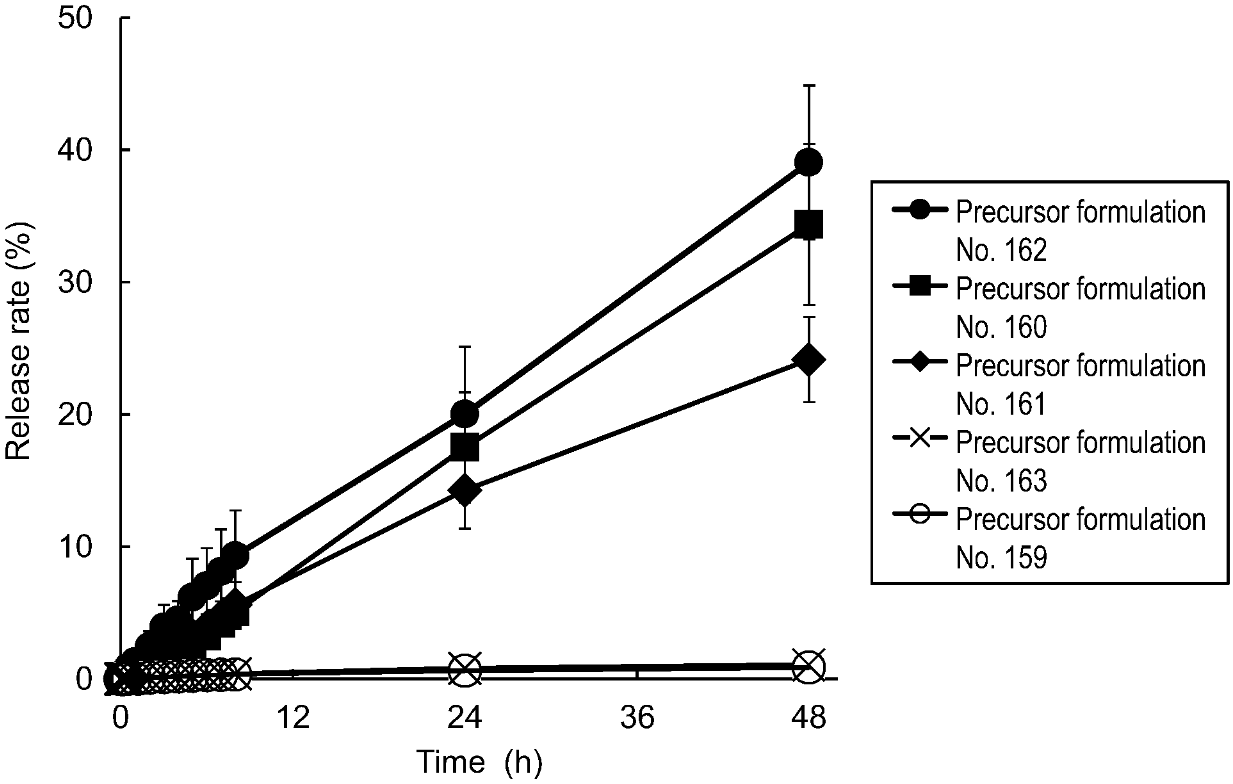
50
40
30
20
10
0
Release rate (%)
0
12
24
36
48
Time (h)
Precursor formulation No. 162
Precursor formulation No. 160
Precursor formulation No. 161
Precursor formulation No. 163
Precursor formulation No. 159

Fig. 5

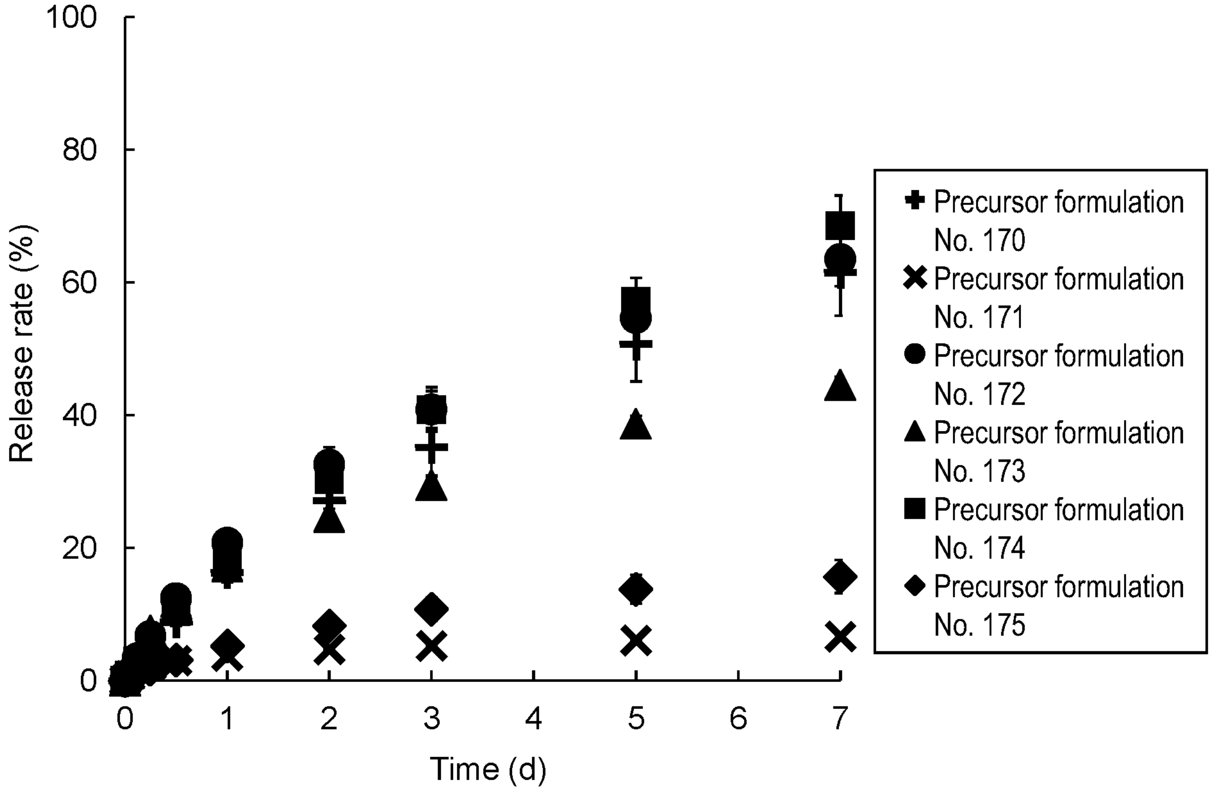
100
80
60
40
20
0
Release rate (%)
0
1
2
3
4
5
6
7
Time (d)
Precursor formulation No. 170
Precursor formulation No. 171
Precursor formulation No. 172
Precursor formulation No. 173
Precursor formulation No. 174
Precursor formulation No. 175

Fig. 6

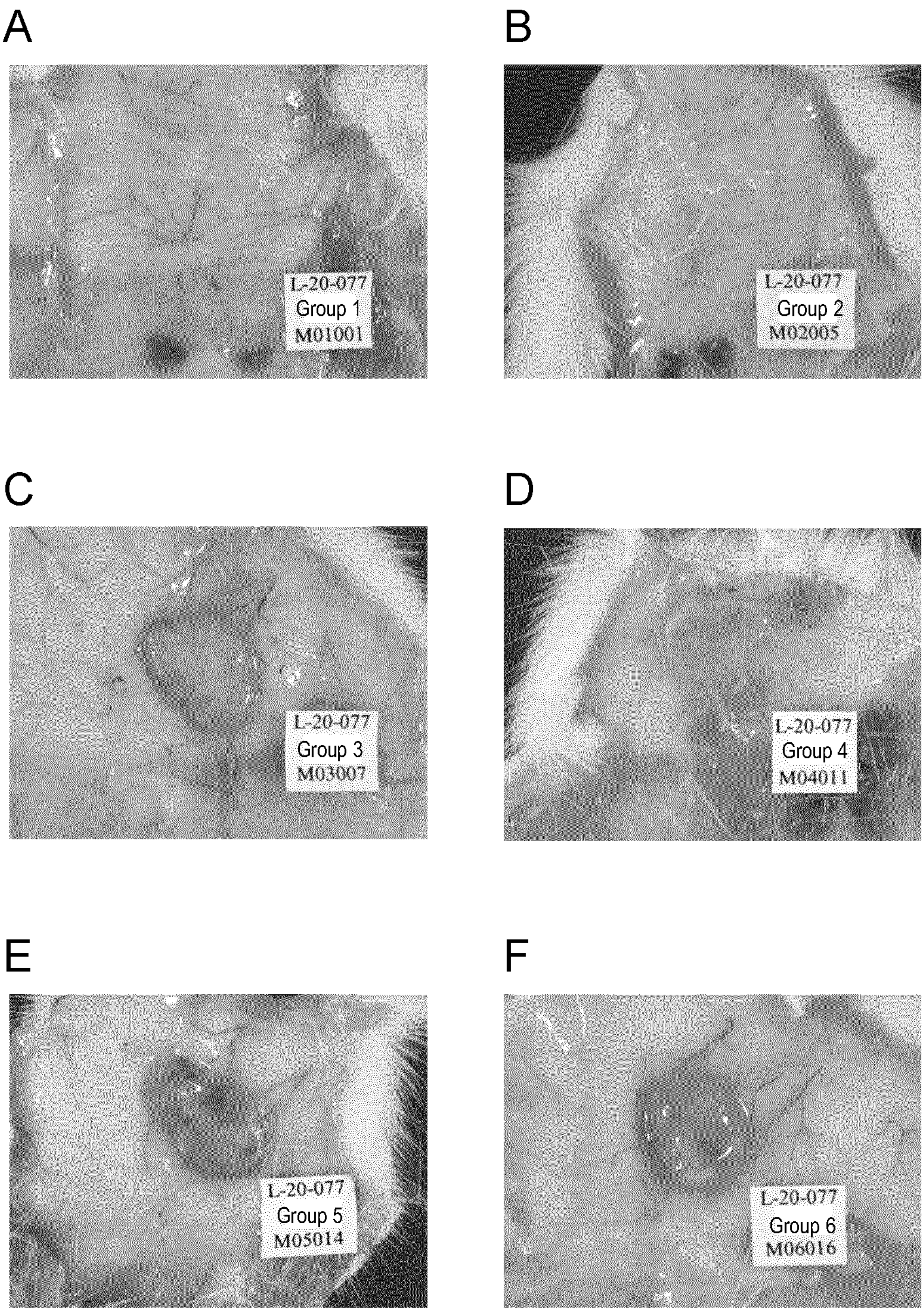
A
L-20-077
Group 1
M01001
B
L-20-077
Group 2
M02005
C
L-20-077
Group 3
M03007
D
L-20-077
Group 4
M04011
E
L-20-077
Group 5
M05014
F
L-20-077
Group 6
M06016

Fig. 7

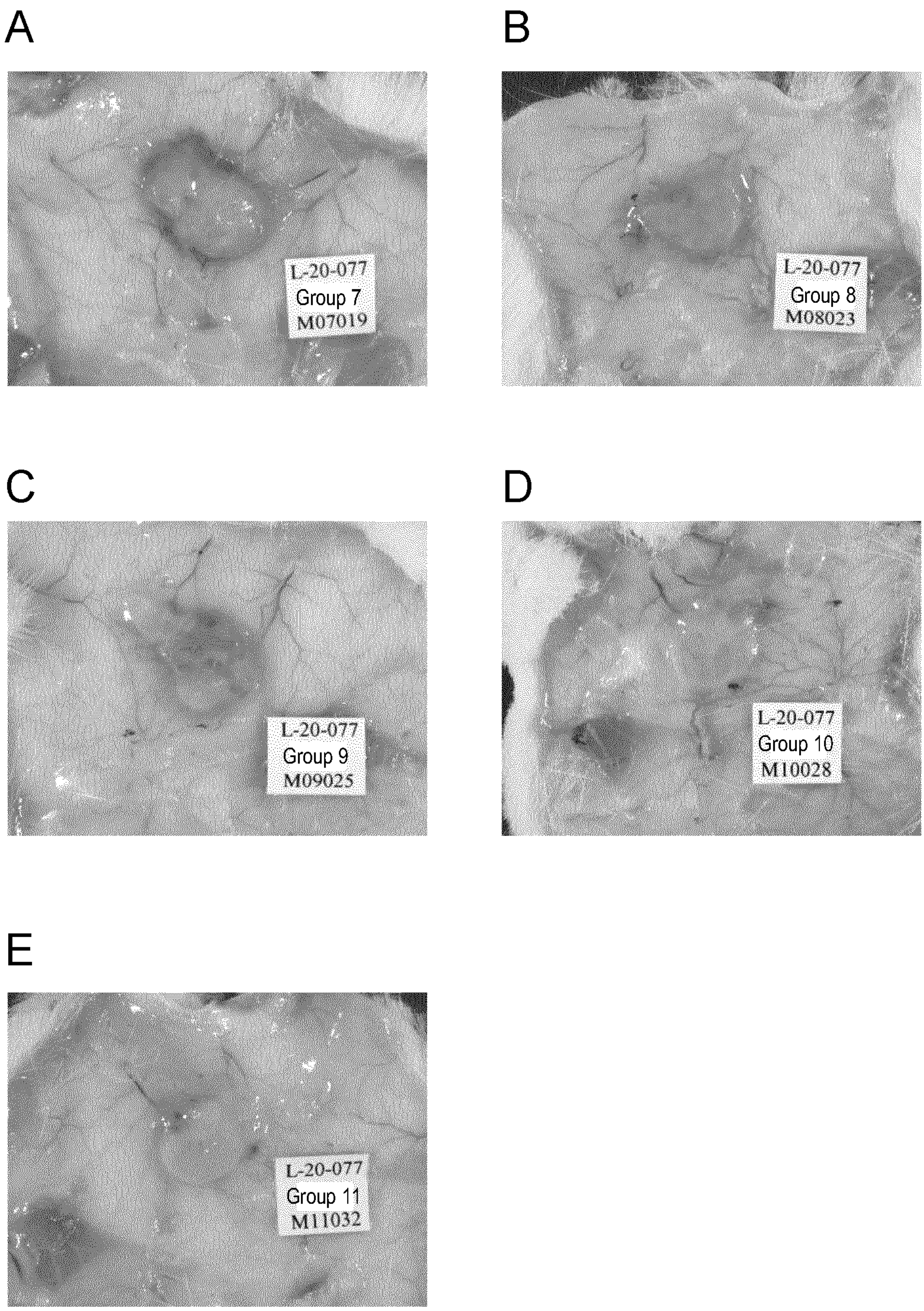
A
L-20-077
Group 7
M07019
B
L-20-077
Group 8
M08023
C
L-20-077
Group 9
M09025
D
L-20-077
Group 10
M10028
E
L-20-077
Group 11
M11032

**INTERNATIONAL SEARCH REPORT**

International application No. PCT/JP2021/002917

A. CLASSIFICATION OF SUBJECT MATTER

A61K 47/24(2006.01)i; A61K 9/08(2006.01)i; A61K 9/107(2006.01)i; A61K 9/12(2006.01)i; A61K 38/08(2019.01)i; A61K 45/00(2006.01)i; A61K 47/14(2006.01)i; A61K 47/69(2017.01)i; A61P 5/06(2006.01)i; A61P 35/00(2006.01)i; A61P 41/00(2006.01)i; C07C 69/003(2006.01)i; C07D 307/20(2006.01)i; C07D 493/04(2006.01)i

FI: A61K47/24; A61K9/107; A61K47/14; A61K47/69; A61K45/00; A61K38/08; A61P35/00; A61P5/06; A61K9/12; A61K9/08; A61P41/00; C07D307/20; C07D493/04 101D; C07C69/003 A CSP

According to International Patent Classification (IPC) or to both national classification and IPC

B. FIELDS SEARCHED

Minimum documentation searched (classification system followed by classification symbols)

A61K47/24; A61K9/08; A61K9/107; A61K9/12; A61K38/08; A61K45/00; A61K47/14; A61K47/69; A61P5/06; A61P35/00; A61P41/00; C07C69/003; C07D307/20; C07D493/04

Documentation searched other than minimum documentation to the extent that such documents are included in the fields searched

| | |
|---|---|
| Published examined utility model applications of Japan | 1922-1996 |
| Published unexamined utility model applications of Japan | 1971-2021 |
| Registered utility model specifications of Japan | 1996-2021 |
| Published registered utility model applications of Japan | 1994-2021 |

Electronic data base consulted during the international search (name of data base and, where practicable, search terms used)

JSTPlus/JMEDPlus/JST7580 (JDreamIII); CAplus/REGISTRY/MEDLINE/EMBASE/BIOSIS/WPIDS (STN)

C. DOCUMENTS CONSIDERED TO BE RELEVANT

| Category* | Citation of document, with indication, where appropriate, of the relevant passages | Relevant to claim No. |
|---|---|---|
| X | OKADA, A. et al., "Controlled release of a model hydrophilic high molecular weight compound from injectable non-lamellar liquid crystal formulations containing different types of phospholipids", International Journal of Pharmaceutics, 21 December 2019, 577 (2020), 118944, 1-9, https://doi.org/10.1016/j-ijpharm.2019.118944, entire text | 1-20 |

☒ Further documents are listed in the continuation of Box C. ☒ See patent family annex.

* Special categories of cited documents:

"A" document defining the general state of the art which is not considered to be of particular relevance

"E" earlier application or patent but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step when the document is taken alone

"Y" document of particular relevance; the claimed invention cannot be considered to involve an inventive step when the document is combined with one or more other such documents, such combination being obvious to a person skilled in the art

"&" document member of the same patent family

| Date of the actual completion of the international search | Date of mailing of the international search report |
|---|---|
| 24 March 2021 (24.03.2021) | 06 April 2021 (06.04.2021) |
| Name and mailing address of the ISA/<br>Japan Patent Office<br>3-4-3, Kasumigaseki, Chiyoda-ku,<br>Tokyo 100-8915, Japan | Authorized officer<br><br>Telephone No. |

Form PCT/ISA/210 (second sheet) (January 2015)

**INTERNATIONAL SEARCH REPORT**

International application No.
PCT/JP2021/002917

C (Continuation). DOCUMENTS CONSIDERED TO BE RELEVANT

| Category* | Citation of document, with indication, where appropriate, of the relevant passages | Relevant to claim No. |
|---|---|---|
| P, X | 岡田 明恵他，26P-pm165S 非ラメラ液晶脂質を用いたデポ製剤の開発，日本薬学会第 140 年会（京都）の Web 要旨集，05 March 2020, https://confit.atlas.jp/guide/event/pharm140/subject/2P449-486-08/advanced, entire text, (OKADA, Akie et al., "26P-pm165S Development of depot formulations using non-lamellar liquid crystalline lipids", Web abstracts of the 140th Annual Meeting of the Pharmaceutical Society of Japan (Kyoto)) | 1-20 |
| P, X | 岡田 明恵 他， 1-4-22* 非ラメラ液晶脂質を基剤としたデポ製剤の検討，日本薬剤学会第 35 年会講演要旨集，08 May 2020, entire text, (OKADA, Akie et al., "1-4-22* Development of depot formulations based on non-lamellar liquid crystalline forming lipids", Lecture abstracts of the 35th Annual Meeting of the Academy of Pharmaceutical Science and Technology, Japan) | 1-20 |
| P, X | 岡田 明恵 他，P-EiEi* 中分子薬物放出制御を可能とする非ラメラ液晶製剤の調製，第 36 回日本 DDS 学会学術集会プログラム予稿集，10 August 2020, page 192, entire text, non-official translation (OKADA, Akie et al., "P-EiEi* Preparation of non-lamellar liquid crystalline formulations that enable control of middle-molecule drug release", Program preprints of the 36th Annual Meeting of the Japan Society of Drug Delivery System) | 1-20 |
| X<br>A | JP 2004-59470 A (KANEBO LTD.) 26 February 2004 (2004-02-26) claim 1, table on page 7<br>entire text | 21, 22<br>23 |
| X<br>A | JP 2012-502922 A (THE PROCTER & GAMBLE COMPANY) 02 February 2012 (2012-02-02) synthesis examples XIV-XVII<br>entire text | 21, 22<br>23 |
| A | WO 2011/078383 A1 (CYTOPATHFINDER, INC.) 30 June 2011 (2011-06-30) entire text | 1-23 |
| A | JP 2019-99478 A (FARNEX INCORPORATED) 24 June 2019 (2019-06-24) entire text | 1-23 |
| A | WO 2014/178256 A1 (FARNEX INCORPORATED) 06 November 2014 (2014-11-06) entire text | 1-23 |

Form PCT/ISA/210 (continuation of second sheet) (January 2015)

**INTERNATIONAL SEARCH REPORT**

International application No.
PCT/JP2021/002917

C (Continuation). DOCUMENTS CONSIDERED TO BE RELEVANT

| Category* | Citation of document, with indication, where appropriate, of the relevant passages | Relevant to claim No. |
|---|---|---|
| A | 白石 盛裕 他, P-67* 非ラメラ液晶リン脂質混合体からの FD-4 放出性に及ぼすリン脂質添加の影響, 第 36 回日本 DDS 学会学術集会プログラム予稿集, 15 June 2019, page 192, entire text, non-official translation (SHIRAISHI, Morihiro et al., "P-67* Effect of phospholipid addition on FD-4 release from phospholipid mixture of non-lamellar liquid crystalline", Program preprints of the 36th Annual Meeting of the Japan Society of Drug Delivery System) | 1-23 |

Form PCT/ISA/210 (continuation of second sheet) (January 2015)

**INTERNATIONAL SEARCH REPORT**
Information on patent family members

International application No.
PCT/JP2021/002917

| Patent Documents referred in the Report | Publication Date | Patent Family | Publication Date |
|---|---|---|---|
| JP 2004-59470 A | 26 Feb. 2004 | (Family: none) | |
| JP 2012-502922 A | 02 Feb. 2012 | JP 2012-503023 A<br>US 2010/0105958 A1<br>synthesis examples XIV-XVII<br>US 2010/0137649 A1<br>US 2011/0034363 A1<br>US 2012/0010432 A1<br>US 2012/0010423 A1<br>WO 2010/033976 A2<br>WO 2010/033979 A2<br>EP 2328856 A1<br>EP 2334628 A1<br>EP 2650275 A1<br>EP 2650280 A1<br>CA 2735688 A<br>CN 102159529 A<br>CN 102159530 A | |
| WO 2011/078383 A1 | 30 Jun. 2011 | JP 2013-129660 A<br>US 2012/0264923 A1<br>US 2014/0194536 A1<br>EP 2518040 A1<br>CN 102762528 A | |
| JP 2019-99478 A | 24 Jun. 2019 | (Family: none) | |
| WO 2014/178256 A1 | 06 Nov. 2014 | JP 2016-40339 A<br>US 2016/0067208 A1<br>EP 2992910 A1<br>CN 105358190 A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2006043705 A
- WO 2011078383 A
- WO 2014178256 A
- JP 2020011237 A
- JP 2020211758 A


### Non-patent literature cited in the description


- **HINTON T.M. et al.** *Toxicology Research,* 2014, vol. 3, 11-22
- **WIBROE P.P. et al.** *Nanomedicine: Nanotechnology, Biology and Medicine,* 2015, vol. 11, 1909-1914